(19)

Europäisches Patentamt
European Patent Office
Office européen des brevets

(11) **EP 3 634 416 B1**

(12) **EUROPEAN PATENT SPECIFICATION**

(45) Date of publication and mention
of the grant of the patent:
**03.12.2025  Bulletin 2025/49**

(21) Application number: **18790576.5**

(22) Date of filing: **27.04.2018**

(51) International Patent Classification (IPC):
**A61K 31/00** *(2006.01)*     **A61K 31/50** *(2006.01)*
**A61K 38/00** *(2006.01)*     **A61K 38/12** *(2006.01)*
**A61K 9/00** *(2006.01)*       **A61K 35/30** *(2015.01)*
**A61K 38/18** *(2006.01)*     **A61K 38/22** *(2006.01)*
**A61K 38/34** *(2006.01)*     **A61K 45/06** *(2006.01)*
**A61P 27/02** *(2006.01)*     **C12N 5/079** *(2010.01)*
**A61K 38/04** *(2006.01)*

(52) Cooperative Patent Classification (CPC):
(C-Sets available)
**A61K 45/06; A61K 31/00; A61K 35/30;**
**A61K 38/046; A61K 38/185; A61K 38/225;**
**A61K 38/2285; A61K 38/34; A61P 27/02;**
**C12N 5/0621;** A61K 9/0019; A61K 9/0048;
C12N 2503/02                                  (Cont.)

(86) International application number:
**PCT/US2018/029875**

(87) International publication number:
**WO 2018/201002 (01.11.2018 Gazette 2018/44)**

(54) **METHODS AND COMPOSITIONS FOR REDUCING CORNEAL ENDOTHELIAL CELL LOSS**

VERFAHREN UND ZUSAMMENSETZUNGEN ZUR VERRINGERUNG VON
HORNHAUTENDOTHELZELLVERLUST

PROCÉDÉS ET COMPOSITIONS POUR RÉDUIRE LA PERTE DE CELLULES ENDOTHÉLIALES
CORNÉENNES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **28.04.2017  US 201762491694 P
28.04.2017  US 201762491671 P**

(43) Date of publication of application:
**15.04.2020  Bulletin 2020/16**

(60) Divisional application:
**21151121.7 / 3 865 132**

(73) Proprietor: **The Schepens Eye Research Institute,
Inc.
Boston, MA 02114 (US)**

(72) Inventors:
• **DANA, Reza**
  **Newton**
  **MA 02458 (US)**
• **KHEIRKHAH, Ahmad**
  **San Antonio**
  **TX 78229 (US)**
• **JURKUNAS, Ula, V.**
  **Winchester**
  **MA 01890 (US)**

(74) Representative: **Fish & Richardson P.C.
Highlight Business Towers
Mies-van-der-Rohe-Straße 8
80807 München (DE)**

(56) References cited:
**WO-A1-2015/077416     WO-A2-2004/004551
US-A1- 2004 161 443     US-A1- 2010 204 136**

EP 3 634 416 B1

**(Cont. next page)**

- CHRISTINE M. CLEMSON ET AL: "The Role of Alpha-MSH as a Modulator of Ocular Immunobiology Exemplifies Mechanistic Differences between Melanocortins and Steroids", OCULAR IMMUNOLOGY AND INFLAMMATION, vol. 25, no. 2, 25 January 2016 (2016-01-25), NL, pages 179 - 189, XP055764641, ISSN: 0927-3948, DOI: 10.3109/ 09273948.2015.1092560
- RU ET AL.: "$\alpha$-Melanocyte-stimulating hormone ameliorates ocular surface dysfunctions and lesions in a scopolamine-induced dry eye model via PKA-CREB and MEK-Erk pathways", SCIENTIFIC REPORTS, vol. 5, no. 1, 21 December 2015 (2015-12-21), pages 1 - 14, XP055535247
- KHEIRKHAH ET AL.: "Reduced corneal endothelial cell density in patients with dry eye disease", AMERICAN JOURNAL OF OPHTHALMOLOGY, vol. 159, no. 6, June 2015 (2015-06-01), pages 1022 - 1026, XP029226630
- LASS ET AL.: "Endothelial cell density to predict endothelial graft failure after penetrating keratoplasty", ARCHIVES OF OPHTHALMOLOGY, vol. 128, no. 1, January 2010 (2010-01-01), pages 63 - 69, XP055535251
- ANG ET AL.: "Evaluation of a micro-optical coherence tomography for the corneal endothelium in an animal model", SCIENTIFIC REPORTS, vol. 6, no. 1, 15 July 2016 (2016-07-15), pages 1 - 7, XP055535255
- PEREZ-RICO ET AL.: "Effect of topical 0.05% cyclosporine A on corneal endothelium in patients with dry eye disease", INTERNATIONAL JOURNAL OF OPHTHALMOLOGY, vol. 6, no. 4, 18 August 2013 (2013-08-18), pages 471 - 474, XP055535258

Remarks:
The complete document including Reference Table(s) and the Sequence Listing(s) can be downloaded from the EPO website

(52) Cooperative Patent Classification (CPC): (Cont.)

C-Sets
A61K 38/046, A61K 2300/00;
A61K 38/185, A61K 2300/00;
A61K 38/225, A61K 2300/00;
A61K 38/2285, A61K 2300/00;
A61K 38/34, A61K 2300/00

**Description**

**FIELD OF THE INVENTION**

The present invention relates generally to the field of ophthalmology.

**BACKGROUND**

The cornea has the highest nerve density in the body. These nerves have been shown to play an important role in maintenance of corneal structure and function. However, how these nerves promote endothelial cell function has not been known.

Improved compositions for use in methods of treating corneal disorders are needed.

Ru et al. describe in Scientific Reports vol 5, no. 1, Deccember 21, 2015 on pages 1-14 $\alpha$-Melanocyte-stimulating hormone ameliorates ocular surface dysfunctions and lesions in a scopolamine-induced dry eye model via PKA-CREB and MEK-Erk pathways.

WO 2015/077416 describes methods of reducing corneal endothelial cell loss that include administering vasoactive intestinal peptide (VIP) or a nucleic acid encoding VIP.

**BRIEF SUMMARY OF THE INVENTION**

The present invention is defined by the independent claim. The dependent claims depict other embodiments of the invention.

In accordance with the invention, provided herein is a melanocortin receptor agonist comprising an $\alpha$-Melanocyte Stimulating Hormone ($\alpha$-MSH) or a melanocortin receptor binding derivative of $\alpha$-MSH, for use in a method for treating or preventing corneal endothelial cell (CEC) loss in a subject, comprising locally administering to an eye of the subject a composition comprising an effective amount of the $\alpha$-MSH or the melanocortin receptor binding derivative of $\alpha$-MSH.

In one embodiment of the melanocortin receptor agonist for use, the subject comprises a corneal injury, a corneal dystrophy, an anterior corneal dystrophy, a stromal corneal dystrophy, a posterior corneal dystrophy, corneal endothelial dystrophy, Fuchs endothelial dystrophy, congenital hereditary endothelial dystrophy, posterior polymorphous corneal dystrophy, Schnyder crystalline corneal dystrophy, bullous keratopathy, an iridocorneal endothelial syndrome, keratitis, photokeratitis, neurotrophic keratophy, pseudoexfoliation syndrome, ocular hypertension, glaucoma, an ocular infection, a cataract, corneal endothelial cell loss due to contact lens wear, corneal endothelial cell loss due to aging, uveitis, intraocular inflammation, inflammatory disciform keratitis, diabetes, or dry eye disease.

A subject who "comprises" an injury, disorder, or disease has the injury, disorder, or disease. For example, the subject has been diagnosed with the injury, disorder, or disease.

In another embodiment of the melanocortin receptor agonist for use, the subject comprises a non-inflammatory ocular disorder, optionally wherein the non-inflammatory ocular disorder is a non-autoimmune ocular disorder, optionally wherein the non-autoimmune ocular disorder comprises a corneal injury, a corneal dystrophy, an anterior corneal dystrophy, a stromal corneal dystrophy, a posterior corneal dystrophy, corneal endothelial dystrophy, Fuchs endothelial dystrophy, congenital hereditary endothelial dystrophy, posterior polymorphous corneal dystrophy, Schnyder crystalline corneal dystrophy, bullous keratopathy, an iridocorneal endothelial syndrome, keratitis, neurotrophic keratopathy, ocular hypertension, glaucoma, diabetes, a cataract, an ocular infection, corneal endothelial cell loss due to contact lens wear, or corneal endothelial cell loss due to aging.

In one embodiment, the subject has been diagnosed as in need of ocular surgery or has received ocular surgery. In one embodiment the subject has been scheduled to receive ocular surgery, optionally wherein the surgery comprises intraocular surgery, cataract surgery, glaucoma surgery, cornea transplantation, intraocular lens implantation, injection of CECs into the eye, Descemet stripping, Descemet stripping automated endothelial keratoplasty, anterior keratoplasty, anterior lamellar keratoplasty, endothelial keratoplasty, Descemet membrane endothelial keratoplasty, Descemet stripping endothelial keratoplasty, Descemet membrane endothelial transfer, phototherapeutic keratectomy, penetrating keratoplasty, or laser eye surgery. In other embodiments, the surgery comprises vision corrective surgery, optionally the surgery comprises laser vision corrective surgery.

In one embodiment , donor CECs have been administered to the subject, optionally the CECs have been injected into an eye of the subject.

In one embodiment of the melanocortin receptor agonist is for use in a method for treating or preventing CEC loss associated with aging, optionally wherein the subject is at least about 0.5, 1, 5, 10, 15, 20, 25, 30, 35, 40, 45, 50, 55, 60, 65, 70, 75, 80, 85, or 90 years old.

In one embodiment, the subject comprises an ocular infection, optionally the ocular infection comprises an infection by a virus, bacterium, fungus, or protozoan, optionally wherein the ocular infection comprises a protozoan infection by an acanthamoeba or the ocular infection comprises a keratitis infection by a herpes simplex virus. In one embodiment, the subject comprises conjunctivitis, optionally wherein the conjunctivitis comprises viral, allergic, bacterial, or chemical conjunctivitis.

In one embodiments, the subject comprises corneal endothelial cell loss due to contact lens wear, and the subject has worn contact lenses at least once per month for at least about 5 years.In one embodiment, the effective amount is effective to increase the number of CECs in the subject, or the effective amount is effective to slow a decrease in the number of CECs in the subject, optionally wherein the effective amount is effective to prevent the density of CECs in the cornea of the subject from decreasing by more than about 50, 100, 150, 200, 250, 300, 350, 400, 450, or 500 cells/mm$^2$ within the first 6 months after ocular surgery, or the effective amount is effective to reduce apoptosis of CECs in the subject, or the effective amount is effective to increase proliferation of CECs in the subject, or the effective amount is effective to increase migration of CECs in the subject.

In one embodiment, the composition is in the form of an aqueous solution, a solid, an ointment, a gel, a liquid, a hydrogel, an aerosol, a mist, a polymer, a contact lens, a film, an emulsion, or a suspension, or said composition is administered topically, optionally the composition is topically administered to the eye of the subject, or said method does not comprise systemic administration or substantial dissemination to non-ocular tissue of the subject, or said method further comprises administering nerve growth factor (NGF) or vasoactive intestinal polypeptide (VIP).

In one embodiment, the composition is administered to the eye of the subject (a) less than 1, 2, 3, 4, 5, or 6 times per day; (b) about 1, 2, 3, 4, 5, 6, or 7 times per week; or (c) once daily, optionally, the composition is administered by the subject.

In one embodiment, the melanocortin receptor agonist for use further comprises detecting CECs of the subject before or after administration of the melanocortin receptor agonist, optionally wherein detecting CEC function comprises measuring corneal thickness with optical coherence tomography (OCT), or wherein detecting CECs of the subject comprises detecting the morphology, density, or number of CECs in the cornea of the subject.

In one embodiment, less than about 60%, 55%, 50%, 45%, 40%, 35%, 30%, 25%, 20%, 15%, 10%, 5%, 4%, 3%, 2%, 1%, 0.5%, 0.1%, 0.01%, 0.001%, or 0.0001% of the CECs in the subject's cornea are in the shape of a hexagon, or wherein none of the CECs in the subject's cornea are in the shape of a hexagon.

Other features and advantages of the invention will be apparent from the following description of the preferred embodiments thereof, and from the claims. Unless otherwise defined, all technical and scientific terms used herein have the same meaning as commonly understood by one of ordinary skill in the art to which this invention belongs. Although methods and materials similar or equivalent to those described herein can be used in the practice or testing of the present invention, suitable methods and materials are described below.

## DESCRIPTION OF THE DRAWINGS

FIGS. 1A-1D are images showing immunohistochemistry (IHC) staining of murine and human CECs with an antibody against the $\alpha$-MSH receptor (melanocortin 1 receptor or MC-1R). (A) Human corneal endothelial cell line (red: propidium iodide; green: MC-1R). (B) Human donor corneal endothelium (blue: DAPI; green: MC-1R). (C) Murine corneal endothelial cell line (red: propidium iodide; green: MC-1R). (D) Murine corneal endothelium (blue: DAPI; green: MC-1R).

FIG. 2 is a graph showing the effects of various concentrations of $\alpha$-MSH on recovery of scratch in a scratch test.

FIG. 3A is a series of images, and FIG. 3B is a graph showing effects of $\alpha$-MSH on reducing CEC apoptosis induced by inflammatory cytokines. There is a significant reduction in percentage of apoptotic cells with addition of various concentrations of $\alpha$-MSH.

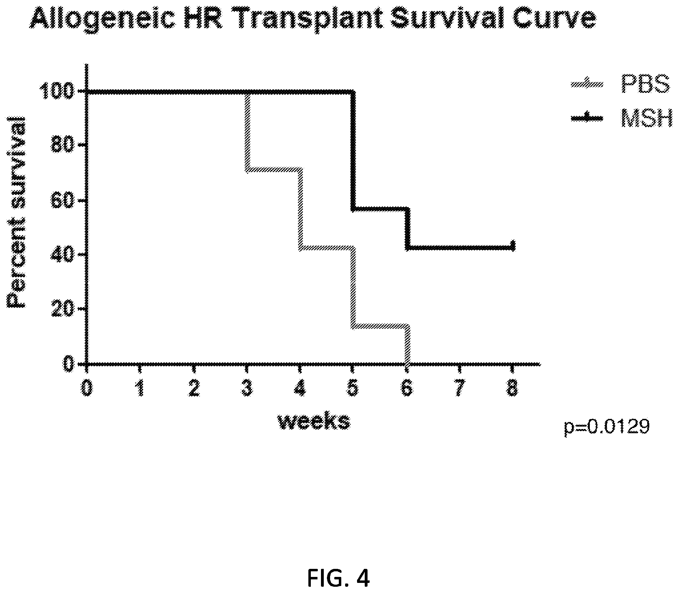

FIG. 4 is a graph showing the effect of $\alpha$-MSH on survival of allogeneic high-risk transplant. A subject is considered rejected, so that it is excluded from the calculations of survival, when the opacity score remains at least 3 for a continuous two week after week 2 of corneal transplant (n=7 in each group).

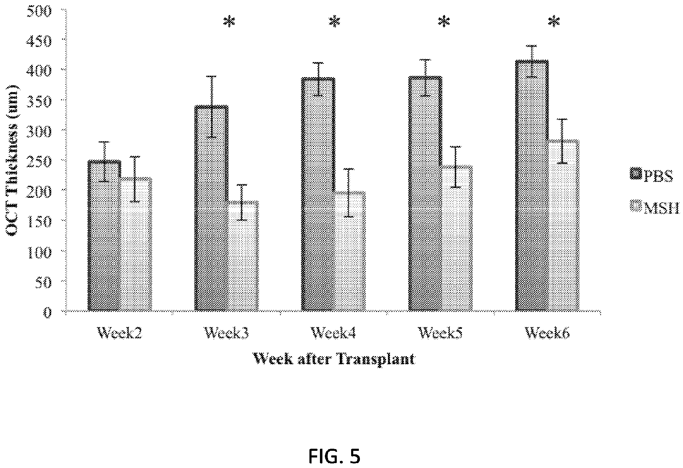

FIG. 5 is a graph showing the effect of $\alpha$-MSH on central corneal thickness in murine model after allogeneic high-risk transplant. The corneal pachymetry is evaluated via OCT, *p<0.05 (n=7 in each group).

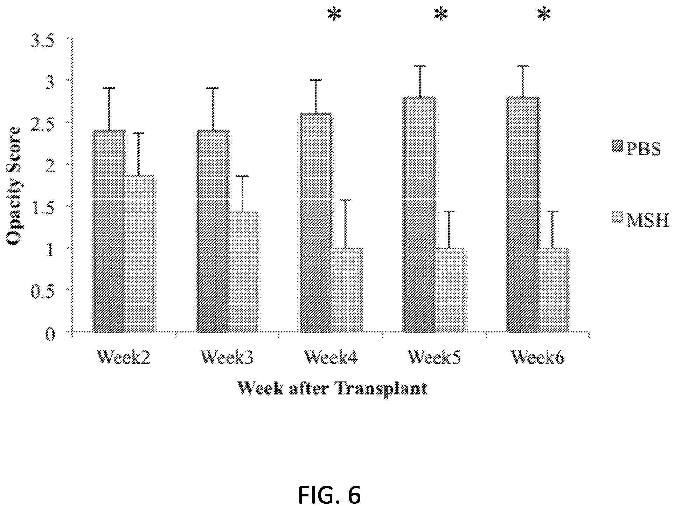

FIG. 6 is a graph showing the effect of $\alpha$-MSH on opacity scores in murine model after allogeneic high-risk transplant, *p<0.05 (n=7 in each group).

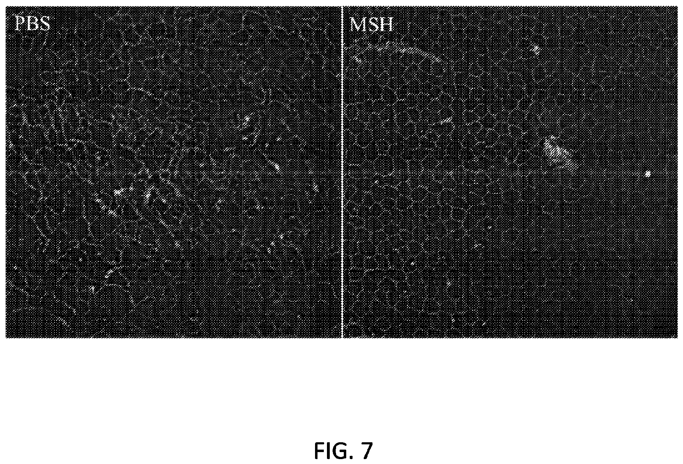

FIG. 7 is a set of confocal images showing CEC density and morphology, stained with ZO-1, 8-week after allogeneic high-risk transplant in murine model. PBS group and $\alpha$-MSH ($10^{-4}$ M), twice per week subconj injection.

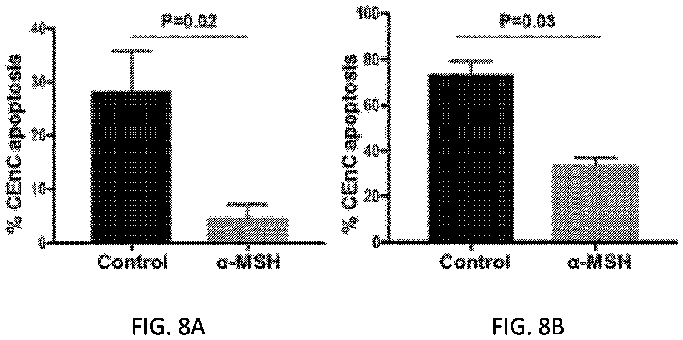

FIG. 8A is a graph showing the suppression effect of $\alpha$-MSH ($10^{-4}$ M) on CEC apoptosis in human corneal endothelium sheet under the stress of IFN-$\gamma$ (60 ng/ml). FIG. 8B is a graph showing the suppression effect of MSH ($10^{-4}$ M) on CEC apoptosis in human corneal endothelium sheet under the stress of H2O2 (1.4 mM for 2hr).

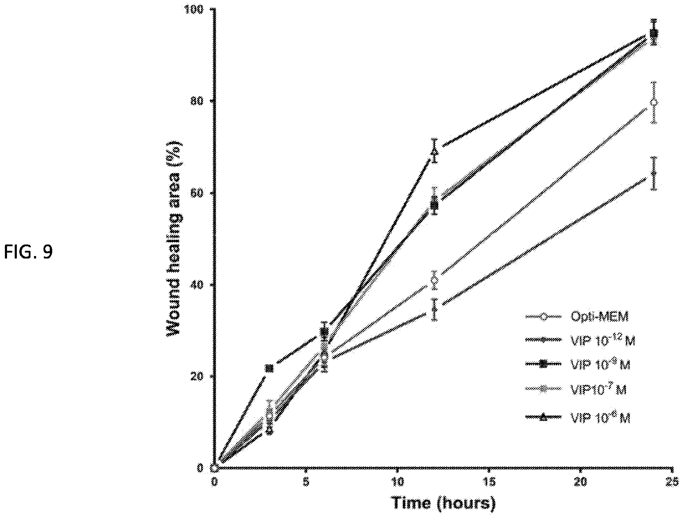

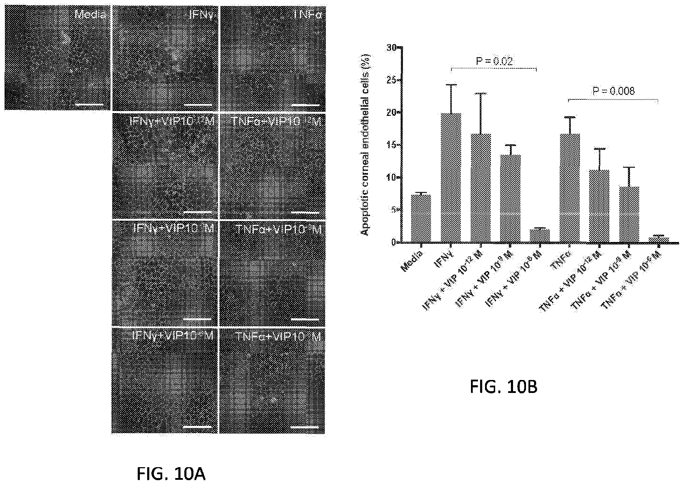

FIG. 9 is a graph showing that VIP treatment of human corneal endothelial cells accelerates corneal endothelial wound healing *in vitro* in a dose-dependent manner. Percentage of healed endothelial area compared to baseline in VIP-treated groups and in control. Human corneal endothelial cells treated with VIP $10^{-9}$, $10^{-7}$ and $10^{-6}$ M demonstrated significantly enhanced wound healing compared to the control group 12 and 24 hours after incubation compared with the control (P<0.05, Mann-Whitney test). A dose-dependent trend was observed in the effect of VIP. Error bars indicate mean $\pm$SEM. Each group consists of n=9, and data from one out of two independent experiments is shown. FIG. 10A is a set of micrographs and FIG. 10B is a graph showing that VIP suppresses IFN$\gamma$- and TNF$\alpha$-mediated corneal endothelial cell apoptosis. FIG 10A: Representative confocal micrographs showing naive C57BL/6 corneal cups incubated with either IFN$\gamma$ or TNF$\alpha$ with or without VIP ($10^{-12}$, $10^{-9}$, $10^{-6}$ M). After 18 hours of incubation, corneas were stained for zonula occluden-1 (ZO-1, green) and terminal deoxynucleotidyl transferase-mediated dUTP nick-end labeling assay (TUNEL, red) to visualize endothelial cell-to-cell junctions and apoptotic cells, respectively. The scale bars are equal to 100 $\mu$m (magnification $\times$40). FIG 10B: Bar diagram showing the percentages of apoptotic (TUNEL-positive) corneal endothelial cells incubated *ex vivo* with either IFN$\gamma$ or TNF$\alpha$ with different doses of VIP. VIP $10^{-6}$ M significantly suppresses IFN$\gamma$- and TNF$\alpha$-mediated corneal endothelial cell apoptosis (p=0.02 and 0.008, respectively; Mann-Whiney test). Data are presented as mean $\pm$SEM. Each group consists of n=5 corneas and data from one out of two independent experiments is shown.

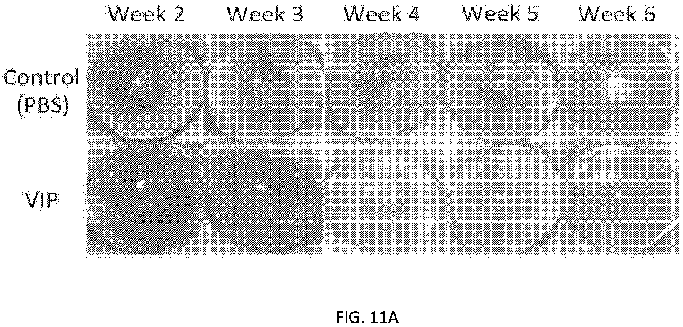

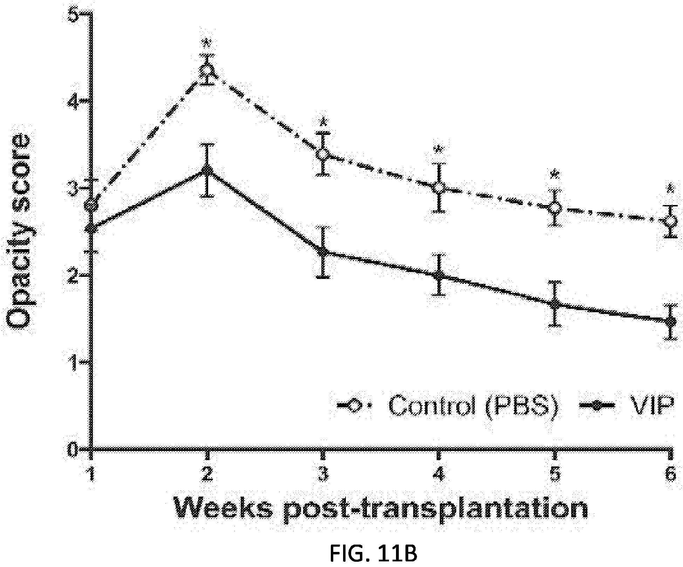

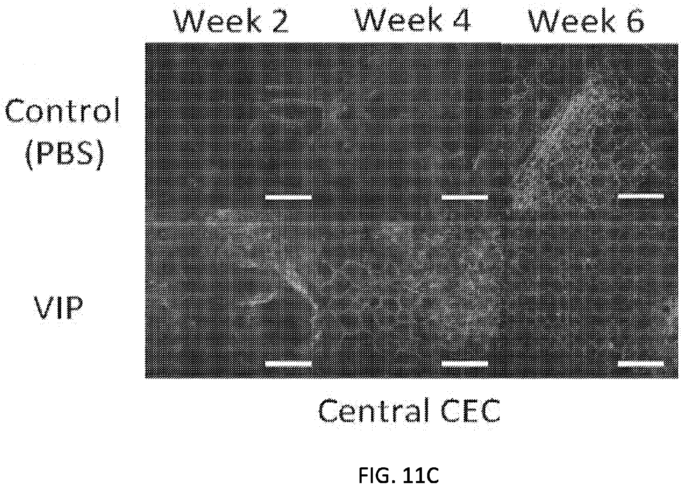

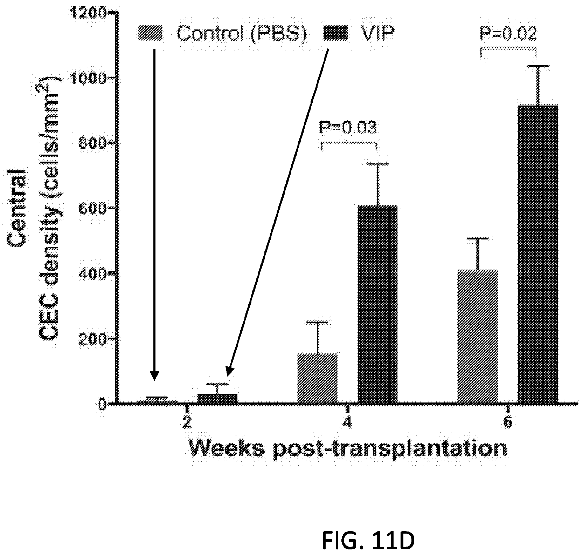

FIGs. 11A-D are images and graphs showing that VIP treatment decreases graft opacity and enhances corneal endothelial wound healing in a murine model of syngeneic corneal transplantation with endothelial injury. FIG. 11A: Representative slit-lamp images showing corneas at week 2-6 post-transplantation (magnification x25). FIG. 11B: Graft opacity scores were significantly decreased in VIP-treated group from week 2 to 6 post-transplantation compared with the control (P<0.05, n=15/group, Mann-Whitney test). FIG. 11C: Representative confocal micrographs of central area of transplanted corneas isolated from VIP-treated mice and the control groups at week 2, 4 and 6 after transplantation. To visualize corneal endothelial cell-to-cell junctions, corneas were stained with zonula occluden-1 (ZO-1, green). The scale bars are equal to 100 $\mu$m (magnification $\times$40). FIG. 11D: Bar diagram showing central endothelial cell density in VIP-treated corneas and controls at 2, 4 and 6 weeks post-transplantation. Data are presented as mean $\pm$SEM. Each group consists of *n*=6 corneas, and data from one out of two independent experiments is shown.

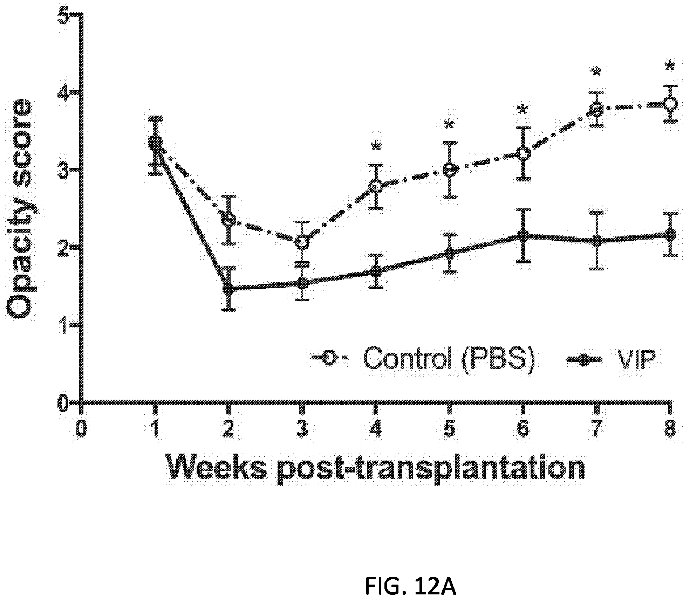

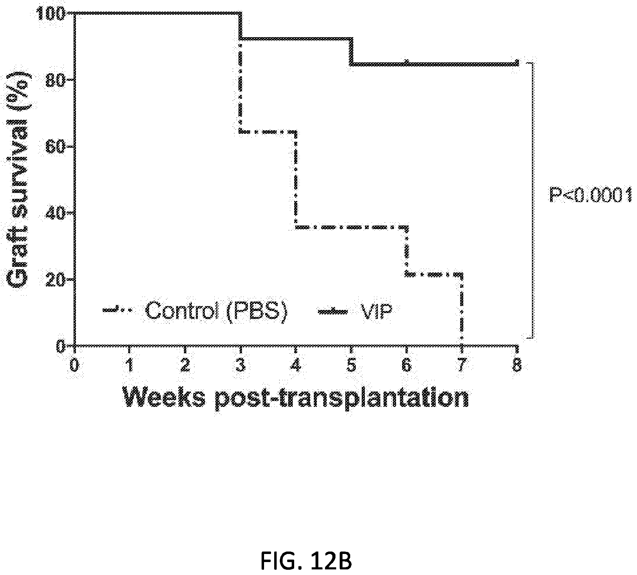

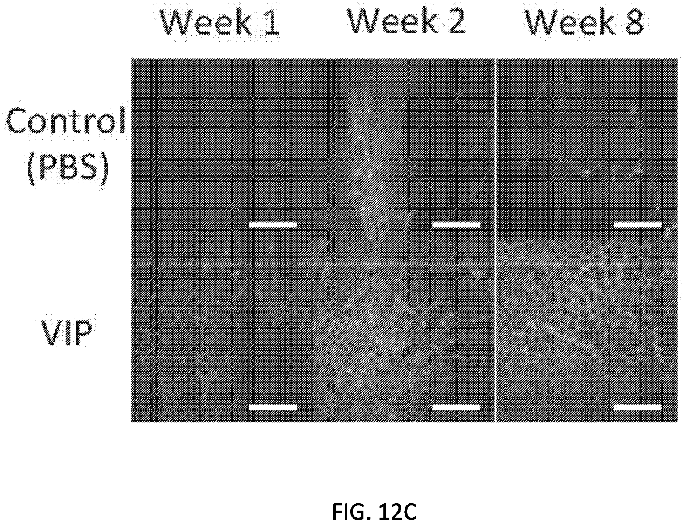

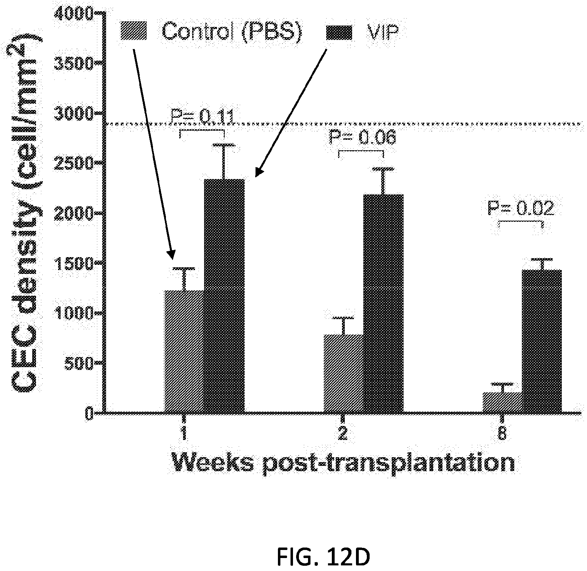

FIGs. 12A-D are graphs and images showing the effect of VIP treatment on high-risk corneal transplant survival. Animals underwent high-risk allogeneic corneal transplantation and received treatment with VIP at 1, 3, 5, 7 and 9 days after transplantation. FIG. 12A: VIP treatment significantly decreased graft opacity scores at 4 to 8 weeks post-transplantation (P<0.05, Mann-Whitney test). FIG. 12B: Weekly examination of grafts for 8 weeks demonstrated a significant increase in graft survival in VIP-treated mice compared with the controls (85% vs. 0%; hazard ratio 0.10, 95% CI 0.04-0.26, P<0.0001, Log rank test). Each group consists of *n*=14. FIG. 12C: Representative confocal micrographs of central area of transplanted corneas in VIP-treated mice and in controls at 1, 2 and 8 weeks post-transplantation. Corneal endothelial cell-to-cell junction were stained and visualized with zonula occluden-1 (ZO-1, green). The scale bars are equal to 100$\mu$m (magnification $\times$40). FIG. 12D: Bar diagram of central CEnC densities show significantly higher CEnC density in VIP-treated group compared with the control at 8 weeks post-transplantation (p=0.02, Man-Whitney test). Horizontal line represents the CEnC density of naïve age-matched C57BL/6 corneas. Each group consists of n=5 corneas. All data are presented as mean $\pm$SEM, and data from one out of two independent experiments is shown.

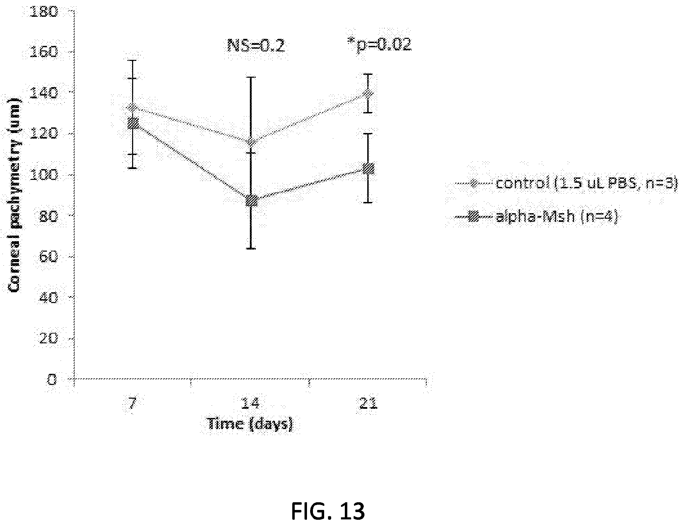

FIG. 13 is a graph showing the effect of $\alpha$-MSH on corneal thickness in murine model after syngeneic corneal transplantation, *p<0.05.

## DETAILED DESCRIPTION

The front part of the eye comprises the cornea, which is a transparent tissue. Corneal transparency is critical for normal vision. One of the important structures responsible for keeping the cornea transparent is CECs. These cells form a monolayer on the back surface of the cornea. Many different conditions are associated with damage to these cells which can result in corneal swelling (*e.g.,* edema) and reduced vision. Treatments to prevent or reduce CEC loss in various ocular and systemic conditions are needed. Corneal transplantation is often performed for those with significant reduction in CECs. Included herein are strategies, methods, and compositions that improve the survival, function, proliferation, and/or migration of these cells.

CECs are critical for normal vision. Without being bound by any scientific theory, CECs control the fluid and solute transport across the posterior surface of the cornea and actively maintain the cornea in the slightly dehydrated state which is required for optical transparency (Hassell et al. 2010 Exp Eye Res 91:326-35; Bourne 2003 Eye (Lond) 17:912-8). Therefore, any damage to these cells may lead to reduced vision. Despite their importance, the mechanisms controlling the structure and function of CECs are not well understood.

Aspects of the present subject matter relate to the identification of an entirely novel function for a neuropeptide, $\alpha$-MSH, in promoting survival and function of corneal endothelial cells. Therapeutic uses for this function are included herein.

Although nerves in the cornea have been shown to be involved in maintenance of corneal structure and function, *e.g.,* providing trophic support for corneal epithelium, their role/function with respect to corneal endothelium, a structurally and functionally different tissue compared to corneal epithelium, was unknown prior to the invention. In addition, the role of each specific nerve-derived molecule (neuropeptide) on corneal endothelium is unknown. More specifically, the effects of α-MSH on these cells were unknown before the discovery disclosed herein.

Surprisingly, some neuropeptides can improve CEC survival, and these neuropeptides can be used to treat a variety conditions to reduce CEC loss. Non-limiting uses of compositions provided herein include (but are not limited to) increasing CEC survival, proliferation, and/or migration in corneal tissue (*e.g.,* in a subject or during the storage of donor tissue), following ocular surgery such as corneal transplantation, and in subjects with a corneal injury, a corneal dystrophy (such as an anterior corneal dystrophy, a stromal corneal dystrophy, a posterior corneal dystrophy, corneal endothelial dystrophy, Fuchs endothelial dystrophy, congenital hereditary endothelial dystrophy, posterior polymorphous corneal dystrophy, or Schnyder crystalline corneal dystrophy), bullous keratopathy, an iridocorneal endothelial (ICE) syndrome, photokeratitis (*e.g.,* caused by sunlight reflection from sand, water, ice, or snow), neurotrophic keratophy, pseudoexfoliation syndrome, ocular hypertension, glaucoma, an ocular infection, a cataract, corneal endothelial cell loss due to contact lens wear, corneal endothelial cell loss due to aging, uveitis, intraocular inflammation, inflammatory disciform keratitis, diabetes, or dry eye disease. The compositions described herein are useful to improve or restore vision in a large number of people suffering from complications of CEC loss.

Aspects of the present subject matter relate to the discovery that α-MSH promotes CEC migration and proliferation, and inhibits CEC apoptosis. In various embodiments, α-MSH reduces cell loss in any condition associated with CEC loss. Fuchs dystrophy (also known as Fuchs' dystrophy) affects about 1% of the general population and there are no known treatments. About 100,000 corneal transplants are performed every year in the United States. The present subject matter includes methods of treating disorders such as (but not limited to) Fuchs endothelial dystrophy, corneal endothelial cell loss due to contact lens wear and aging, neurotrophic keratopathy, and pseudoexfoliation syndrome. Also included are compositions for use in methods, *e.g.,* storage or preservation solutions for donor cornea storage/maintenance (such as for eye banking), isolated endothelial cell (such as CEC) storage and culturing, and corneal tissues comprising endothelial cells (such as CECs) for use in endothelial keratoplasty procedures.

Human CECs are post-mitotic and exhibit poor regenerative capacity *in vivo* (Armitage et al. 2003 Investig Opthalmology Vis Sci. 2003 Aug 1;44(8):3326). CEC density decreases with increasing age, and the residual cells spread and enlarge (Yee et al. 1985 Curr Eye Res. 4(6):671-8). After corneal transplantation, the loss of CECs is accelerated (Bourne 2001 Cornea 20(6):560-9). Decreased endothelial cell density has been shown to be predictive of late endothelial failure, a key cause of graft failure in keratoplasty (Bourne 2001 Cornea 20(6):560-9).

In developed countries, CEC dysfunction is the principal indication for corneal transplantation (Peh et al. 2011 Transplantation 91(8):811-9). Furthermore, endothelial dysfunction is the most common cause of graft failure (Guilbert et al., 2013 Am J Ophthalmol 155(3):560-569.e2). Provided herein are strategies of using melanocortin receptor agonists to support CECs in both eye banking and corneal transplantation to improve CEC survival and reduce graft failure rates.

In eye banking, loss of CECs stored in Optisol is well established (Means et al. 1995 Arch Ophthalmol Jun 1;113(6):805). Studies have demonstrated that grafts with a lower CEC density, either preoperatively or postoperatively, have increased rates of late corneal endothelial failure (Bourne 2001 Cornea 20(6):560-9; Nishimura et al. 1999 Ophthalmology 106(10):1962-5). Improving CEC health prior to transplantation is particularly important in the current era of increased endothelial keratoplasty procedures, since these techniques entail more donor tissue manipulation than penetrating keratoplasty (Price et al. 2008 Ophthalmology 115(5):857-65).

While grafts performed in non-vascularized host beds or low-risk grafts enjoy a success rate of approximately 90%, in high-risk corneal transplantation (characterized by a vascularized and inflamed host bed) corneal graft rejection can exceed 50% (Dana et al. 2000 Cornea 19(5):625-43). In various embodiments, melanocortin receptor agonists demonstrate a protective effect on endothelium in an inflammatory microenvironment, *e.g.,* in subjects with both low and high risk of tissue rejection upon corneal transplantation.

In non-limiting examples, a neuropeptide (such as α-MSH, VIP, CGRP, and/or BDNF) is used to reduce the CEC loss in a condition as detailed below:

1. Corneal Transplantation

Corneal transplantation is well known to be associated with postoperative CEC loss. Here, a neuropeptide (such as α-MSH, VIP, CGRP, and/or BDNF) can be used to reduce this CEC loss after any form of corneal transplantation such as penetrating keratoplasty or endothelial keratoplasty. In addition, a neuropeptide (such as α-MSH, VIP, CGRP, and/or BDNF) can also be used to reduce the CEC loss associated with an episode of graft rejection.

### 2. Donor Cornea Storage (Eye Banking)

Storage of the donor cornea is associated with CEC loss. Here, a neuropeptide (such as $\alpha$-MSH, VIP, CGRP, and/or BDNF) can be used to reduce this natural CEC loss during storage.

### 3. Cataract Surgery or Other Intraocular Surgeries

All intraocular surgeries, including cataract surgery, glaucoma surgery, and intraocular lens implantation, are associated with CEC loss. When this loss is severe, it can lead to bullous keratopathy and other conditions associated with corneal edema. Here, a neuropeptide (such as $\alpha$-MSH, VIP, CGRP, and/or BDNF) can be used to reduce CEC loss after any intraocular surgery.

### 4. Fuchs endothelial dystrophy

This dystrophy is the most common corneal endothelial dystrophy, which can be associated with significant CEC loss. Here, a neuropeptide (such as $\alpha$-MSH, VIP, CGRP, and/or BDNF) can be used to reduce CEC loss over time or after any intraocular surgery in patients with Fuchs dystrophy, who are always at heightened risk of CEC loss.

### 5. Other Corneal Endothelial Dystrophies

A neuropeptide (such as $\alpha$-MSH, VIP, CGRP, and/or BDNF) can be used to reduce the CEC loss in patients with different corneal endothelial dystrophies, including congenital hereditary endothelial dystrophy.

### 6. Corneal Injuries

A neuropeptide (such as $\alpha$-MSH, VIP, CGRP, and/or BDNF) is useful to reduce CEC loss after any form mechanical, chemical, or physical injury. In addition, in patients with exposure of CEC to chemical materials, such as toxic substances, a neuropeptide (such as $\alpha$-MSH, VIP, CGRP, and/or BDNF) can be used to reduce the CEC loss. Furthermore, a neuropeptide (such as $\alpha$-MSH, VIP, CGRP, and/or BDNF) can also be used to reduce CEC loss in cases with surgical trauma to the corneal endothelium.

### 7. Intraocular Inflammation

Any form of intraocular inflammation, such as uveitis (autoimmune and infectious), can be associated with CEC loss. A neuropeptide (such as $\alpha$-MSH, VIP, CGRP, and/or BDNF) can be used to reduce the cell loss in subjects with uveitis.

### 8. Increased Intraocular Pressure

Increased intraocular pressure is known to cause CEC loss. A neuropeptide (such as $\alpha$-MSH, VIP, CGRP, and/or BDNF) can be used to reduce the cell loss due to increased intraocular pressure.

### 9. Corneal Infections

Corneal infections due to a variety of organisms, such as viral, bacterial, fungal, and acanthamoeba organisms, can be associated with CEC loss. A neuropeptide (such as $\alpha$-MSH, VIP, CGRP, and/or BDNF) can be used to reduce cell loss in subjects with corneal infections. In addition, in noninfectious complications of these infections, such as inflammatory disciform keratitis, a neuropeptide (such as $\alpha$-MSH, VIP, CGRP, and/or BDNF) can be used to reduce the CEC loss.

### 10. Contact Lens Wear

Contact lens wear has been known to be associated with CEC loss. A neuropeptide (such as $\alpha$-MSH, VIP, CGRP, and/or BDNF) can be used to reduce the cell loss.

### 11. Diabetes Mellitus

Diabetes may be associated with CEC loss. A neuropeptide (such as $\alpha$-MSH, VIP, CGRP, and/or BDNF) can be used to reduce cell loss in subjects with diabetes (*e.g.,* Type I or Type II diabetes).

12. Neurotrophic Keratopathy

It has been shown that neurotrophic keratopathy due to various reasons, including neurosurgically induced causes, can be associated with CEC loss. A neuropeptide (such as $\alpha$-MSH, VIP, CGRP, and/or BDNF) can be used to reduce the cell loss in subjects with neurotrophic keratopathy.

13. Dry Eye Disease

Dry eye disease can be associated with a significant CEC loss. A neuropeptide (such as $\alpha$-MSH, VIP, CGRP, and/or BDNF) can be used to reduce the CEC loss.

14. Pseudoexfoliation syndrome

Pseudoexfoliation syndrome can be associated with CEC loss. A neuropeptide (such as $\alpha$-MSH, VIP, CGRP, and/or BDNF) can be used to reduce cell loss associated in subjects who have pseudoexfoliation syndrome.

15. Aging

Aging is associated with progressive CEC loss over time. A neuropeptide (such as $\alpha$-MSH, VIP, CGRP, and/or BDNF) can be used to reduce cell loss that is associated with aging.

In certain embodiments, compositions provided herein may further comprise, or may comprise, consist essentially of, or consist of one or more neuropeptides (such as $\alpha$-MSH, VIP, CGRP, and/or BDNF).

Various compositions provided herein may comprise, consist essentially of, or consist of a melanocortin receptor agonist such as $\alpha$-MSH or an $\alpha$-MSH agonist. As used herein a "melanocortin receptor agonist" is a compound that binds to at least one melanocortin receptor resulting in signaling that occurs when melanocortin binds its receptor. An "$\alpha$-MSH agonist" is a melanocortin receptor agonist other than $\alpha$-MSH. In some embodiments, the amount of signaling that results from $\alpha$-MSH agonist binding to a receptor is greater than when $\alpha$-MSH binds the receptor, *i.e.* the melanocortin receptor agonist activates the receptor more than $\alpha$-MSH. In some embodiments, the receptor for $\alpha$-MSH is one or more melanocortin receptors. In certain embodiments, the one or more melanocortin receptors is/are $MC_1$, $MC_3$, $MC_4$, or $MC_5$, or any combination thereof. In various embodiments, the melanocortin receptor(s) does not comprise $MC_2$. In some embodiments, an $\alpha$-MSH agonist is an $\alpha$-MSH derivative. In certain embodiments, the $\alpha$-MSH derivative is a synthetic or natural compound that binds to one or more melanocortin receptors resulting in signaling that typically occurs when $\alpha$-MSH binds the one or more melanocortin receptors.

In some embodiments, the melanocortin receptor agonist (*e.g.,* $\alpha$-MSH or a derivative thereof) is native human $\alpha$-MSH (*e.g.,* has a wild-type amino acid sequence and structure), recombinant $\alpha$-MSH, a variant of $\alpha$-MSH comprising at least about 80%, 85%, or 90% sequence identity to native human $\alpha$-MSH, a fragment of $\alpha$-MSH (*e.g.,* comprising about 3, 4, 5, 6, 7, 8, 9, 10, 11, or 12 amino acids) that binds to a receptor for $\alpha$-MSH resulting in signaling that typically occurs when $\alpha$-MSH binds its receptor, or a molecule derived from $\alpha$-MSH (*e.g.,* that has $\alpha$-MSH activity, such as binding to a receptor for $\alpha$-MSH resulting in signaling that occurs when $\alpha$-MSH binds its receptor). In certain embodiments, the melanocortin receptor antagonist comprises a small molecule $\alpha$-MSH agonist. In some embodiments, $\alpha$-MSH is used (*e.g.,* is administered or is present in a composition) alone (*e.g.,* as a monotherapy). Alternatively, $\alpha$-MSH is used (*e.g.,* is administered or is present in a composition) in combination with other active agents.

Included herein are compositions for use in methods for treating or preventing CEC loss comprising an $\alpha$-MSH agonist (*e.g.,* in a corresponding embodiment of any method or composition disclosed herein that comprises $\alpha$-MSH), *e.g.,* a small molecule $\alpha$-MSH agonist. In various embodiments, the $\alpha$-MSH agonist binds at least one $\alpha$-MSH receptor (such as a melanocortin receptor).

Non-limiting examples of $\alpha$-MSH agonists are described in U.S. Patent No. 8,703,702 issued April 22, 2014 and U.S. Patent No. 7,169,603 issued January 30, 2007.

Receptor activation upon $\alpha$-MSH agonist binding can be confirmed using, *e.g.,* tests for binding to radioactive [35S]GTP$\gamma$S or tests for fluorescence resonance energy transfer (FRET) nanosensor.

Exemplary Conditions and Diseases

Provided herein are compositions for use in methods for the treatment of any condition or disease (*e.g.,* ocular condition or disease) that is associated with a loss of CECs, abnormal CEC morphology, and/or CEC dysfunction. In various embodiments, the condition comprises a disease, aging, an injury (*e.g.,* trauma), prolonged contact lens use (*e.g.,* use of contact lenses for at least about 6 to 12 hours per day for each of at least about 1, 2, 3, 4, 5, 6 or 7 days per week for at least about 0.5, 1, 2, 3, 4, 5, 6, 7, 8, 9, 10, 15, or 20 years), or surgical intervention (*e.g.,* a transplant, laser eye surgery, cataract surgery, glaucoma surgery, *etc*.). In some embodiments, the disease is not an inflammatory disease (*i.e.,* the disease is a non-inflammatory disease). In various embodiments, the disease is not an autoimmune diseasei (*i.e.,* the disease is a non-autoimmune disease). In some embodiments, the subject does not have an inflammatory disease. In certain embodiments, the subject does not have an autoimmune disease. In various embodiments, the disease comprises inflammation. In some embodiments, the disease is an inflammatory disease. In certain embodiments, the disease is an autoimmune disease.

An ocular inflammatory disease is a disease that includes aberrant inflammation in an eye. In various embodiments, the aberrant inflammation occurs where no trauma (*e.g.,* injury) has occurred. In some embodiments, the aberrant inflammation persists (*e.g.,* for more than 1 week) after damage from trauma has healed or would normally be expected to heal in a corresponding subject who does not have the inflammatory disease. In certain embodiments, the aberrant inflammation occurs where infection has occurred. In various embodiments, the aberrant inflammation persists (*e.g.,* for more than 1 week) after a pathogen that has caused an infection is cleared from the affected tissue (*e.g.,* due to clearance by the immune system and/or therapeutic intervention). In certain embodiments, the inflammation is chronic inflammation. In various embodiments, the aberrant inflammation is an increased response to an injury or antigen (*e.g.,* from a pathogen) compared to a corresponding subject who does not have the inflammatory disease. In some embodiments, the aberrant inflammation is an allergic reaction. In some embodiments, the inflammation is acute inflammation. An autoimmune disease is a disease in which a subject's immune system (*e.g.,* immune cells) attacks a component of the subject's body, such as one or more types of the subject's cells (*e.g.,* one or more types of ocular cells). In certain embodiments, ocular inflammation is observable and measurable visually. In some embodiments, ocular inflammation is clinically invisible, *i.e.* is only reflected by high expression levels of pro-inflammatory molecules (*e.g.,* pro-inflammatory cytokines such as interleukin-1 (IL-1), IL-12, and IL-18, tumor necrosis factor alpha (TNF-$\alpha$), interferon gamma (IFN-$\gamma$), and granulocyte-macrophage colony stimulating factor). In various embodiments, the pro-inflammatory molecules are sufficient to cause or indicate a "subclinical" (meaning clinically non-evident, *e.g.,* by visual inspection) disease.

As used herein, a "symptom" associated with a condition includes any clinical or laboratory manifestation associated with the condition, and is not limited to what the subject can feel or observe. In certain embodiments, the method described herein may include identifying a subject having one or more of these symptoms. Non-limiting examples of symptoms include CEC death, abnormal CEC morphology, and CEC dysfunction (*e.g.,* as evidenced by corneal edema).

"Treating" (or treatment of) a condition includes ameliorating at least one symptom of the particular condition, even if the underlying pathophysiology is not affected. In some embodiments, the condition is an injury (*e.g.*. trauma or a post-operative state). In certain embodiments, the condition is a disease. The efficacy of the treatment can be evaluated, e.g., as compared to a standard, *e.g.,* improvement in the value or quality of a parameter (*e.g.,* self-reported pain level, vision quality, CEC number, CEC morphology, cornea structure or clarity, and/or the existence of level of cornea edema) as compared to the value or quality of the parameter prior to treatment. As another example, the efficacy of treatment can be evaluated, *e.g.,* as compared to a standard, *e.g.,* slowing progression of the condition as compared to a usual time course for the condition in a cohort that has not been treated or compared to historical data on progression. Treating a condition also includes slowing its progress; and/or relieving the condition, *e.g.,* causing regression of the condition. In some embodiments, the progressive worsening (*e.g.,* the increasing intensity) of a symptom is slowed, reduced, or halted.

"Preventing" (or prevention of) a condition in a subject includes stopping a condition from occurring in the subject, who may be at risk of (*e.g.,* predisposed to) the condition but has not yet been diagnosed as having it. Preventing a condition also includes delaying the onset of the condition. The efficacy of the prevention can be evaluated, *e.g.,* as compared to a standard, *e.g.,* delaying onset of the condition as compared to a usual time of onset for the condition in a cohort that has not been treated or compared to historical data on condition onset. In various embodiments, the condition is a disease. In some embodiments, the condition comprises CEC loss (*i.e.* due to cell death), dysfunction, and/or abnormal morphology.

As used herein and depending on the context in which it is used, "therapeutically effective amount" refers to an amount which is effective in reducing, eliminating, treating, preventing or controlling a symptom of a disorder or condition. In various embodiments, the symptom comprises CEC loss (*i.e.* due to cell death), dysfunction, and/or abnormal morphology. The term "controlling" is intended to refer to all processes wherein there may be a slowing, interrupting, arresting, or stopping of the progression of a condition described herein, but does not necessarily indicate a total elimination of all symptoms, and is intended to include prophylactic treatment.

Corneal Dystrophy

Aspects of the present subject matter provide compositions for use in methods for treating a corneal dystrophy. Corneal dystrophies are eye disorders (often genetic and progressive) in which abnormal material often accumulates in the cornea. *See, e.g.,* The Corneal Dystrophy Foundation, 2016 What is Corneal Dystrophy? available at www.corneal-dystrophyfoundation.org/what-is-corneal-dystrophy. In some embodiments, a subject with a corneal dystrophy does not have a symptom such as vision impairment, corneal edema, or eye discomfort (*i.e.*, the corneal dystrophy is "asymptomatic"). In certain embodiments, a subject with a corneal dystrophy has vision impairment, corneal edema, and/or eye discomfort. In various embodiments, the age of onset and/or specific symptoms vary among the different forms of corneal dystrophy. In some embodiments, the corneal dystrophy affects both eyes (*i.e.,* is bilateral). Alternatively, the corneal dystrophy affects one eye. In certain embodiments, the corneal dystrophy does not comprise symptoms in other areas of the body. In various embodiments, at least 1, 2, 3, or 4, cousins, aunts, uncles, grandparents, parents, and/or siblings of the subject has a corneal dystrophy. In some embodiments, the corneal dystrophy is inherited as an autosomal dominant trait. In certain embodiments, the corneal dystrophy is inherited as an autosomal recessive trait.

The cornea comprises five layers: (1) the Epithelium, which is the outermost, protective layer of the cornea; (2) the Bowman's membrane, which is tough and difficult to penetrate, further protecting the eye; (3) the Stroma, which is the thickest layer of the cornea, comprising water, collagen fibers and other connective tissue components that help give the cornea strength, elasticity and clarity; (4) the Descemet membrane, which is a thin, strong inner layer that also acts as a protective layer; and (5) the Endothelium, which the innermost layer comprising CECs. Corneal dystrophies typically include the accumulation of abnormal material in one or more of layers (1), (2), (3), (4), or (5), and/or between layers of the cornea. In some embodiments, the material causes the cornea to lose its transparency. In certain embodiments, the material causes loss of vision or blurred vision.

In certain embodiments, the presence of a corneal dystrophy is found incidentally during a routine eye examination. In various embodiments, a diagnosis is confirmed by a clinical evaluation, a detailed patient history, and/or one or more of a variety of tests, such as a slit lamp examination [in which a special microscope (slit lamp) allows a physician to view the eye through high magnification]. Some specific corneal dystrophies can be diagnosed with molecular genetic tests even before symptoms such as vision impairment, corneal edema, or eye discomfort develop.

In various embodiments, a treatment regimen or composition provided herein is administered in a subject who has asymptomatic corneal dystrophy (*e.g.,* the subject does not have a symptom such as vision impairment, corneal edema, or eye discomfort). In some embodiments, a treatment or composition is administered as a monotherapy or in conjunction with another treatment. Non-limiting examples of treatments for corneal dystrophies include eye drops, ointments, lasers, and corneal transplant. In various embodiments, ocular surgery is performed. In various embodiments, a corneal transplant (*e.g.,* a keratoplasty) may be performed, and a neuropeptide is administered before, during, and/or after the surgery. In some embodiments, the neuropeptide comprises CGRP, BDNF, VIP, and/or α-MSH).In certain embodiments, a corneal transplant (*e.g.,* a keratoplasty) may be performed, and a melanocortin receptor agonist such as α-MSH is administered before, during, and/or after the surgery. Alternatively or in addition, a corneal transplant (*e.g.,* a keratoplasty) may be performed, and VIP is administered before, during, and/or after the surgery.

In certain embodiments, a neuropeptide (such as CGRP, BDNF, and/or VIP) and/or a melanocortin receptor agonist (which may also be a neuropeptide such as α-MSH) is used to treat a corneal dystrophy that is associated with abnormal CEC morphology or density. In various embodiments, a melanocortin receptor agonist such as α-MSH is used to treat a corneal dystrophy that is associated with abnormal CEC morphology or density. In some embodiments, VIP is used to treat a corneal dystrophy that is associated with abnormal CEC morphology or density. Non-limiting examples of corneal dystrophies include posterior corneal dystrophies (*e.g.,* congenital hereditary endothelial corneal dystrophy, Fuchs endothelial corneal dystrophy, posterior polymorphous corneal dystrophy, and Schnyder crystalline corneal dystrophy).

Various embodiments relate to the treatment of posterior corneal dystrophies, especially dystrophies that comprise a CEC abnormality. Posterior corneal dystrophies affect the innermost layers of the cornea such as the Descemet membrane and/or the endothelium. In some embodiments, a posterior corneal dystrophy progresses or has progressed to affect another layer (*e.g.,* one or more other layers) or all layers of the cornea. Non-limiting examples include congenital hereditary endothelial corneal dystrophy, Fuchs endothelial corneal dystrophy, posterior polymorphous corneal dystrophy, and Schnyder crystalline corneal dystrophy.

Types of congenital hereditary corneal dystrophy or congenital-hereditary-endothelial-dystrophy (CHED) include:

> 1) an autosomal dominant variant that presents after 1 year of age and is progressive (CHED1); and
> 2) an autosomal recessive variant that presents at birth and is stationary (CHED2). *See, e.g.,* Trief et al. 2016 Review of Ophthalmology, (available from www.reviewofophthalmology.com/article/congenital-hereditary-endothelial-dystrophy).

In some embodiments, a subject with CHED1 was born with a clear cornea and has developed clouding over the first 1, 2, or 3 years of life. In certain embodiments, a subject with CHED1 complains of epiphora and/or photophobia. In various embodiments, a subject with CHED1 does not have nystagmus. In certain embodiments, a subject with CHED1 has small flakes, spots, and/or irregular white areas throughout the stroma of 1 or 2 eyes. In various embodiments, a subject with CHED1 comprises multiple abnormal endothelial layers and/or microvilli.

In some embodiments, a subject with CHED2 has corneal clouding from birth with accompanying nystagmus. In certain embodiments, a subject with CHED2 does not complain of epiphora or photophobia. In various embodiments, the corneal clouding in a subject with CHED2 is more advanced than is typical for a subject with CHED1, and results in a worse visual acuity than is typical for a subject with CHED1.

Recently, the international classification of corneal dystrophies (IC3D) has been revised to eliminate CHED1 from classification. *See, e.g.,* Weiss et al. IC3D classification of corneal dystrophies--edition 2. Cornea 2015;34:117-59. Additionally, the autosomal recessive CHED (CHED2) was renamed CHED. However, CHED1 and CHED2 may be referred to as discussed herein to distinguish different classes of subjects with congenital hereditary corneal dystrophy.

In some embodiments, a subject with congenital hereditary corneal dystrophy has corneal edema. In certain embodiments, a subject with congenital hereditary corneal dystrophy has corneal clouding, and the clouding is diffuse (limbus to limbus) or the clouding is relatively uniform. In some embodiments, the cornea is cloudy (even milky) but the iris is visible. In certain embodiments, aside from corneal abnormalities, the structure of a subject's affected eye (or eyes) is normal.

In various embodiments, the subject does not have congenital glaucoma. In some embodiments, a subject has high intraocular pressure secondary to thick pachymetry (*e.g.,* the cornea is more than 625 $\mu$m thick). In various embodiments, congenital hereditary corneal dystrophy occurs concomitantly with glaucoma. In certain embodiments, a subject with congenital hereditary corneal dystrophy comprises a mutation at chromosome 20 loci 20p13. In various embodiments, the subject comprises a mutation in a gene encoding solute carrier family 4, sodium borate transporter member 11 (SLC4A11). This gene encodes a sodium borate transporter. In some embodiments, the mutation comprises a coding region mutation in the SLC4A11 gene.

In various embodiments, the subject comprises Fuchs endothelial corneal dystrophy (also known as, *e.g.,* Fuchs endothelial dystrophy and Fuchs dystrophy). In some embodiments, Fuchs dystrophy develops in a subject during middle age (*e.g.,* when the subject is about 25 to 45 or 30 to 40 years old). In certain embodiments, the subject does not have vision impairment, corneal edema, or eye discomfort initially. Fuchs dystrophy is characterized by CEC dysfunction. In Fuchs dystrophy CECs deteriorate (and may die). In various embodiments, the cornea fills with water and swells (*i.e.,* corneal edema occurs). In some embodiments, the swelling worsens and blurred vision occurs that is worse in the morning, but gradually improves throughout the day. In certain embodiments, tiny blisters form on the cornea. In various embodiments, tiny blisters on the cornea rupture and causing pain. In some embodiments, a subject has a gritty or sandy feeling within the eye (foreign body sensation), is abnormally sensitive to light, and/or see a glare or halo when looking at lights. In certain embodiments, as the disease progresses, vision no longer improves during the day and significant vision loss may occur. In various embodiments, the subject receives a corneal transplant.

In some embodiments, an early-onset variant of Fuchs endothelial dystrophy is associated with a mutation in the COL8A2 gene, which encodes a protein that is part of type VIII collagen. Type VIII collagen is a major component of the Descemet membrane. In certain embodiments, a subject with a COL8A2 gene mutation and Fuchs endothelial dystrophy has an abnormal Descemet membrane. In various embodiments, CECs die in such subjects, leading and/or contributing to vision problems.

In various embodiments, a subject has posterior polymorphous dystrophy. In some embodiments, posterior polymorphous dystrophy presents at birth (with clouding of the cornea). In certain embodiments, posterior polymorphous dystrophy presents later during life and is characterized by lesions affecting the endothelium. In various embodiments, the subject does not have a symptom such as vision impairment, corneal edema, or eye discomfort. In some embodiments, effects on the cornea are slowly progressive. In certain embodiments, both eyes are affected, but one eye is more severely affected than the other (*i.e.,* the posterior polymorphous dystrophy is asymmetric). In various embodiments, a subject with posterior polymorphous dystrophy has swelling (edema) of the stroma, an abnormal sensitivity to light (photophobia), decreased vision, and/or the sensation of foreign material in the eye. In some embodiments, the subject has increased intraocular pressure.

Compositions provided herein are useful in methods for the prevention and/or treatment, *e.g.,* reduction in a clinical manifestation, of any symptom or type of corneal dystrophy (including CEC loss).

<u>Bullous Keratopathy</u>

In certain embodiments, a subject has bullous keratopathy. Bullous keratopathy is a pathological condition in which small vesicles, or bullae, are formed in the cornea due to endothelial dysfunction. In a healthy cornea, CECs keep the tissue from excess fluid absorption. In various embodiments, then affected by some reason, such as Fuchs dystrophy or a trauma during ocular surgery (such as a transplant or cataract removal), CECs may suffer mortality or damage. In some embodiments, when endothelial cell counts drop too low, excess fluid moves anterior into the stroma and epithelium, resulting in swelling of the cornea. In certain embodiments, as fluid accumulates between the basal epithelium cells, blister like formations form (bullae) and they undergo painful ruptures releasing their fluid content. In various embodiments, these malformations disrupt vision and create pain sensations. Compositions provided herein are useful in methods for the prevention and/or treatment of bullous keratopathy.

Iridocorneal Endothelial (ICE) Syndrome

In some embodiments, a subject has an ICE syndrome. ICE syndromes are a spectrum of diseases characterized by slowly progressive abnormalities of the corneal endothelium and features including corneal edema, iris distortion, and secondary angle-closure glaucoma. Compositions provided herein are useful in methods for the prevention and/or treatment of any type or symptom of ICE syndrome (including CEC loss).

Neurotrophic Keratopathy

In certain embodiments, a subject has neurotrophic keratopathy. Neurotrophic keratopathy is a disease characterized by decreased corneal sensitivity and poor corneal healing. *See, e.g.,* Graham (2016) Neurotrophic Keratopathy, Medscape, available at emedicine.medscape.com/article/1194889-overview. In various embodiments, this disorder leaves the cornea susceptible to injury and decreases reflex tearing. In some embodiments, a subject comprises epithelial breakdown. In certain embodiments, a subject has a corneal ulceration, infection, melting, and/or perforation secondary to poor healing. Prognostic indicators in neurotrophic keratopathy include the degree of sensory loss, the duration of the condition, and the presence of other ocular surface disease. Neurotrophic keratopathy can occur in any age group. In some embodiments, the subject is at least about 0.5, 1, 5, 10, 15, 20, 25, 30, 35, 40, 45, 50, 55, 60, 65, 70, 75, 80, 85, 90, or 95 years old. In certain embodiments, the subject has corneal hypesthesia. In various embodiments, the neurotrophic keratopathy results during or following a herpetic infection of the cornea, a surgery for trigeminal neuralgia, or a surgery for acoustic neuroma. In some embodiments, the neurotrophic keratopathy has been caused by a herpes simplex or herpes zoster infection, or leprosy. In certain embodiments, a subject shows rose bengal staining of the inferior palpebral conjunctiva, has decreased tear breakup time, has increased mucous viscosity, and has punctate epithelial fluorescein staining. In various embodiments, the subject has an epithelial defect (such as an oval defect in the superior cornea), a defect surrounded by a rim of loose epithelium (optionally, the edges are smooth and rolled), stromal swelling with folds in the Descemet membrane, and/or inflammation in the anterior chamber.

Compositions provided herein are useful in methods for the prevention and/or treatment of any type or symptom of neurotrophic keratopathy (including CEC loss).

Pseudoexfoliation Syndrome

In some embodiments, a subject has pseudoexfoliation syndrome. Pseudoexfoliation syndrome is an aging-related systemic disease manifesting itself primarily in the eyes which is characterized by the accumulation of microscopic granular amyloid-like protein fibers. In certain embodiments, the subject is at least about 65, 70, 75, 80, 85, 90, or 95 years old. In various embodiments, the subject self-identifies as being of Scandinavian descent. In some embodiments, the buildup of protein clumps blocks normal drainage of the eye fluid called the aqueous humor. In certain embodiments, this block of normal drainage causes an increase in ocular pressure leading to glaucoma and loss of vision.

In various embodiments, a subject has no specific symptoms. In some embodiments, a subject complains of lessened visual acuity or a change in the perceived visual field. In certain embodiments, such changes are secondary to or different from symptoms normally associated with cataracts or glaucoma.

In various embodiments, the pseudoexfoliation syndrome comprises tiny microscopic white or grey granular flakes that are clumps of proteins within the eye. In some embodiments, the flakes are visible during an examination of the lens of an eye by an ophthalmologist or optometrist. In certain embodiments, the white fluffy material is seen in many tissues both ocular and extraocular: in the anterior chamber structures, trabecular meshwork, central disc, zonular fibres, anterior hyaloid membrane, pupillary and anterior iris, trabecula, and occasionally the cornea. In various embodiments, the flakes are widespread.

In some embodiments, pseudoexfoliation syndrome includes the flakes becoming enmeshed in a "spongy area" known as the trabecular meshwork and block its normal functioning. In certain embodiments, the flakes interact with degenerative changes in the Schlemm's canal and the juxtacanalicular area. In various embodiments, the blockage leads to greater-than-normal elevated intraocular pressure. In some embodiments, a subject's optic nerve is damaged. In certain embodiments, pseudoexfoliation syndrome is associated with a weakening of structures within the eye which help hold the eye's lens in place, called lens zonules.

Compositions provided herein are useful in methods for the prevention and/or treatment of any type or symptom of pseudoexfoliation syndrome (including CEC loss).

Increased Intraocular Pressure

In various embodiments, a subject has been diagnosed with or is characterized by comprising increased intraocular pressure, such as any type of glaucoma or ocular hypertension.

In some embodiments, a subject has ocular hypertension. As used herein, the term "ocular hypertension" refers to any situation in which the pressure inside the eye, called intraocular pressure, is higher than normal. Eye pressure may be measured in, *e.g.,* millimeters of mercury (mm Hg). Normal eye pressure ranges from 10-21 mmHg. Ocular hypertension comprises an eye pressure of greater than 21 mmHg. *See, e.g.,* WebMD, Ocular Hypertension, available at www.webmd.com/eye-health/occular-hypertension#3-4. In certain embodiments, a subject with ocular hypertension may have an eye pressure of at least about 21.5 mmHg, 22 mmHg, 22.5 mmHg, 23 mmHg, 23.5 mmHg, 24 mmHg, or 24.5 mmHg.

In various embodiments, ocular hypertension is commonly defined as a condition comprising (i) intraocular pressure of greater than 21 mm Hg is measured in one or both eyes at two or more time points (*e.g.,* on different dates, *e.g.,* at least about 1, 2, 3, 4, 5, 6, or 12 months apart ); (ii) a normal appearance of the optic nerve; (iii) no sign of glaucoma evident on visual field testing; and (iv) no sign of another ocular disease. In some embodiments, the pressure inside the eye is measured using an instrument such as a tonometer. In certain embodiments, a subject with ocular hypertension is observed more closely than the general population for the onset of glaucoma.

In various embodiments, increased intraocular pressure results from another eye condition. In some embodiments, ocular hypertension inside the eye is caused by an imbalance in the production and drainage of fluid in the eye (aqueous humor). For example, the channels that normally drain the fluid from inside the eye do not function properly.

Compositions provided herein are useful in methods for the prevention and/or treatment of any symptom or type of ocular hypertension (including CEC loss).

In some embodiments, the subject has glaucoma. Glaucoma results in damage to the optic nerve and vision loss, especially if left untreated. In certain embodiments, the glaucoma is open-angle glaucoma. In various embodiments, the glaucoma is closed-angle glaucoma. In some embodiments, the glaucoma is normal-tension glaucoma. Typically, open-angle glaucoma develops slowly over time and there is no pain. In some embodiments, side vision begins to decrease followed by central vision resulting in blindness if not treated. In certain embodiments, closed-angle glaucoma can present gradually or suddenly. In various embodiments, the sudden presentation involves severe eye pain, blurred vision, a mid-dilated pupil, redness of the eye, and nausea.

Non-limiting examples of risk factors for glaucoma include increased pressure in the eye, a family history of the condition, migraines, high blood pressure, and obesity. In some embodiments, for eye pressure a value of greater than 21 mmHg or 2.8 kPa is often used with higher pressures leading to a greater risk. However, a subject may have high eye pressure for years and never develop damage. Conversely, optic nerve damage may occur with normal pressure, known as normal-tension glaucoma.

Compositions provided herein are useful in methods for the prevention and/or treatment of any symptom or type of glaucoma (including CEC loss).

Keratitis

In various embodiments, a subject has keratitis. As used herein, keratitis is a condition in which a cornea becomes inflamed. Non-limiting examples of symptoms associated with keratitis include pain, impaired eyesight, photophobia, red eye, and a 'gritty' sensation. Examples of keratitis include acute epithelial keratitis, nummular keratitis, interstitial keratitis, disciform keratitis, neurotrophic keratitis, mucous plaque keratitis, herpes simplex keratitis, herpes zoster keratitis, bacterial keratitis, fungal keratitis, acanthamoebic keratitis, onchocercal keratitis, superficial punctate keratitis, ulcerative keratitis, exposure keratitis, photokeratitis, and contact lens acute red eye.

In some embodiments, the keratitis comprises inflammatory disciform keratitis. For example, the inflammatory disciform keratitis may result from a viral infection such as a herpes simplex virus (HSV) infection. In certain embodiments, an HSV infection induces inflammation of the corneal endothelium in a condition known as HSV endotheliitis. In various embodiments, HSV endotheliitis comprises secondary inflammation caused by the virus and/or direct infection of CECs. In various embodiments, HSV endotheliitis comprises endothelial dysfunction and stromal edema and/or opacity. In some embodiments, a subject receives corneal transplantation in the form of either full-thickness penetrating keratoplasty or anterior lamellar keratoplasty to restore corneal clarity.

Compositions provided herein are useful in methods for the prevention and/or treatment of any symptom or type of keratitis, as well as CEC loss associated with any treatment for keratitis.

Diabetes Mellitus

In certain embodiments, the subject has diabetes mellitus (DM). DM, commonly referred to as diabetes, is a group of metabolic diseases in which there are high blood sugar levels over a prolonged period. In various embodiments, the subject has frequent urination, increased thirst, and increased hunger. In some embodiments, the subject has heart disease, chronic kidney failure, foot ulcers, has had a stroke, and/or has damage to the eyes. Exemplary long-term complications relate to the damage to blood vessels, such as small blood vessels in the eyes, kidneys, and nerves. In certain embodiments, the subject has damage to the eyes such as diabetic retinopathy. In various embodiments, the subject has had gradual vision loss and/or blindness. In various embodiments, a subject has type 1 diabetes. In certain embodiments, a subject has type 2 diabetes.

Compositions provided herein are useful in methods for the prevention and/or treatment of vision and/or CEC loss associated with any type of diabetes.

Intraocular Inflammation and Uveitis

In various embodiments, a subject has intraocular inflammation, such as uveitis. Non-limiting examples of causes of intraocular inflammation include infection (*e.g.,* a viral, fungal, or parasitic infection), rheumatoid arthritis, Gout, and injuries to the eye. In some embodiments, the cause is unknown. Exemplary symptoms of intraocular inflammation include eye redness, blurred vision, perceiving floaters (small dark or blurry dots or shapes that may appear to move), decreased vision, and light sensitivity.

Uveitis is swelling and irritation of the uvea, the middle layer of the eye. In various embodiments, the uveitis comprises anterior uveitis (which involves inflammation in the front part of the eye). In some embodiments, the uveitis comprises iritis. In certain embodiments, the uveitis affect one or two of the subject's eyes. In various embodiments, the uveitis comprises posterior uveitis. In some embodiments, the uveitis affects the choroid, which is a layer of blood vessels and connective tissue in the middle part of the eye. In certain embodiments, the uveitis comprises choroiditis. In various embodiments, the uveitis comprises chorioretinitis. In various embodiments, the uveitis comprises pars planitis. In some embodiments, the subject is male. In certain embodiments, the subject is female. Uveitis can occur at any age. In various embodiments, the subject is under the age of 95, 85, 80, 75, 70, 65, 60, 55, 50, 45, 40, 35, 30, 25, 20, 15, 10, 5, 1, or 0.5 years old.

In various embodiments, the uveitis is autoimmune uveitis. As used herein "autoimmune uveitis" refers to uveitis that is caused or associated with an autoimmune disorder. In certain embodiments, the uveitis is associated with or caused by an autoimmune disorder such as rheumatoid arthritis or ankylosing spondylitis. In some embodiments, the subject has Crohn's disease and/or multiple sclerosis. Non-limiting examples of autoimmune uveitis symptoms include redness of the eye; blurred vision; photophobia; sensitivity to light; irregular pupil; eye pain; floaters, which are dark spots that appear to float in the visual field; headaches; dilated ciliary vessels; presence of cells and flare in the anterior chamber; keratic precipitates ("KP") on the posterior surface of the cornea; a hypopyon; pigment deposits on the lens; a festooned pupil on dilation of pupil; busacca nodules (inflammatory nodules located on the surface of the iris); synechia; and photopsia or seeing flashing lights.

Compositions provided herein are useful in methods for the prevention and/or treatment of any symptom or type of intraocular inflammation (including CEC loss), including any symptom or type of uveitis (e.g., such as autoimmune uveitis or uveitis from an infection), including CEC loss.

Dry Eye Disease and Related Disorders

In some embodiments, the subject has Dry Eye Disease (DED). DED is a multifactorial disorder of the tears and ocular surface that results in symptoms of discomfort, visual disturbance, and tear film instability, with potential damage to the ocular surface. In some embodiments, it is accompanied by increased osmolarity of the tear film and inflammation of the ocular surface (Lemp MA. Report of the National Eye Institute/Industry Workshop on clinical trials in dry eyes. CLAO J 1995;21:221-2). For a more detailed definition, see The definition and classification of dry eye disease: report of the Definition and Classification Subcommittee of the International Dry Eye WorkShop. Ocular Surface. 2007 Apr;5(2):75-92. DED and related diseases may be caused or exacerbated by, *e.g.,* autoimmune and environmental conditions as well as any activity that decreases the rate of blinking. DED and related diseases may also be caused by decreased tear production or a change in tear composition that results in inadequate lubrication of the eye. Contact lens use, eye surgery, and eye injury can induce DED. In certain embodiments, DED occurs as a consequence of aging and hormonal changes. DED can occur at any age. In various embodiments, the subject is at least about 0.5, 1, 5, 10, 15, 20, 25, 30, 35, 40, 45, 50, 55, 60, 65, 70, 75, 80, 85, 90, or 95 years old.

Non-limiting examples and related disorders of DED include, but are not limited to, keratoconjunctivitis sicca (KCS), Sjögren syndrome (SS), Sjögren syndrome associated keratoconjunctivitis sicca, non-Sjögren syndrome associated keratoconjunctivitis sicca, keratitis sicca, sicca syndrome, xerophthalmia, tear film disorder, decreased tear production, aqueous tear deficiency (ATD), meibomian gland dysfunction (MGD), and evaporative loss. In some embodiments, a subject is identified as suffering from DED or a related disorder by detecting a sign or symptom selected from the group consisting of dry, scratchy, stingy, itchy, burning or pressured sensations, irritation, pain, redness, inflammation, discharge, and excessive eye watering. In certain embodiments, a subject is identified as suffering from DED or a related disorder if their tear composition is insufficient for proper eye tissue lubrication. In various embodiments, a method of therapy for DED inhibits or reduces the severity of at least one of sign or symptom of DED.

In some embodiments, a subject is at risk of developing DED. Subjects at risk of developing DED include subjects who are taking antihistamines, nasal decongestants, tranquilizers, certain blood pressure medicines, Parkinson's medications, birth control pills and/or anti-depressants; subjects with a skin disease on or around the eyelids can result in dry eye; subjects suffering from a disease of the glands in the eyelids (such as meibomian gland dysfunction); subjects who are pregnant; female subjects who are on hormone replacement therapy (such as estrogen and/or progesterone); subjects who have had the refractive surgery known as laser-assisted *in situ* keratomileusis (LASIK); subjects who have suffered from a chemical or thermal burn on the membrane lining the eyelids and covering the eye; subjects afflicted with allergies; subjects afflicted with an immune system disorder (such as Sjögren's syndrome, lupus, or rheumatoid arthritis); subjects who have had an eye infection; subjects who have had ocular exposure to an irritant such as a chemical fume or tobacco smoke; and subjects with exposure keratitis.

Non-limiting examples of DED symptoms include pain (such as stinging or burning of the eye); ulcers or scars on the cornea; decrease tolerance for dry environments; reduced vision; a sandy or gritty feeling as if something is in the eye; episodes of excess tears following very dry eye periods; a stringy discharge from the eye; redness of the eye; episodes of blurred vision; heavy eyelids; inability to cry when emotionally stressed; uncomfortable contact lenses; decreased tolerance of reading, working on the computer, or any activity that requires sustained visual attention; and eye fatigue.

Compositions provided herein are useful in methods for the treatment of any type or symptom of DED or disorder that is related to DED as indicated above (including CEC loss).

Corneal Injury

As used herein, a "corneal injury" comprises a wound to the cornea. In some embodiments the wound comprises an injury to the outer surface of the cornea. In certain embodiments, the wound comprises an injury (*e.g.,* a deep injury) such as a full-thickness or partial thickness (*e.g.,* penetrating at least 5, 10, 15, 20, 25, 30, 35, 40, 45, 50, 55, 60, 65, 70, 75, 80, 85, 90, or 95% of the way through a part of the cornea) lacerations (*e.g.,* cuts or tears) or punctures. Non-limiting examples of injuries to the outer surface of the cornea include injuries from abrasions (such as scratches or scrapes on the surface of the cornea, *e.g.,* from trauma or a foreign body such as sand or dust), lacerations, punctures, chemical injuries (*e.g.,* caused by a fluid or gas comprising a toxic agent that contacts the eye), contact lens problems (*e.g.,* overuse, poor fit, or a sensitivity to a contact lens care solution), ultraviolet light (*e.g.,* from sunlight, a sun lamp, snow or water reflections, or arc-welding), or an infection.

Compositions provided herein are useful in methods for the treatment of any type or symptom of corneal injury (including CEC loss).

CEC Loss Associated with Surgery

Compositions provided herein are useful in methods of increasing CEC survival, proliferation, and/or migration following ocular surgery, such as intraocular surgery. There are many types of intraocular surgeries. Any type of intraocular surgery can result in CEC loss. In various embodiments, a neuropeptide (such as $\alpha$-MSH, VIP, CGRP, and/or BDNF) is used to prevent and/or treat CEC loss associated with any intraocular surgery. In certain embodiments, a melanocortin receptor agonist such as $\alpha$-MSH is used to prevent and/or treat CEC loss associated with any intraocular surgery. In some embodiments, VIP is used to prevent and/or treat CEC loss associated with any intraocular surgery.

In various embodiments, the ocular surgery comprises Descemet stripping (sometimes referred to as "descemetorhexis"). Descemet stripping may comprise (i) the removal of at least a portion (*e.g.,* at least about 10%, 20%, 30%, 40%, 50%, 60%, 70%, 80%, 90%, or all) of the Descemet membrane (sometimes referred to as "Descemet's membrane"), as well as CECs that are attached to the removed membrane; or (ii) the removal of CECs that are attached to the Descemet membrane, *e.g.,* without substantial removal of the Descemet membrane. In certain embodiments, Descemet stripping consists of or consists essentially of (i) the removal of at least a portion (*e.g.,* at least about 10%, 20%, 30%, 40%, 50%, 60%, 70%, 80%, 90%, or all) of the Descemet membrane, as well as CECs that are attached to the removed membrane; or (ii) the removal of CECs that are attached to the Descemet membrane, *e.g.,* without substantial removal of the Descemet membrane. The Descemet membrane is the basement membrane that lies between the corneal proper substance (also called the stroma), and the endothelial layer of the cornea. In some embodiments, Descemet stripping is not followed by endothelial keratoplasty, and a subject's CECs (*e.g.,* peripheral CECs, which are along the outer edge of the cornea's endothelial layer, or CECs from a region of the Descemet membrane that has not been stripped) repopulate the cornea to form a new layer of CECs where the Descemet membrane and/or preexisting CECs were removed. Compositions provided herein are useful in methods for increasing the growth, survival, and/or migration of a subject's CECs after Descemet stripping.

In some embodiments, the ocular surgery comprises a cornea transplant such as penetrating keratoplasty (PK) or endothelial keratoplasty (EK). In certain embodiments, PK comprises the removal of a full-thickness section of tissue a diseased or injured cornea, and replacement of the tissue with donor cornea tissue (*e.g.,* a matching section of donor tissue). In various embodiments, the tissue is removed using either a surgical cutting instrument (such as a trephine) or laser (such as a femtosecond laser). Typically, the donor tissue is sutured into place.

In various embodiments, the ocular surgery comprises EK. For example, the CECs may be the subject's CECs and/or donor CECs. In various embodiments, EK selectively replaces the diseased (*i.e.* endothelial) layer of the cornea with donor tissue, leaving healthy areas or layers intact [for example the innermost layer of the cornea (endothelium) is replaced and the overlying healthy corneal tissue is left intact other than, optionally, an incision]. In some embodiments, EK comprises Descemet Stripping Endothelial Keratoplasty (DSEK), Descemet Membrane Endothelial Keratosplaty (DMEK), Descemet Membrane Endothelial Transfer (DMET), or Descemet Stripping Automated Endothelial Keratoplasty (DSAEK).

DSEK is a CEC replacement procedure comprising Descemet stripping (which includes removal of the corneal endothelium comprising CECs) followed by replacement of the corneal endothelium (comprising CECs) with a graft comprising a donor's endothelium, Descemet membrane, and some part of the stroma. In some embodiments, the graft comprises the back 20-30% of the donor cornea.

DMEK comprises Descemet stripping followed by replacement of the corneal endothelium with donor graft comprising a thin layer (e.g., about 5% or less of corneal thickness) of donor tissue. For example, the donor tissue in DMEK comprises, consists essentially of, or consists of donor CECs attached the donor's Descemet membrane or a portion thereof. In certain embodiments, the graft has been peeled off the back of the donor cornea.

In various embodiments, the ocular surgery comprises DMET. DMET comprises Descemet stripping followed by administration of a graft comprising a thin layer (e.g., about 5% or less of corneal thickness) of donor tissue that is mostly free floating in the anterior chamber of the cornea. In some embodiments, the graft is attached only to the corneal incision site. In certain embodiments, the donor tissue in DMET comprises, consists essentially of, or consists of donor CECs attached the donor's Descemet membrane or a portion thereof.

In various embodiments, the ocular surgery comprises DSAEK. DSAEK is a partial thickness cornea transplant procedure that involves selective removal of the patient's Descemet membrane and endothelium, followed by transplantation of donor corneal endothelium in addition to donor corneal stroma. The transplanted tissue is approximately 100-200 microns thick. If the endothelium of the graft makes contact with any surgical instruments, it will be damaged and the graft may fail; therefore, the surgical procedure is designed to avoid contacting the donor endothelium. A tunneled corneoscleral incision is created, the recipient endothelium and Descemet membrane is removed, the graft is folded and inserted (*e.g.,* with non-coapting forceps, wich are forceps that do not meet at the tips), and an air bubble is placed in the anterior chamber to support graft adherence.

In various embodiments, the ocular surgery comprises the injection of donor CECs into the cornea of a subject. For example, the CECs may be injected to replace CECs that have died in a subject's corneal endothelium, and/ to replace CECs that were removed by Descemet stripping.

In some embodiments, the ocular surgery comprises phototherapeutic keratectomy (PTK). PTK is a type of eye surgery that uses a laser to treat various ocular disorders by removing tissue (such as CECs) from the cornea. Common indications for PTK are corneal dystrophies, scars, opacities, and bullous keratopathy.

Aspects of the present subject matter relate to reducing CEC loss in subjects who have glaucoma, as well as CEC loss resulting from surgery to great glaucoma. Alternatively or in addition, compositions herein are useful in methods for increasing the number of CECs in subjects who have glaucoma or who have received surgery for glaucoma. In some embodiments, the surgery comprises laser surgery, such as trabeculoplasty or iridotomy. In various embodiments, the surgery comprises invasive surgery such as trabeculectomy or the implantation of a glaucoma drainage device. In certain embodiments, the surgery comprises canaloplasty. Compositions provided herein are useful in methods for preventing or treating CEC loss associated with any surgical treatment for glaucoma.

In some embodiments, the surgery comprises cataract surgery. In various embodiments, cataract surgery is an operation that removes the cloudy lens and replaces it with a clear artificial lens (*i.e.,* an intraocular lens (IOL)). *See, e.g.,* Boyd (2016) Cataract Surgery American Academy of Ophthalmology available at www.aao.org/eye-health/diseases/-what-is-cataract-surgery. Compositions provided herein are useful in methods for preventing or treating CEC loss associated with any surgical treatment for cataracts.

Any type of intraocular surgery can result in CEC loss. A melanocortin receptor agonist such as $\alpha$-MSH can be used and/or treat CEC loss in all these conditions. In various embodiments, a neuropeptide (such as $\alpha$-MSH, VIP, CGRP, and/or BDNF) is used and/or treat CEC loss in all these conditions. In certain embodiments, the ocular surgery comprises laser eye surgery. Non-limiting examples of laser eye surgery include laser-assisted *in situ* keratomileusis, as well as procedures to change eye color (*e.g.,* from brown to blue).

Exemplary Neuropeptides

$\alpha$-MSH is generated from a precursor hormone called pro-opiomelanocortin (POMC) (Eipper and Mains (1980) Endocr Rev 1 (1): 1-27). This molecule serves as the source for several peptide hormones such as adrenocorticotrophin (ACTH), α-MSH, β-MSH and γ-MSH, and the endogenous opioids including β-endorphin. α-MSH is a tridecapeptide which, upon proteolytic cleavage, is generated from its precursor ACTH. Non-limiting descriptions relating to α-MSH are provided in Luger and Brzoska (2007) Ann Rheum Dis 66 Suppl 3:iii52-5.

An exemplary (non-limiting) amino acid sequence of the human POMC preprotein is available in public databases, *e.g.,* under National Center for Biotechnology Information (NCBI) Accession No. NP_001306134.1 and UniProt Accession No. P01189, and is as follows:

MPRSCCSRSGALLLALLLQASMEVRGWCLESSQCQDLTTESNLLECIRACKPDLSAE

TPMFPGNGDEQPLTENPRKYVMGHFRWDRFGRRNSSSSGSSGAGQKREDVSAGEDC

GPLPEGGPEPRSDGAKPGPREGKRSYSMEHFRWGKPVGKKRRPVKVYPNGAEDESA

EAFPLEFKRELTGQRLREGDGPDGPADDGAGAQADLEHSLLVAAEKKDEGPYRMEH

FRWGSPPKDKRYGGFMTSEKSQTPLVTLFKNAIIKNAYKKGE

In the amino acid sequence above, positions 1 to 26 correspond to the signal peptide; positions 27 to 102 correspond to the N-terminal peptide of pro-piomelanocortin (NPP); positions 77 to 87 correspond to γ-MSH; positions 105 to 134 correspond to a potential peptide; positions 138 to 176 correspond to corticotropin; positions 138 to 150 correspond to α-MSH; positions 156 to 176 correspond to corticotropin-like intermediary peptide; positions 179 to 267 correspond to lipotropin beta; positions 179 to 234 correspond to lipotropin gamma; positions 217-234 correspond to β-MSH; positions 237 to 267 correspond to β-endorphin; and positions 237 to 241 correspond to met-enkephalin.

The amino acid sequence of human α-MSH is as follows: SYSMEHFRWGKPV (SEQ ID NO: 1). The International Union of Pure and Applied Chemistry (IUPAC) name for α-MSH is *N*-acetyl-L-seryl-L-tyrosyl-L-seryl-L-methionyl-L-α-glutamyl-L-histidyl-L-phenylalanyl-L-arginyl-L-tryptophylglycyl-L-lysyl-L-prolyl-L-valinamide. α-MSH is also known as:

α-melanocortin,
α-melanotropin,
α-intermedin,
Ac-Ser-Tyr-Ser-Met-Glu-His-Phe-Arg-Trp-Gly-Lys-Pro-Val-NH$_2$,
Ac-DL-Ser-DL-Tyr-DL-Ser-DL-Met-DL-Glu-DL-His-DL-Phe-DL-Arg-DL-Trp-Gly-DL-Lys-DL-Pro-DL-Val-NH2, and
[acetyl]-SYSMEHFRWGKPV-[NH2].
The PubChem CID for human α-MSH is 16132636.

An exemplary (non-limiting) nucleotide sequence that encodes the human POMC preprotein is available from public databases under NCBI Accession No. NM_001319205.1 and is as follows (the start and stop codons are bolded and underlined):

GTTCTAAGCGGAGACCCAACGCCATCCATAATTAAGTTCTTCCTGAGGGCGAGCG
GCCAGGTGCGCCTTCGGCAGGACAGTGCTAATTCCAGCCCCTTTCCAGCGCGTCT
CCCCGCGCTCGTCCCCGTCTGGAAGCCCCCTCCCACGCCCCGCGGCCCCCTT
CCCCTGGCCCGGGGAGCTGCTCCTTGTGCTGCCGGGAAGGTCAAAGTCCCGCGCC
CACCAGGAGAGCTCGGCAAGTATATAAGGACAGAGGAGCGCGGGACCAAGCGG
CGGCGAAGGAGGGGAAGAAGAGCCGCGACCGAGAGAGGCCGCCGAGCGTCCCC
GCCCTCAGAGAGCAGCCTCCCGAGACAGGGGTCCCACCAATCTTGTTTGCTTCTG
CAGAGCCTCAGCCTGCCTGGAAG**ATG**CCGAGATCGTGCTGCAGCCGCTCGGGGG
CCCTGTTGCTGGCCTTGCTGCTTCAGGCCTCCATGGAAGTGCGTGGCTGGTGCCT
GGAGAGCAGCCAGTGTCAGGACCTCACCACGGAAAGCAACCTGCTGGAGTGCAT
CCGGGCCTGCAAGCCCGACCTCTCGGCCGAGACTCCCATGTTCCCGGGAAATGG
CGACGAGCAGCCTCTGACCGAGAACCCCCGGAAGTACGTCATGGGCCACTTCCG
CTGGGACCGATTCGGCCGCCGCAACAGCAGCAGCAGCGGCAGCAGCGGCGCAG
GGCAGAAGCGCGAGGACGTCTCAGCGGGCGAAGACTGCGGCCCGCTGCCTGAGG
GCGGCCCCGAGCCCCGCAGCGATGGTGCCAAGCCGGGCCCGCGCGAGGGCAAGC
GCTCCTACTCCATGGAGCACTTCCGCTGGGGCAAGCCGGTGGGCAAGAAGCGGC
GCCCAGTGAAGGTGTACCCTAACGGCGCCGAGGACGAGTCGGCCGAGGCCTTCC
CCCTGGAGTTCAAGAGGGAGCTGACTGGCCAGCGACTCCGGGAGGGAGATGGCC
CCGACGGCCCTGCCGATGACGGCGCAGGGGCCCAGGCCGACCTGGAGCACAGCC
TGCTGGTGGCGGCCGAGAAGAAGGACGAGGGCCCCTACAGGATGGAGCACTTCC
GCTGGGGCAGCCCGCCCAAGGACAAGCGCTACGGCGGTTTCATGACCTCCGAGA
AGAGCCAGACGCCCCTGGTGACGCTGTTCAAAAACGCCATCATCAAGAACGCCT
ACAAGAAGGGCGAG**TGA**GGGCACAGCGGGGCCCCAGGGCTACCCTCCCCCAGG
AGGTCGACCCCAAAGCCCCTTGCTCTCCCCTGCCCTGCTGCCGCCTCCCAGCCTG
GGGGGTCGTGGCAGATAATCAGCCTCTTAAAGCTGCCTGTAGTTAGGAAATAAA

ACCTTTCAAATTTCACATCCACCTCTGACTTTGAATGTAAACTGTGTGAATAAAG
TAAAAATACGTAGCCGTCAAATAACAGC (SEQ ID NO: 2)

NGF is also known as beta-NGF. An exemplary (non-limiting) amino acid sequence of human nerve growth factor (NGF) is available in public databases, e.g., under UniProt Accession No. P01138, and is as follows:

MSMLFYTLITAFLIGIQAEPHSESNVPAGHTIPQAHWTKLQHSLDTALRRARSAPAAA
IAARVAGQTRNITVDPRLFKKRRLRSPRVLFSTQPPREAADTQDLDFEVGGAAPFNRT
HRSKRSSSHPIFHRGEFSVCDSVSVWVGDKTTATDIKGKEVMVLGEVNINNSVFKQY
FFETKCRDPNPVDSGCRGIDSKHWNSYCTTTHTFVKALTMDGKQAAWRFIRIDTAC
VCVLSRKAVRRA (SEQ ID NO: 3)

An exemplary (non-limiting) nucleotide sequence that encodes human NGF is available from public databases under GenBank Accession No. CR541855.1 and is as follows (the start and stop codons are bolded and underlined):

**<u>ATG</u>**TCCATGTTGTTCTACACTCTGATCACAGCTTTTCTGATCGGCATACAGGCGG
AACCACACTCAGAGAGCAATGTCCCTGCAGGACACACCATCCCCCAAGCCCACT
GGACTAAACTTCAGCATTCCCTTGACACTGCCCTTCGCAGAGCCCGCAGCGCCCC
GGCAGCGGCGATAGCTGCACGCGTGGCGGGGCAGACCCGCAACATTACTGTGGA
CCCCAGGCTGTTTAAAAAGCGGCGACTCCGTTCACCCCGTGTGCTGTTTAGCACC
CAGCCTCCCCGTGAAGCTGCAGACACTCAGGATCTGGACTTCGAGGTCGGTGGTG
CTGCCCCCTTCAACAGGACTCACAGGAGCAAGCGGTCATCATCCCATCCCATCTT
CCACAGGGGCGAATTCTCGGTGTGTGACAGTGTCAGCGTGTGGGTTGGGGATAA
GACCACCGCCACAGACATCAAGGGCAAGGAGGTGATGGTGTTGGGAGAGGTGA
ACATTAACAACAGTGTATTCAAACAGTACTTTTTTGAGACCAAGTGCCGGGACCC
AAATCCCGTTGACAGCGGGTGCCGGGGCATTGACTCAAAGCACTGGAACTCATA
TTGTACCACGACTCACACCTTTGTCAAGGCGCTGACCATGGATGGCAAGCAGGCT
GCCTGGCGGTTTATCCGGATAGATACGGCCTGTGTGTGTGTGCTCAGCAGGAAGG
CTGTGAGAAGAGCC**<u>TGA</u>** (SEQ ID NO: 4)

An exemplary (non-limiting) amino acid sequence of human VIP is available in public databases, e.g., under UniProt Accession No. P01282, and is as follows:

MDTRNKAQLLVLLTLLSVLFSQTSAWPLYRAPSALRLGDRIPFEGANEPDQVSLKEDI
DMLQNALAENDTPYYDVSRNARHADGVFTSDFSKLLGQLSAKKYLESLMGKRVSS

NISEDPVPVKRHSDAVFTDNYTRLRKQMAVKKYLNSILNGKRSSEGESPDFPEELEK
(SEQ ID NO: 5)

An exemplary (non-limiting) nucleotide sequence that encodes human VIP is available from public databases under GenBank Accession No. M36634.1 and is as follows (the start and stop codons are bolded and underlined):

GGTCAGCTCCAAAACAATCCGGAACGGCCAGCTCCGGGGGAGCACGACTGGGCG
AGAGGCACAGAA**ATG**GACACCAGAAATAAGGCCCAGCTCCTTGTGCTCCTGACT
CTTCTCAGTGTGCTCTTCTCACAGACTTCGGCATGGCCTCTTTACAGGGCACCTTC
TGCTCTCAGGTTGGGTGACAGAATACCCTTTGAGGGAGCAAATGAACCTGATCA
AGTTTCATTAAAAGAAGACATTGACATGTTGCAAAATGCATTAGCTGAAAATGA
CACACCCTATTATGATGTATCCAGAAATGCCAGGCATGCTGATGGAGTTTTCACC
AGTGACTTCAGTAAACTCTTGGGTCAACTTTCTGCCAAAAAGTACCTTGAGTCTC
TTATGGGAAACGTGTTAGCAGTAACATCTCAGAAGACCCTGTACCAGTCAAAC
GTCACTCAGATGCAGTCTTCACTGACAACTATACCCGCCTTAGAAAACAAATGGC
TGTAAAGAAATATTTGAACTCAATTCTGAATGGAAAGAGGAGCAGTGAGGGAGA
ATCTCCCGACTTTCCAGAAGAGTTAGAAAAA**TGA**TGAAAAAGACCTTGGAGCA
AAGCTGATGACAACTTCCCAGTGAATTCTTGAAGGAAATGATACGCAACATAA
TTAAATTTTAGATTCTACATAAGTAATTCAAGAAAACAACTTCAATATCCAAACC
AAATAAAAATATTGTGTTGTGAATGTTGTGATGTATTCTAGCTAATGTAATAACT
GTGAAGTTTACATTGTAAATAGTATTTGAGAGTTCTAAATTTTGTCTTTAACTCAT
AAAAAGCCTGCAATTTCATATGCTGTATATCCTTTCTAACAAAAAAATATATTTT
AATGATAAGTAATGCTAGGTTAATCCAATTATATGAGACGTTTTTGGAAGAGTAG
TAATAGAGCAAAATTGATGTGTTTATTTATAGAGTGTACTTAACTATTCAGGAGA
GTAGAACAGATAATCAGTGTGTCTAAATTTGAATGTTAAGCAGATGGAATGCTGT
GTTAAATAAACCTCAAAATGTCTAAGATAGTAACAATGAAGATAAAAAGACATT
CTTCCAAAAAGATTTTCAGAAAATATTATGTGTTTCCATATTTTATAGGCAACCTT
TATTTTTAATGGTGTTTTAAAAAATCTCAAATTTGGATTGCTAATCACCAAAGGCT
CTCTCCTGATAGTCTTTCAGTTAAGGAGAACGACCCCTGCTTCTGACACTGAAAC
TTCCCTTTCTGCTTGTGTTAAGTATGTGTAAAATGTGAAGTGAATGAAACACTCA
GTTGTTCAATAATAAATATTTTTGCCATAATGACTCAGAATATTGCTTTGGTCATA
TGAGCTTCCTTCTGTGAAATACATTTTGGAGACACAACTATTTTTCCAAAATAATT
TTAAGAAATCAAAGAGAGAAATAAAGACCTTGCTTATGATTGCAGATAAAAAA
AAAAAAAAAAAAAAAAAAAAAAAAAAAAAAA  (SEQ ID NO: 6)

CGRP is a member of the calcitonin family of peptides, which in humans exists in two forms, $\alpha$-CGRP and $\beta$-CGRP. $\alpha$-CGRP (also known as Calcitonin gene-related peptide 1 or CALCA) is a 37-amino acid peptide and is formed from the alternative splicing of the calcitonin/CGRP gene. $\beta$-CGRP differs in three amino acids (in humans) and is encoded in a separate gene.

An exemplary (non-limiting) amino acid sequence for human CALCA is available in public databases, *e.g.,* under UniProt Accession No. P06881, and is as follows:

MGFQKFSPFLALSILVLLQAGSLHAAPFRSALESSPADPATLSEDEARLLLAALVQNY
VQMKASELEQEQEREGSRIIAQKRACDTATCVTHRLAGLLSRSGGVVKNNFVPTNV
GSKAFGRRRRDLQA (SEQ ID NO: 7)

An exemplary (non-limiting) nucleotide sequence that encodes human CGRP is available from public databases under NCBI Accession No. NM_001033953.2 and is as follows (the start and stop codons are bolded and underlined):

CCGCCGCTGCCACCGCCTCTGATCCAAGCCACCTCCCGCCAGGTGAGCCCCGAGA
TCCTGGCTCAGAGAGGTGTC**ATG**GGCTTCCAAAAGTTCTCCCCCTTCCTGGCTCT
CAGCATCTTGGTCCTGTTGCAGGCAGGCAGCCTCCATGCAGCACCATTCAGGTCT
GCCCTGGAGAGCAGCCCAGCAGACCCGGCCACGCTCAGTGAGGACGAAGCGCGC
CTCCTGCTGGCTGCACTGGTGCAGGACTATGTGCAGATGAAGGCCAGTGAGCTG
GAGCAGGAGCAAGAGAGAGAGGGCTCCAGAATCATTGCCCAGAAGAGAGCCTG
TGACACTGCCACCTGTGTGACTCATCGGCTGGCAGGCTTGCTGAGCAGATCAGGG
GGTGTGGTGAAGAACAACTTTGTGCCCACCAATGTGGGTTCCAAAGCCTTTGGCA
GGCGCCGCAGGGACCTTCAAGCC**TGA**GCAGCTGAATGACTCAAGAAGGTCACAA
TAAAGCTGAACTCCTTTTAATGTGTAATGAAAGCAATTTGTAGGAAAGGCTCCAT
GGAAGACATACATATAGGCATCCTTCTTGATACTGAAAACTATCTTCTTTGTTTG
AAAGGAACTATTGCTAAATGCAGAACAAGCTCATTGCAGTTACCTATTGTGCATC
TTTTTAAATACTTGATTATGTAACCATAAATCTGACAGCATGTCTCATTGGCTTAT
CTGGTAGCAAATCTAGGCCCCGTCAGCCACCCTATTGACATTGGTGGCTCTGCTA
AACCTCAGGGGGACATGAAATCACTGCCTCTTGGGCATCTGGGGACACATGGTA
ATGCTGTGCCTTGACAGAAGTATTTGTTTAAAGAAATGTCAATGCTGTCATTTGT
GAACTCTATCAAAATTAAAAATGTATTTTCTATACCCTTCA  (SEQ ID NO: 8)

An exemplary (non-limiting) amino acid sequence of human BDNF is available in public databases, *e.g.,* under UniProt Accession No. P23560, and is as follows:

MTILFLTMVISYFGCMKAAPMKEANIRGQGGLAYPGVRTHGTLESVNGPKAGSRGL
TSLADTFEHVIEELLDEDQKVRPNEENNKDADLYTSRVMLSSQVPLEPPLLFLLEEYK
NYLDAANMSMRVRRHSDPARRGELSVCDSISEWVTAADKKTAVDMSGGTVTVLEK
VPVSKGQLKQYFYETKCNPMGYTKEGCRGIDKRHWNSQCRTTQSYVRALTMDSKK
RIGWRFIRIDTSCVCTLTIKRGR (SEQ ID NO: 9)

An exemplary (non-limiting) nucleotide sequence that encodes human BDNF is available from public databases under GenBank Accession No. X60201.1 and is as follows (the start and stop codons are bolded and underlined):

GAATTCGGGGCTGCCGCCGCCGCGCCCGGGCGCACCCGCCCGCTCGCTGTCCCGC
GCACCCCGTAGCGCCTCGGGCTCCCGGGCCGGACAGAGGAGCCAGCCCGGTGCG
CCCCTCCACCTCCTGCTCGGGGGGCTTTAATGAGACACCCACCGCTGCTGTGGGG
CCGGCGGGGAGCAGCACCGCGACGGGGACCGGGGCTGGGCGCTGGAGCCAGAA
TCGGAACCACGATGTGACTCCGCCGCCGGGGACCCGTGAGGTTTGTGTGGACCC
CGAGTTCCACCAGGTGAGAAGAGTGATGACCATCCTTTTCCTTACTATGGTTATT
TCATACTTTGGTTGCATGAAGGCTGCCCCCATGAAAGAAGCAAACATCCGAGGA
CAAGGTGGCTTGGCCTACCCAGGTGTGCGGACCCATGGGACTCTGGAGAGCGTG
AATGGGCCCAAGGCAGGTTCAAGAGGCTTGACATCATTGGCTGACACTTTCGAA
CACGTGATAGAAGAGCTGTTGGATGAGGACCAGAAAGTTCGGCCCAATGAAGAA
AACAATAAGGACGCAGACTTGTACACGTCCAGGGTGATGCTCAGTAGTCAAGTG
CCTTTGGAGCCTCCTCTTCTCTTTCTGCTGGAGGAATACAAAAATTACCTAGATGC
TGCAAACATGTCCATGAGGGTCCGGCGCCACTCTGACCCTGCCCGCCGAGGGGA
GCTGAGCGTGTGTGACAGTATTAGTGAGTGGGTAACGGCGGCAGACAAAAAGAC
TGCAGTGGACATGTCGGGCGGGACGGTCACAGTCCTTGAAAAGGTCCCTGTATC
AAAAGGCCAACTGAAGCAATACTTCTACGAGACCAAGTGCAATCCCATGGGTTA
CACAAAAGAAGGCTGCAGGGGCATAGACAAAAGGCATTGGAACTCCCAGTGCCG
AACTACCCAGTCGTACGTGCGGGCCCTTACCATGGATAGCAAAAAGAGAATTGG
CTGGCGATTCATAAGGATAGACACTTCTTGTGTATGTACATTGACCATTAAAAGG
GGAAGATAGTGGATTTATGTTGTATAGATTAGATTATATTGAGACAAAAATTATC
TATTTGTATATATACATAACAGGGTAAATTATTCAGTTAAGAAAAAAATAATTTT
ATGAACTGCATGTATAAATGAAGTTTATACAGTACAGTGGTTCTACAATCTATTT
ATTGGACATGTCCATGACCAGAAGGGAAACAGTCATTTGCGCACAACTTAAAAA
GTCTGCATTACATTCCTTGATAATGTTGTGGTTTGTTGCCGTTGCCAAGAACTGAA

AACATAAAAAGTTAAAAAAAATAATAAATTGCATGCTGCCCGAATTC (SEQ ID NO: 10)

In some embodiments, α-MSH is administered to a subject. Alternatively or in addition, VIP is administered to a subject. Alternatively or in addition, CGRP and/or BDNF is administered to a subject. In various embodiments, another or an additional neuropeptide is administered to the subject, such as NGF, Substance P (SP), neurotrophin-3, Neurotrophin-4 (NTF-4), or neurotrophin-6. In various embodiments, an additional neuropeptide is administered to the subject, such as NGF.

An exemplary (non-limiting) amino acid sequence of human neurotrophin-3 is available in public databases, *e.g.,* under UniProt Accession No. P20783, and is as follows:

MSILFYVIFLAYLRGIQGNNMDQRSLPEDSLNSLIIKLIQADILKNKLSKQMVDVKEN
YQSTLPKAEAPREPERGGPAKSAFQPVIAMDTELLRQQRRYNSPRVLLSDSTPLEPPP
LYLMEDYVGSPVVANRTSRRKRYAEHKSHRGEYSVCDSESLWVTDKSSAIDIRGHQ
VTVLGEIKTGNSPVKQYFYETRCKEARPVKNGCRGIDDKHWNSQCKTSQTYVRALT
SENNKLVGWRWIRIDTSCVCALSRKIGRT (SEQ ID NO: 11)

An exemplary (non-limiting) nucleotide sequence that encodes human neurotrophin-3 is available from public databases under GenBank Accession No. BC107075.1 and is as follows (the start and stop codons are bolded and underlined):

CACACTCAGCTGCCAGAGCCTGCTCTTAACACCTGTGTTTCCTTTTCAGATCTTAC
AGGTGAACAAGGTG**ATG**TCCATCTTGTTTTATGTGATATTTCTCGCTTATCTCCGT
GGCATCCAAGGTAACAACATGGATCAAAGGAGTTTGCCAGAAGACTCGCTCAAT
TCCCTCATTATTAAGCTGATCCAGGCAGATATTTTGAAAAACAAGCTCTCCAAGC
AGATGGTGGACGTTAAGGAAAATTACCAGAGCACCCTGCCCAAAGCTGAGGCTC
CCCGAGAGCCGGAGCGGGGAGGGCCCGCCAAGTCAGCATTCCAGCCAGTGATTG
CAATGGACACCGAACTGCTGCGACAACAGAGACGCTACAACTCACCGCGGGTCC
TGCTGAGCGACAGCACCCCCTTGGAGCCCCCGCCCTTGTATCTCATGGAGGATTA
CGTGGGCAGCCCCGTGGTGGCGAACAGAACATCACGGCGGAAACGGTACGCGG
AGCATAAGAGTCACCGAGGGGAGTACTCGGTATGTGACAGTGAGAGTCTGTGGG
TGACCGACAAGTCATCGGCCATCGACATTCGGGGACACCAGGTCACGGTGCTGG
GGGAGATCAAAACGGGCAACTCTCCTGTCAAACAATATTTTTATGAAACGCGAT
GTAAGGAAGCCAGGCCGGTCAAAAACGGTTGCAGGGGTATTGATGATAAACACT
GGAACTCTCAGTGCAAAACATCCCAAACCTACGTCCGAGCACTGACTTCAGAGA
ACAATAAACTCGTGGGCTGGCGGTGGATACGGATAGACGTCCTGTGTGTGTG

CCTTGTCGAGAAAAATCGGAAGAACA**TGA**ATTGGCATCTCTCCCCATATATAAAT
TATTACTTTAAATTATATGATATGCATGTAGCATATAAATGTTTATATTGTTTTTA
TATATTATAAGTTGACCTTTATTTATTAAACTTCAGCAACCCTACAGTATATAAGC
TTTTTTCTCAATAAAATCAGTGTGCTTGCCTTCCCTCAGGCCTCTCCCATCT (SEQ ID NO: 12)

NTF-4 is also known as neurotrophin-5 (NTF5). An exemplary (non-limiting) amino acid sequence of human NTF-4 is available in public databases, e.g., under UniProt Accession No. P34130, and is as follows:

MLPLPSCSLPILLLFLLPSVPIESQPPPSTLPPFLAPEWDLLSPRVVLSRGAPAGPPLLF
LLEAGAFRESAGAPANRSRRGVSETAPASRRGELAVCDAVSGWVTDRRTAVDLRGR
EVEVLGEVPAAGGSPLRQYFFETRCKADNAEEGGPGAGGGGCRGVDRRHWVSECK
AKQSYVRALTADAQGRVGWRWIRIDTACVCTLLSRTGRA (SEQ ID NO: 13)

An exemplary (non-limiting) nucleotide sequence that encodes human NTF-4 is available from public databases under GenBank Accession No. BT019368.1 and is as follows (the start and stop codons are bolded and underlined):

<u>**ATG**</u>CTTCCTCTCCCCTCATGCTCCCTCCCCATCCTCCTCCTTTTCCTCCTCCCCAGT
GTGCCAATTGAGTCCCAACCCCCACCCTCAACATTGCCCCCTTTTCTGGCCCCTG
AGTGGGACCTTCTCTCCCCCCGAGTAGTCCTGTCTAGGGGTGCCCCTGCTGGGCC
CCCTCTGCTCTTCCTGCTGGAGGCTGGGGCCTTTCGGGAGTCAGCAGGTGCCCCG
GCCAACCGCAGCCGGCGTGGGGTGAGCGAAACTGCACCAGCGAGTCGTCGGGGT
GAGCTGGCTGTGTGCGATGCAGTCAGTGGCTGGGTGACAGACCGCCGGACCGCT
GTGGACTTGCGTGGGCGCGAGGTGGAGGTGTTGGGCGAGGTGCCTGCAGCTGGC
GGCAGTCCCCTCCGCCAGTACTTCTTTGAAACCCGCTGCAAGGCTGATAACGCTG
AGGAAGGTGGCCCGGGGGCAGGTGGAGGGGGCTGCCGGGGAGTGGACAGGAGG
CACTGGGTATCTGAGTGCAAGGCCAAGCAGTCCTATGTGCGGGCATTGACCGCTG
ATGCCCAGGGCCGTGTGGGCTGGCGATGGATTCGAATTGACACTGCCTGCGTCTG
CACACTCCTCAGCCGGACTGGCCGAGCC<u>**TAG**</u> (SEQ ID NO: 14)

SP is an undecapeptide (a peptide composed of a chain of 11 amino acid residues) member of the tachykinin neuropeptide family. Substance P and its closely related neurokinin A (NKA) are produced from a polyprotein precursor after differential splicing of the preprotachykinin A gene. An exemplary (non-limiting) deduced amino acid sequence of substance P is as follows:
Arg Pro Lys Pro Gln Gln Phe Phe Gly Leu Met (RPKPQQFFGLM) (SEQ ID NO: 15) with an amidation at the C-terminus. *See, e.g.,* Wong and Jeng (1994) Journal of Neuroscience Research 37 (1): 97-102.
An exemplary (non-limiting) nucleotide sequence that encodes a precursor of human substance P [*Homo sapiens* tachykinin precursor 1 (TAC1)] is available from public databases under NCBI Accession No. NM_013996.2 and is as follows (the start and stop codons are bolded and underlined):

CACGCAAGCGAAAGGAGAGGAGGCGGCTAATTAAATATTGAGCAGAAAGTCGC
GTGGGGAGAATGTCACGTGGGTCTGGAGGCTCAAGGAGGCTGGGATAAATACCG
CAAGGCACTGAGCAGGCGAAAGAGCGCGCTCGGACCTCCTTCCCGGCGGCAGCT
ACCGAGAGTGCGGAGCGACCAGCGTGCGCTCGGAGGAACCAGAGAAACTCAGC
ACCCCGCGGGACTGTCCGTCGCAAAATCCAAC**ATG**AAAATCCTCGTGGCCTTGG
CAGTCTTTTTTCTTGTCTCCACTCAGCTGTTTGCAGAAGAAATAGGAGCCAATGA
TGATCTGAATTACTGGTCCGACTGGTACGACAGCGACCAGATCAAGGAGGAACT
GCCGGAGCCCTTTGAGCATCTTCTGCAGAGAATCGCCCGGAGACCCAAGCCTCA
GCAGTTCTTTGGATTAATGGGCAAACGGGATGCTGATTCCTCAATTGAAAAACAA
GTGGCCCTGTTAAAGGCTCTTTATGGACATGGCCAGATCTCTCACAAAATGGCTT
ATGAAAGGAGTGCAATGCAGAATTATGAAAGAAGACGT**TAA**TAAACTACCTAAC
ATTATTTATTCAGCTTCATTTGTGTCAATGGGCAATGACAGGTAAATTAAGACAT
GCACTATGAGGAATAATTATTTATTTAATAACAATTGTTTGGGGTTGAAAATTCA
AAAAGTGTTTATTTTTCATATTGTGCCAATATGTATTGTAAACATGTGTTTTAATT
CCAATATGATGACTCCCTTAAAATAGAAATAAGTGGTTATTTCTCAACAAAGCAC
AGTGTTAAATGAAATTGTAAAACCTGTCAATGATACAGTCCCTAAAGAAAAAAA
ATCATTGCTTTGAAGCAGTTGTGTCAGCTACTGCGGAAAAGGAAGGAAACTCCTG
ACAGTCTTGTGCTTTTCCTATTTGTTTTCATGGTGAAAATGTACTGAGATTTTGGT
ATTACACTGTATTTGTATCTCTGAAGCATGTTTCATGTTTTGTGACTATATAGAGA
TGTTTTTAAAAGTTTCAATGTGATTCTAATGTCTTCATTTCATTGTATGATGTGTT
GTGATAGCTAACATTTTAAATAAAAGAAAAAATATCTTGAA (SEQ ID NO: 16)

Each of the amino acid and nucleotide sequences provided is exemplary and not limiting unless explicitly stated otherwise. Functional fragments, isoforms, and other variants of compounds with the exemplary amino acid sequences mentioned above are also included herein.

Pharmaceutical Formulations, Delivery to the Eye, and Cornea Storage

Dosages, formulations, dosage volumes, regimens, and compositions for use in methods for reducing or preventing CEC loss, increasing CEC proliferation, and/or increasing CEC migration can vary. Thus, minimum and maximum effective dosages vary depending on the method of administration.

In various embodiments of the invention, a composition comprising a neuropeptide (such as VIP, $\alpha$-MSH, CGRP, and/or BDNF) may be administered only once or multiple times. For example, a neuropeptide (such as VIP, $\alpha$-MSH, CGRP, and/or BDNF) may be administered using a method disclosed herein at least about once, twice, three times, four times, five times, six times, or seven times per day week, month, or year. In some embodiments, a composition comprising a neuropeptide (such as VIP, $\alpha$-MSH, CGRP, and/or BDNF) is administered once per month. In certain embodiments, the composition is administered once per month via intravitreal or subconjunctival injection. In certain embodiments, the composition is administered via intravitreal injection. In certain embodiments, the composition is administered via subconjunctival injection. In various embodiments, such as embodiments involving eye drops, a composition is self-administered.

Preferred formulations are in the form of a solid, a paste, an ointment, a gel, a liquid, an aerosol, a mist, a polymer, a contact lens, a film, a solution, an emulsion, or a suspension. In some embodiments, the formulations are administered topically, *e.g.,* the composition is delivered to and directly contacts the eye. In certain embodiments, a neuropeptide (such as VIP, $\alpha$-MSH, CGRP, and/or BDNF) can be administered at any dose, and the dose thereof and/or frequency of administration may be adjusted (*e.g.,* increased or decreased) to arrive at an effective dose. In various embodiments, a neuropeptide (such as VIP, $\alpha$-MSH, CGRP, and/or BDNF) is present at a concentration of about, at least about, or less than about 0.000001 $\mu$M, 0.00001 $\mu$M, 0.0001 $\mu$M, 0.001 $\mu$M, 0.01 $\mu$M, 0.1 $\mu$M, 1 $\mu$M, 2 $\mu$M, 3 $\mu$M, 4 $\mu$M, 5 $\mu$M, 6 $\mu$M, 7 $\mu$M, 8 $\mu$M, 9 $\mu$M, 10 $\mu$M, 11 $\mu$M, 12 $\mu$M, 13 $\mu$M, 14 $\mu$M, 15 $\mu$M, 16 $\mu$M, 17 $\mu$M, 18 $\mu$M, 19 $\mu$M, 20 $\mu$M, 21 $\mu$M, 22 $\mu$M, 23 $\mu$M, 24 $\mu$M, 25 $\mu$M, 30 $\mu$M, 35 $\mu$M, 40 $\mu$M, 45 $\mu$M, 50 $\mu$M, 55 $\mu$M, 60 $\mu$M, 65 $\mu$M, 70 $\mu$M, 75 $\mu$M, 80 $\mu$M, 85 $\mu$M, 90 $\mu$M, 95 $\mu$M, 100 $\mu$M, 150 $\mu$M, 200 $\mu$M, 250 $\mu$M, 300 $\mu$M, 350 $\mu$M, 400 $\mu$M, 500 $\mu$M, 600 $\mu$M, 700 $\mu$M, 800 $\mu$M, 900 $\mu$M, or 1000 $\mu$M or 0.0000001-100 $\mu$M, 0.000001-100 $\mu$M, 0.0000001-10 $\mu$M, 0.000001-10 $\mu$M, 0.00001-0.001 $\mu$M, 0.0001-0.01 $\mu$M, 0.001-0.01 $\mu$M, 0.001-0.1 $\mu$M, 0.001-1 $\mu$M, 1-10 $\mu$M, 1-50 $\mu$M, 1-100 $\mu$M, 10-25 $\mu$M, 10-50 $\mu$M, 10-100 $\mu$M, 25-50 $\mu$M, 25-100 $\mu$M, 25-500 $\mu$M, 50-100 $\mu$M, 50-250 $\mu$M, 50-500 $\mu$M, 100-250 $\mu$M, 100-500 $\mu$M, 250-500 $\mu$M, 250-750 $\mu$M, or 500-1000 $\mu$M. In some embodiments, a neuropeptide (such as VIP, $\alpha$-MSH, CGRP, and/or BDNF) is present at a concentration of at least about 0.0000001 $\mu$M, 0.000001 $\mu$M, 0.00001 $\mu$M, 0.0001 $\mu$M, 0.001 $\mu$M, 0.01 $\mu$M, 0.1 $\mu$M, or 1 $\mu$M and less than about 10 $\mu$M, 15 $\mu$M, 20 $\mu$M, 25 $\mu$M, 30 $\mu$M, 35 $\mu$M, 40 $\mu$M, 45 $\mu$M, 50 $\mu$M, 55 $\mu$M, 60 $\mu$M, 65 $\mu$M, 70 $\mu$M, 75 $\mu$M, 80 $\mu$M, 85 $\mu$M, 90 $\mu$M, 95 $\mu$M, 100 $\mu$M, 150 $\mu$M, 200 $\mu$M, 250 $\mu$M, 300 $\mu$M, 350 $\mu$M, 400 $\mu$M, 500 $\mu$M, 600 $\mu$M, 700 $\mu$M, 800 $\mu$M, 900 $\mu$M, or 1000 $\mu$M. In certain embodiments, a neuropeptide (such as VIP, $\alpha$-MSH, CGRP, and/or BDNF) is present at a concentration of at least about 0.000000001%, 0.00000001%, 0.0000001%, 0.000001%, 0.00001%, 0.0001%, 0.001%, 0.01%, 0.1%, 1%, 5%, 10%, 20%, 30%, 40%, 50% or about 0.000000001-0.000001%, 0.000000001-0.0001%, 0.00000001-0.001%, 0.00000001-0.01%, 0.00000001-0.1%, 0.00000001-1%, 0.000001-0.00001%, 0.000001-0.0001%, 0.000001-0.001%, 0.000001-0.01%, 0.000001-0.1%, 0.000001-1%, 1-5%, 1-50%, 5-10%, 5-10%, 10-25%, 10-50%, 25-50%, or 0.000000001 - 50% (weight/volume). In certain embodiments, a neuropeptide (such as VIP, $\alpha$-MSH, CGRP, and/or BDNF) is present at concentrations of 0.000000001% (weight/volume), 0.0000001% (weight/volume), 0.00001% (weight/volume), 0.01% (weight/volume), 0.1% (weight/volume), 1% (weight/volume), 10% (weight/volume), 20% (weight/volume), 25% (weight/volume), 30% (weight/volume), 40% (weight/volume), 50% (weight/volume), or any percentage point in between. In various embodiments, the method does not involve systemic administration or planned substantial dissemination of the composition to non-ocular tissue.

In some embodiments, a neuropeptide (such as VIP, $\alpha$-MSH, CGRP, and/or BDNF) is present in a composition or administered at a dose of about, at least about, or less than about 0.5 microgram ($\mu$g), 1 $\mu$g, 2 $\mu$g, 3 $\mu$g, 4 $\mu$g, 5 $\mu$g, 6 $\mu$g, 7 $\mu$g, 8 $\mu$g, 9 $\mu$g, 10 $\mu$g, 11 $\mu$g, 12 $\mu$g, 13 $\mu$g, 14 $\mu$g, 15 $\mu$g, 16 $\mu$g, 17 $\mu$g, 18 $\mu$g, 19 $\mu$g, 20 $\mu$g, 21 $\mu$g, 22 $\mu$g, 23 $\mu$g, 24 $\mu$g, 25 $\mu$g, 30 $\mu$g, 35 $\mu$g, 40 $\mu$g, 45 $\mu$g, 50 $\mu$g, 55 $\mu$g, 60 $\mu$g, 65 $\mu$g, 70 $\mu$g, 75 $\mu$g, 80 $\mu$g, 85 $\mu$g, 90 $\mu$g, 95 $\mu$g, 100 $\mu$g, 150 $\mu$g, 200 $\mu$g, 250 $\mu$g, 300 $\mu$g, 350 $\mu$g, 400 $\mu$g, 500 $\mu$g, 600 $\mu$g, 700 $\mu$g, 800 $\mu$g, 900 $\mu$g, 1000 $\mu$g or 0.5-100 $\mu$g, 1-10 $\mu$g, 100-1000 $\mu$g, 1-50 $\mu$g, 1-100 $\mu$g, 10-25 $\mu$g, 10-50 $\mu$g, 10-100 $\mu$g, 25-50 $\mu$g, 25-100 $\mu$g, 25-500 $\mu$g, 50-100 $\mu$g, 50-250 $\mu$g, 50-500 $\mu$g, 100-250 $\mu$g, 100-500 $\mu$g, 250-500 $\mu$g, 250-750 $\mu$g, or 500-1000 $\mu$g. In some embodiments, a neuropeptide (such as VIP, $\alpha$-MSH, CGRP, and/or BDNF) is present at a concentration of at least about 0.5 $\mu$g, 1 $\mu$g, 2 $\mu$g, 3 $\mu$g, 4 $\mu$g, 5 $\mu$g, 6 $\mu$g, 7 $\mu$g, 8 $\mu$g, 9 $\mu$g, 10 $\mu$g and less than about 25 $\mu$g, 30 $\mu$g, 35 $\mu$g, 40 $\mu$g, 45 $\mu$g, 50 $\mu$g, 55 $\mu$g, 60 $\mu$g, 65 $\mu$g, 70 $\mu$g, 75 $\mu$g, 80 $\mu$g, 85 $\mu$g, 90 $\mu$g, 95 $\mu$g, 100 $\mu$g, 150 $\mu$g, 200 $\mu$g, 250 $\mu$g, 300 $\mu$g, 350 $\mu$g, 400 $\mu$g, 500 $\mu$g, 600 $\mu$g, 700 $\mu$g, 800 $\mu$g, 900 $\mu$g, or 1000 $\mu$g.

In various embodiments of the invention, a composition comprising a melanocortin receptor agonist such as $\alpha$-MSH may be administered only once or multiple times. For example, a melanocortin receptor agonist such as $\alpha$-MSH may be administered using a method disclosed herein at least about once, twice, three times, four times, five times, six times, or seven times per day week, month, or year. In some embodiments, a composition comprising a melanocortin receptor agonist such as $\alpha$-MSH is administered once per month. In certain embodiments, the composition is administered once per month via intravitreal or subconjunctival injection. In certain embodiments, the composition is administered via intravitreal injection. In certain embodiments, the composition is administered via subconjunctival injection. In various embodiments, such as embodiments involving eye drops, a composition is self-administered.

Preferred formulations are in the form of a solid, a paste, an ointment, a gel, a liquid, an aerosol, a mist, a polymer, a contact lens, a film, a solution, an emulsion, or a suspension. In some embodiments, the formulations are administered topically, *e.g.,* the composition is delivered to and directly contacts the eye. In certain embodiments, a melanocortin receptor agonist such as $\alpha$-MSH can be administered at any dose, and the dose thereof and/or frequency of administration may be adjusted (*e.g.,* increased or decreased) to arrive at an effective dose. In various embodiments, a melanocortin receptor agonist such as $\alpha$-MSH is present at a concentration of about, at least about, or less than about 0.0000001 $\mu$M, 0.000001 $\mu$M, 0.00001 $\mu$M, 0.0001 $\mu$M, 0.001 $\mu$M, 0.01 $\mu$M, 0.1 $\mu$M, 1 $\mu$M, 2 $\mu$M, 3 $\mu$M, 4 $\mu$M, 5 $\mu$M, 6 $\mu$M, 7 $\mu$M, 8 $\mu$M, 9 $\mu$M, 10 $\mu$M, 11 $\mu$M, 12 $\mu$M, 13 $\mu$M, 14 $\mu$M, 15 $\mu$M, 16 $\mu$M, 17 $\mu$M, 18 $\mu$M, 19 $\mu$M, 20 $\mu$M, 21 $\mu$M, 22 $\mu$M, 23 $\mu$M, 24 $\mu$M, 25 $\mu$M, 30 $\mu$M, 35 $\mu$M, 40 $\mu$M, 45 $\mu$M, 50 $\mu$M, 55 $\mu$M, 60 $\mu$M, 65 $\mu$M, 70 $\mu$M, 75 $\mu$M, 80 $\mu$M, 85 $\mu$M, 90 $\mu$M, 95 $\mu$M, 100 $\mu$M, 150 $\mu$M, 200 $\mu$M, 250 $\mu$M, 300 $\mu$M, 350 $\mu$M, 400 $\mu$M, 500 $\mu$M, 600 $\mu$M, 700 $\mu$M, 800 $\mu$M, 900 $\mu$M, 1000 $\mu$M, or 0.0000001-100 $\mu$M, 0.000001-100 $\mu$M, 0.0000001-10 $\mu$M, 0.000001-10 $\mu$M, 0.00001-0.001 $\mu$M, 0.0001-0.01 $\mu$M, 0.001-0.01 $\mu$M, 0.001-0.1 $\mu$M, 0.001-1 $\mu$M, 1-10 $\mu$M, 1-50 $\mu$M, 1-100 $\mu$M, 10-25 $\mu$M, 10-50 $\mu$M, 10-100 $\mu$M, 25-50 $\mu$M, 25-100 $\mu$M, 25-500 $\mu$M, 50-100 $\mu$M, 50-250 $\mu$M, 50-500 $\mu$M, 100-250 $\mu$M, 100-500 $\mu$M, 250-500 $\mu$M, 250-750 $\mu$M, or 500-1000 $\mu$M. In some embodiments, a melanocortin receptor agonist such as $\alpha$-MSH is present at a concentration of at least about 0.0000001 $\mu$M, 0.000001 $\mu$M, 0.00001 $\mu$M, 0.0001 $\mu$M, 0.001 $\mu$M, 0.01 $\mu$M, 0.1 $\mu$M, or 1 $\mu$M and less than about 10 $\mu$M, 15 $\mu$M, 20 $\mu$M, 25 $\mu$M, 30 $\mu$M, 35 $\mu$M, 40 $\mu$M, 45 $\mu$M, 50 $\mu$M, 55 $\mu$M, 60 $\mu$M, 65 $\mu$M, 70 $\mu$M, 75 $\mu$M, 80 $\mu$M, 85 $\mu$M, 90 $\mu$M, 95 $\mu$M, 100

μM, 150 μM, 200 μM, 250 μM, 300 μM, 350 μM, 400 μM, 500 μM, 600 μM, 700 μM, 800 μM, 900 μM, 1000 μM. In certain embodiments, a melanocortin receptor agonist such as α-MSH is present at a concentration of at least about 0.000000001%, 0.00000001%, 0.0000001%, 0.000001%, 0.00001%, 0.0001%, 0.001%, 0.01%, 0.1%, 1%, 5%, 10%, 20%, 30%, 40%, 50% or about 0.000000001-0.000001%, 0.000000001-0.0001%, 0.00000001-0.001%, 0.00000001-0.01%, 0.00000001-0.1%, 0.00000001-1%, 0.000001-0.00001%, 0.000001-0.0001%, 0.000001-0.001%, 0.000001-0.01%, 0.000001-0.1%, 0.000001-1%, 1-5%, 1-50%, 5-10%, 5-10%, 10-25%, 10-50%, 25-50%, or 0.000000001 - 50% (weight/volume). In certain embodiments, a melanocortin receptor agonist such as α-MSH is present at concentrations of 0.000000001% (weight/volume), 0.0000001% (weight/volume), 0.00001% (weight/volume), 0.01% (weight/volume), 0.1% (weight/volume), 1% (weight/volume), 10% (weight/volume), 20% (weight/volume), 25% (weight/-volume), 30% (weight/volume), 40% (weight/volume), 50% (weight/volume), or any percentage point in between. In various embodiments, the method does not involve systemic administration or planned substantial dissemination of the composition to non-ocular tissue.

In some embodiments, a melanocortin receptor agonist such as α-MSH is present in a composition or administered at a dose of about, at least about, or less than about 0.5 microgram (μg), 1 μg, 2 μg, 3 μg, 4 μg, 5 μg, 6 μg, 7 μg, 8 μg, 9 μg, 10 μg, 11 μg, 12 μg, 13 μg, 14 μg, 15 μg, 16 μg, 17 μg, 18 μg, 19 μg, 20 μg, 21 μg, 22 μg, 23 μg, 24 μg, 25 μg, 30 μg, 35 μg, 40 μg, 45 μg, 50 μg, 55 μg, 60 μg, 65 μg, 70 μg, 75 μg, 80 μg, 85 μg, 90 μg, 95 μg, 100 μg, 150 μg, 200 μg, 250 μg, 300 μg, 350 μg, 400 μg, 500 μg, 600 μg, 700 μg, 800 μg, 900 μg, 1000 μg or 0.5-100 μg, 1-10 μg, 100-1000 μg, 1-50 μg, 1-100 μg, 10-25 μg, 10-50 μg, 10-100 μg, 25-50 μg, 25-100 μg, 25-500 μg, 50-100 μg, 50-250 μg, 50-500 μg, 100-250 μg, 100-500 μg, 250-500 μg, 250-750 μg, or 500-1000 μg. In some embodiments, a melanocortin receptor agonist is present at a concentration of at least about 0.5 μg, 1 μg, 2 μg, 3 μg, 4 μg, 5 μg, 6 μg, 7 μg, 8 μg, 9 μg, 10 μg and less than about 25 μg, 30 μg, 35 μg, 40 μg, 45 μg, 50 μg, 55 μg, 60 μg, 65 μg, 70 μg, 75 μg, 80 μg, 85 μg, 90 μg, 95 μg, 100 μg, 150 μg, 200 μg, 250 μg, 300 μg, 350 μg, 400 μg, 500 μg, 600 μg, 700 μg, 800 μg, 900 μg, or 1000 μg.

In various embodiments, a volume of about, at least about, or less than about 1 μl, 10 μl, 50 μl, 100 μl, 500 μl, 1000 μl, 2500 μl, or 5000 μl of a composition comprising a melanocortin receptor agonist is administered to a subject. In some embodiments, the volume is about 1-10 μl, 10-50 μl, 10-100 μl, 50-100 μl, 50-500 μl, 100-500 μl, 1-5000 μl, 100-5000 μl, or 500-5000 μl. Optionally, the composition further contains a pharmaceutically-acceptable carrier. Exemplary pharmaceutical carriers include, but are not limited to, compounds selected from the group consisting of a physiological acceptable salt, poloxamer analogs with carbopol, carbopol/hydroxypropyl methyl cellulose (HPMC), carbopol-methyl cellulose, a mucolytic agent, carboxymethylcellulose (CMC), hyaluronic acid, cyclodextrin, and petroleum. In certain embodiments, the mucolytic agent is N-acetyl cysteine.

In some embodiments relating to the treatment or prevention of CEC loss, a neuropeptide (such as VIP, α-MSH, CGRP, and/or BDNF) (*e.g.,* a pharmaceutical composition comprising a neuropeptide) may be administered locally, *e.g.,* as a topical eye drop or by injection, such as intracameral injection (into the anterior chamber), by intravitreal injection, by subconjunctival injection, by an intraocular injection, by an intraocular implant, by subtenon injection, by retrobulbar injection, with a peri-ocular device (*e.g.,* which can actively or passively deliver drug), by iontophoresis, by intracorneal injection, or by intraretinal injection.

In some embodiments relating to the treatment or prevention of CEC loss, a melanocortin receptor agonist such as α-MSH (*e.g.*, a pharmaceutical composition comprising α-MSH) may be administered locally, e.g., as a topical eye drop or by injection, such as intracameral injection (into the anterior chamber), by intravitreal injection, by subconjunctival injection, by an intraocular injection, by an intraocular implant, by subtenon injection, by retrobulbar injection, with a peri-ocular device (*e.g.,* which can actively or passively deliver drug), by iontophoresis, by intracorneal injection, or by intraretinal injection.

Pharmaceutical formulations adapted for topical administration may be formulated as, *e.g.,* aqueous solutions, ointments, creams, suspensions, lotions, powders, solutions, pastes, gels, sprays, aerosols, liposomes, microcapsules, microspheres, or oils.

In various embodiments, pharmaceutical formulations adapted for topical administrations to the eye include eye drops wherein a neuropeptide (such as VIP, α-MSH, CGRP, and/or BDNF) is dissolved or suspended in a suitable carrier, especially an aqueous solvent.

In certain embodiments, pharmaceutical formulations adapted for topical administrations to the eye include eye drops wherein a melanocortin receptor agonist such as α-MSH is dissolved or suspended in a suitable carrier, especially an aqueous solvent.

Preferably, formulations to be administered to the eye will have ophthalmically compatible pH and osmolality. The term "ophthalmically acceptable vehicle" means a pharmaceutical composition having physical properties (*e.g.,* pH and/or osmolality) that are physiologically compatible with ophthalmic tissues.

In some embodiments, an ophthalmic composition of the present invention is formulated as sterile aqueous solutions having an osmolality of from about 200 to about 400 milliosmoles/kilogram water ("mOsm/kg") and a physiologically compatible pH. The osmolality of the solutions may be adjusted by means of conventional agents, such as inorganic salts (*e.g.,* NaCl), organic salts (*e.g.,* sodium citrate), polyhydric alcohols (*e.g.,* propylene glycol or sorbitol) or combinations thereof.

In various embodiments, the ophthalmic formulations of the present invention may be in the form of liquid, solid or semisolid dosage form. In certain embodiments, the ophthalmic formulations of the present invention may comprise, depending on the final dosage form, suitable ophthalmically acceptable excipients. In some embodiments, the ophthalmic formulations are formulated to maintain a physiologically tolerable pH range. In certain embodiments, the pH range of the ophthalmic formulation is in the range of from about 5 to about 9. In some embodiments, pH range of the ophthalmic formulation is in the range of from about 6 to about 8, or is about 6.5, about 7, or about 7.5.

In some embodiments, the composition is in the form of an aqueous solution, such as one that can be presented in the form of eye drops. By means of an exemplary suitable dispenser, a desired dosage of the active agent can be metered by administration of a known number of drops into the eye, such as by one, two, three, four, or five drops.

In certain embodiments, one or more ophthalmically acceptable pH adjusting agents and/or buffering agents can be included in a composition provided herein, including acids such as acetic, boric, citric, lactic, phosphoric, and hydrochloric acids; bases such as sodium hydroxide, sodium phosphate, sodium borate, sodium citrate, sodium acetate, and sodium lactate; and buffers such as citrate/dextrose, sodium bicarbonate, and ammonium chloride. Such acids, bases, and buffers can be included in an amount required to maintain pH of the composition in an ophthalmically acceptable range. Optionally, one or more ophthalmically acceptable salts can be included in the composition in an amount sufficient to bring osmolality of the composition into an ophthalmically acceptable range. Such salts include those having sodium, potassium, or ammonium cations and chloride, citrate, ascorbate, borate, phosphate, bicarbonate, sulfate, thiosulfate, or bisulfite anions.

Pharmaceutical compositions for ocular delivery also include *in situ* gellable aqueous composition. Such a composition comprises a gelling agent in a concentration effective to promote gelling upon contact with the eye or with lacrimal fluid. Suitable gelling agents include but are not limited to thermosetting polymers. The term *"in situ* gellable" as used herein includes not only liquids of low viscosity that form gels upon contact with the eye or with lacrimal fluid, but also includes more viscous liquids such as semi-fluid and thixotropic gels that exhibit substantially increased viscosity or gel stiffness upon administration to the eye. See, for example, Ludwig, Adv. Drug Deliv. Rev. 3; 57:1595-639 (2005).

Also included herein are compositions comprising a neuropeptide (such as VIP, $\alpha$-MSH, CGRP, and/or BDNF) for the storage of a cornea, a CEC, or a membrane or sheet of cells comprising CECs (such as a Descemet membrane). In some embodiments, the composition comprises a corneal storage medium, corneal tissue preservation solution, or CEC preservation solution. For example, the composition may be corneal storage medium or solution that comprises a neuropeptide (such as VIP, $\alpha$-MSH, CGRP, and/or BDNF). In certain embodiments, the storage medium is a culture medium that comprises a neuropeptide (such as VIP, $\alpha$-MSH, CGRP, and/or BDNF). In various embodiments, a neuropeptide (such as VIP, $\alpha$-MSH, CGRP, and/or BDNF) is present at a concentration of about, at least about, or less than about 0.0000001 $\mu$M, 0.000001 $\mu$M, 0.00001 $\mu$M, 0.0001 $\mu$M, 0.001 $\mu$M, 0.01 $\mu$M, 0.1 $\mu$M, 1 $\mu$M, 2 $\mu$M, 3 $\mu$M, 4 $\mu$M, 5 $\mu$M, 6 $\mu$M, 7 $\mu$M, 8 $\mu$M, 9 $\mu$M, 10 $\mu$M, 11 $\mu$M, 12 $\mu$M, 13 $\mu$M, 14 $\mu$M, 15 $\mu$M, 16 $\mu$M, 17 $\mu$M, 18 $\mu$M, 19 $\mu$M, 20 $\mu$M, 21 $\mu$M, 22 $\mu$M, 23 $\mu$M, 24 $\mu$M, 25 $\mu$M, 30 $\mu$M, 35 $\mu$M, 40 $\mu$M, 45 $\mu$M, 50 $\mu$M, 55 $\mu$M, 60 $\mu$M, 65 $\mu$M, 70 $\mu$M, 75 $\mu$M, 80 $\mu$M, 85 $\mu$M, 90 $\mu$M, 95 $\mu$M, 100 $\mu$M, 150 $\mu$M, 200 $\mu$M, 250 $\mu$M, 300 $\mu$M, 350 $\mu$M, 400 $\mu$M, 500 $\mu$M, 600 $\mu$M, 700 $\mu$M, 800 $\mu$M, 900 $\mu$M, 1000 $\mu$M, or 0.0000001-100 $\mu$M, 0.000001-100 $\mu$M, 0.0000001-10 $\mu$M, 0.000001-10 $\mu$M, 0.00001-0.001 $\mu$M, 0.0001-0.01 $\mu$M, 0.001-0.01 $\mu$M, 0.001-0.1 $\mu$M, 0.001-1 $\mu$M, 1-10 $\mu$M, 1-50 $\mu$M, 1-100 $\mu$M, 10-25 $\mu$M, 10-50 $\mu$M, 10-100 $\mu$M, 25-50 $\mu$M, 25-100 $\mu$M, 25-500 $\mu$M, 50-100 $\mu$M, 50-250 $\mu$M, 50-500 $\mu$M, 100-250 $\mu$M, 100-500 $\mu$M, 250-500 $\mu$M, 250-750 $\mu$M, 500-1000 $\mu$M. In certain embodiments, a neuropeptide (such as VIP, $\alpha$-MSH, CGRP, and/or BDNF) is present at a concentration of at least about 0.0000001 $\mu$M, 0.000001 $\mu$M, 0.00001 $\mu$M, 0.0001 $\mu$M, 0.001 $\mu$M, 0.01 $\mu$M, 0.1 $\mu$M, or 1 $\mu$M and less than about 10 $\mu$M, 15 $\mu$M, 20 $\mu$M, 25 $\mu$M, 30 $\mu$M, 35 $\mu$M, 40 $\mu$M, 45 $\mu$M, 50 $\mu$M, 55 $\mu$M, 60 $\mu$M, 65 $\mu$M, 70 $\mu$M, 75 $\mu$M, 80 $\mu$M, 85 $\mu$M, 90 $\mu$M, 95 $\mu$M, 100 $\mu$M, 150 $\mu$M, 200 $\mu$M, 250 $\mu$M, 300 $\mu$M, 350 $\mu$M, 400 $\mu$M, 500 $\mu$M, 600 $\mu$M, 700 $\mu$M, 800 $\mu$M, 900 $\mu$M, or 1000 $\mu$M.

Also provided herein are compositions comprising a melanocortin receptor agonist such as $\alpha$-MSH for the storage of a cornea, a CEC, or a membrane or sheet of cells comprising CECs (such as a Descemet membrane). In some embodiments, the composition comprises a corneal storage medium, corneal tissue preservation solution, or CEC preservation solution. For example, the composition may be corneal storage medium or solution that comprises a melanocortin receptor agonist such as $\alpha$-MSH. In certain embodiments, the storage medium is a culture medium that comprises a melanocortin receptor agonist such as $\alpha$-MSH. In various embodiments, a melanocortin receptor agonist such as $\alpha$-MSH is present at a concentration of about, at least about, or less than about 0.0000001 $\mu$M, 0.000001 $\mu$M, 0.00001 $\mu$M, 0.0001 $\mu$M, 0.001 $\mu$M, 0.01 $\mu$M, 0.1 $\mu$M, 1 $\mu$M, 2 $\mu$M, 3 $\mu$M, 4 $\mu$M, 5 $\mu$M, 6 $\mu$M, 7 $\mu$M, 8 $\mu$M, 9 $\mu$M, 10 $\mu$M, 11 $\mu$M, 12 $\mu$M, 13 $\mu$M, 14 $\mu$M, 15 $\mu$M, 16 $\mu$M, 17 $\mu$M, 18 $\mu$M, 19 $\mu$M, 20 $\mu$M, 21 $\mu$M, 22 $\mu$M, 23 $\mu$M, 24 $\mu$M, 25 $\mu$M, 30 $\mu$M, 35 $\mu$M, 40 $\mu$M, 45 $\mu$M, 50 $\mu$M, 55 $\mu$M, 60 $\mu$M, 65 $\mu$M, 70 $\mu$M, 75 $\mu$M, 80 $\mu$M, 85 $\mu$M, 90 $\mu$M, 95 $\mu$M, 100 $\mu$M, 150 $\mu$M, 200 $\mu$M, 250 $\mu$M, 300 $\mu$M, 350 $\mu$M, 400 $\mu$M, 500 $\mu$M, 600 $\mu$M, 700 $\mu$M, 800 $\mu$M, 900 $\mu$M, 1000 $\mu$M, or 0.0000001-100 $\mu$M, 0.000001-100 $\mu$M, 0.0000001-10 $\mu$M, 0.000001-10 $\mu$M, 0.00001-0.001 $\mu$M, 0.0001-0.01 $\mu$M, 0.001-0.01 $\mu$M, 0.001-0.1 $\mu$M, 0.001-1 $\mu$M, 1-10 $\mu$M, 1-50 $\mu$M, 1-100 $\mu$M, 10-25 $\mu$M, 10-50 $\mu$M, 10-100 $\mu$M, 25-50 $\mu$M, 25-100 $\mu$M, 25-500 $\mu$M, 50-100 $\mu$M, 50-250 $\mu$M, 50-500 $\mu$M, 100-250 $\mu$M, 100-500 $\mu$M, 250-500 $\mu$M, 250-750 $\mu$M, 500-1000 $\mu$M. In certain

embodiments, a melanocortin receptor agonist such as $\alpha$-MSH is present at a concentration of at least about 0.0000001 $\mu$M, 0.000001 $\mu$M, 0.00001 $\mu$M, 0.0001 $\mu$M, 0.001 $\mu$M, 0.01 $\mu$M, 0.1 $\mu$M, or 1 $\mu$M and less than about 10 $\mu$M, 15 $\mu$M, 20 $\mu$M, 25 $\mu$M, 30 $\mu$M, 35 $\mu$M, 40 $\mu$M, 45 $\mu$M, 50 $\mu$M, 55 $\mu$M, 60 $\mu$M, 65 $\mu$M, 70 $\mu$M, 75 $\mu$M, 80 $\mu$M, 85 $\mu$M, 90 $\mu$M, 95 $\mu$M, 100 $\mu$M, 150 $\mu$M, 200 $\mu$M, 250 $\mu$M, 300 $\mu$M, 350 $\mu$M, 400 $\mu$M, 500 $\mu$M, 600 $\mu$M, 700 $\mu$M, 800 $\mu$M, 900 $\mu$M, or 1000 $\mu$M.

In some embodiments, the composition comprises chondroitin sulphate. Non-limiting examples of corneal storage media include Optisol GS (also referred to herein as Optisol) and Dexsol. These media differ mainly in the concentration of chondroitin sulphate, which is 2.5% in Optisol and 1.35% in Dexsol, and the addition of multiple components (vitamins, hydroxyproline, and ATP precursors) to the Optisol solution. Non-limiting features of exemplary compositions that are useful for storing cornea tissue are described in Greenbaum (2004) Optisol vs Dexsol as storage media for preservation of human corneal epithelium. Eye 18, 519-524; Lindstrom et al. (1992) Optisol corneal storage medium. Am J Ophthalmol 114(3):345-56; Armitage (2011) Preservation of Human Cornea, Transfus Med Hemother 38(2): 143-147. Constituents of Optisol and Dexsol are listed in Table 1 below.

Table 1. Constituents of Two Examples of Corneal Storage Media

| Constituent | Optisol | Dexsol |
|---|---|---|
| Base Medium | Hybrid of TC-199 and MEM | MEM |
| Chondroitin sulphate, (%) | 2.5 | 1.35 |
| Dextran, (%) | 1 | 1 |
| HEPES buffer | Yes | Yes |
| Gentamycin sulphate | Yes | Yes |
| Streptomycin | Yes | No |
| Nonessential amino acids (mmol/l) | 0.1 | 0.1 |
| Sodium pyruvate (mmol/l) | 1 | 1 |
| Additional antioxidants | Yes | Yes |
| Sodium bicarbonate | Yes | Yes |
| MEM=minimum essential medium; TC-199=tissue culture medium 199; HEPES=N-2-hydroxyethylpiperazine-N'-2-ethane sulfonic acid. | | |

In certain embodiments, a cornea, cornea tissue (e.g., a portion of a cornea), a CEC, or a membrane or sheet of cells comprising CECs (such as a Descemet membrane to which CECs are attached) remains suitable for transplantation into a subject when stored in a composition comprising a neuropeptide (such as VIP, $\alpha$-MSH, CGRP, and/or BDNF) (e.g., at a temperature of about 4°C) for at least about 1, 2, 3, 4, 5, 6, 12, 24, 48, 72, or 120 hours longer than is typical for such a cornea, cornea tissue, CEC, or membrane or sheet when stored in a corresponding composition without the neuropeptide. In some embodiments, a cornea or cornea tissue may be stored in a composition provided herein for at least about 7, 8, 9, 10, 11, 12, 13, 14, 15, 16, 17, 18, 19, 20, or 21 days. In certain embodiments, a cornea or cornea tissue may be stored for at least about 10-15, 10-20, 15-20, or more days. In various embodiments, at least about 70%, 75%, 80%, 85%, 90%, 95%, 96%, 97%, 98%, 99%, or more of the CECs in a cornea, tissue, membrane, or sheet of cells comprising CECs remains viable when stored in a composition comprising a neuropeptide (such as VIP, $\alpha$-MSH, CGRP, and/or BDNF) for at least about 7, 8, 9, 10, 11, 12, 13, 14, 15, 16, 17, 18, 19, 20, or 21 days. The present subject matter also includes corneas, cornea tissue, CECs, and membranes or sheets of cells comprising CECs that have been stored (e.g., immersed) in a composition comprising a neuropeptide (such as VIP, $\alpha$-MSH, CGRP, and/or BDNF) for at least about 7, 8, 9, 10, 11, 12, 13, 14, 15, 16, 17, 18, 19, 20, or 21 days.

In various embodiments, a cornea, cornea tissue (e.g., a portion of a cornea), a CEC, or a membrane or sheet of cells comprising CECs (such as a Descemet membrane to which CECs are attached) remains suitable for transplantation into a subject when stored in a composition comprising a melanocortin receptor agonist such as $\alpha$-MSH (e.g., at a temperature of about 4°C) for at least about 1, 2, 3, 4, 5, 6, 12, 24, 48, 72, or 120 hours longer than is typical for such a cornea, cornea tissue, CEC, or membrane or sheet when stored in a corresponding composition without the a melanocortin receptor agonist. In some embodiments, a cornea or cornea tissue may be stored in a composition provided herein for at least about 7, 8, 9, 10, 11, 12, 13, 14, 15, 16, 17, 18, 19, 20, or 21 days. In certain embodiments, a cornea or cornea tissue may be stored for at least about 10-15, 10-20, 15-20, or more days. In various embodiments, at least about 70%, 75%, 80%, 85%, 90%, 95%, 96%, 97%, 98%, 99%, or more of the CECs in a cornea, tissue, membrane, or sheet of cells comprising CECs remains viable when stored in a composition comprising a melanocortin receptor agonist such as

$\alpha$-MSH for at least about 7, 8, 9, 10, 11, 12, 13, 14, 15, 16, 17, 18, 19, 20, or 21 days. The present subject matter also includes corneas, cornea tissue, CECs, and membranes or sheets of cells comprising CECs that have been stored (e.g., immersed) in a composition comprising a melanocortin receptor agonist such as $\alpha$-MSH for at least about 7, 8, 9, 10, 11, 12, 13, 14, 15, 16, 17, 18, 19, 20, or 21 days.

Drug Delivery by Contact Lens

Included herein is a contact lens and a composition that inhibits CEC loss. For example, the composition may be incorporated into or coated onto the lens. In various embodiments, the composition is chemically bound or physically entrapped by the contact lens polymer. In some embodiments, a color additive is chemically bound or physically entrapped by the polymer composition that is released at the same rate as the therapeutic drug composition, such that changes in the intensity of the color additive indicate changes in the amount or dose of therapeutic drug composition remaining bound or entrapped within the polymer. In certain embodiments, an ultraviolet (UV) absorber is chemically bound or physically entrapped within the contact lens polymer. In various embodiments, the contact lens is either hydrophobic or hydrophilic.

Exemplary materials used to fabricate a hydrophobic lens with means to deliver the compositions of the invention include, but are not limited to, amefocon A, amsilfocon A, aquilafocon A, arfocon A, cabufocon A, cabufocon B, carbosilfocon A, crilfocon A, crilfocon B, dimefocon A, enflufocon A, enflofocon B, erifocon A, flurofocon A, flusilfocon A, flusilfocon B, flusilfocon C, flusilfocon D, flusilfocon E, hexafocon A, hofocon A, hybufocon A, itabisfluorofocon A, itafluorofocon A, itafocon A, itafocon B, kolfocon A, kolfocon B, kolfocon C, kolfocon D, lotifocon A, lotifocon B, lotifocon C, melafocon A, migafocon A, nefocon A, nefocon B, nefocon C, onsifocon A, oprifocon A, oxyfluflocon A, paflufocon B, paflufocon C, paflufocon D, paflufocon E, paflufocon F, pasifocon A, pasifocon B, pasifocon C, pasifocon D, pasifocon E, pemufocon A, porofocon A, porofocon B, roflufocon A, roflufocon B, roflufocon C, roflufocon D, roflufocon E, rosilfocon A, satafocon A, siflufocon A, silafocon A, sterafocon A, sulfocon A, sulfocon B, telafocon A, tisilfocon A, tolofocon A, trifocon A, unifocon A, vinafocon A, and wilofocon A.

Exemplary materials used to fabricate a hydrophilic lens with means to deliver the compositions of the invention include, but are not limited to, abafilcon A, acofilcon A, acofilcon B, acquafilcon A, alofilcon A, alphafilcon A, amfilcon A, astifilcon A, atlafilcon A, balafilcon A, bisfilcon A, bufilcon A, comfilcon A, crofilcon A, cyclofilcon A, darfilcon A, deltafilcon A, deltafilcon B, dimefilcon A, droxfilcon A, elastofilcon A, epsilfilcon A, esterifilcon A, etafilcon A, focofilcon A, galyfilcon A, genfilcon A, govafilcon A, hefilcon A, hefilcon B, hefilcon C, hilafilcon A, hilafilcon B, hioxifilcon A, hioxifilcon B, hioxifilcon C, hydrofilcon A, lenefilcon A, licryfilcon A, licryfilcon B, lidofilcon A, lidofilcon B, lotrafilcon A, lotrafilcon B, mafilcon A, mesafilcon A, methafilcon B, mipafilcon A, nelfilcon A, netrafilcon A, ocufilcon A, ocufilcon B, C, ocufilcon D, ocufilcon E, ofilcon A, omafilcon A, oxyfilcon A, pentafilcon A, perfilcon A, pevafilcon A, phemfilcon A, polymacon, senofilcon A, silafilcon A, siloxyfilcon A, surfilcon A, tefilcon A, tetrafilcon A, trilfilcon A, vifilcon A, vifilcon B, and xylofilcon A.

CEC Density, Morphology, and Identification of Pathology

Methods of detecting the morphology (e.g., shape), density, or number of CECs are well known in the art. These aspects of CECs may be determined non-invasively using various techniques. See, for example, McCarey et al. (2008) Review of Corneal Endothelial Specular Microscopy for FDA Clinical Trials of Refractive Procedures, Surgical Devices, and New Intraocular Drugs and Solutions. Cornea 27(1):1-16 (herein after "McCarey et al. 2008"); Patel et al. (2013) Quantitative analysis of in vivo confocal microscopy images: A review. Survey of Opthamology 58:466-475; Cornea: Fundamentals, Diagnosis and Management, Part ii, Section 3, Chapter 14, Specular Microscopy, pages 160-179, by Sayegh et al. Elsevier (2017); and Cornea: Fundamentals, Diagnosis and Management, Part ii, Section 3, Chapter 15, Confocal Microscopy, pages 180-191, by Petroll et al. Elsevier (2017). Non-limiting methods for evaluating CECs include *in vivo* confocal microscopy (using a confocal microscope) and non-invasive specular microscopy (using a specular microscope). These methods are not invasive.

According to McCarey et al. 2008, when the human corneal endothelium is damaged, the healing is a process of cellular enlargement and spreading to create a contiguous layer of cells on the inner surface of the cornea. In various embodiments, the degree of endothelial cell loss from, for example, disease, injury, or chemical toxicity can be detected with specular microscopy. In some embodiments, the degree of endothelial cell loss is detected as an increase in individual cell surface area and a decrease in the endothelial cell density for the cornea. In certain embodiments, corneal endothelial cell wound repair is also reflected as an increase in the variation of individual cell areas, *i.e.,* polymegethism or coefficient of variation (CV). According to McCarey et al. 2008, six-sided cells are an indication of an even distribution of membrane surface tension and of normal cells (the polygon that has the greatest surface area relative to its perimeter is the hexagon). Thus, the most efficient cell shape to cover a given area is the hexagon (*i.e.,* a perfect cornea should have 100% hexagons). In various embodiments, a healthy cornea has 60% of the endothelial cells as hexagons. In certain embodiments, stress to the endothelial cells results or has resulted in a decrease from the normal 60% distribution of 6-sided cells. In some embodiments, endothelial cell morphology analysis includes the following: cell area $\pm$ SD (square

micrometers), cell density (cells/square millimeter), polymegethism (CV), and pleomorphism (percentage of 6-sided cells).

In certain embodiments, cell density is determined from the average cell area with the following relationship in Equation 1:

$$\text{cell density} = \frac{10^6}{\text{average cell area}} \qquad (\text{Equation } 1)$$

with cell density (cells/square millimeter), average cell area (square micrometers), and the value $10^6$ is used to convert units of measure.

In various embodiments, a subject's corneal endothelium comprises cells of various surface areas. In some embodiments, a polymegethism value is detected or calculated. The polymegethism value is a coefficient describing the variation in cell area. According to McCarey et al. 2008, as the standard deviation (SD) of the average cell area increases, the accuracy of the estimated true cell density decreases. Therefore, in some embodiments, increases in polymegethism result in a decrease in the accuracy of the average cell area. In some embodiments, polymegethism is defined by the CV value determined with Equation 2.

$$\text{CV} = \frac{\text{SD}_{\text{cell area}}}{\text{mean cell area, } \mu\text{m}^2} \qquad (\text{Equation } 2)$$

with CV as coefficient of variation and SD as standard deviation of the mean cell area.

In various embodiments, a subject who has worn contact lenses for, *e.g.,* at least about 10, 15, 20, or 25 years or a subject with diabetes has CEC polymegethism while still retaining healthy cell density for the subject's age. In some embodiments, contact lens wear is stressing or has stressed the endothelium to alter the lateral endothelial cell borders, resulting in cells expressing a large anterior-surface area with a small posterior surface area or vice versa. Thus, in certain embodiments, polymegethism may not alter the cell volume while altering the appearance of the cell surface interfacing with the aqueous humor in the anterior chamber.

In some embodiments, the corneal endothelial surface area of a human subject is about 100, 110, 120, 130, 140, or 150 mm$^2$.

In various embodiments, the normal cell density of a 3, 4, 5, 6, or 3-6 year-old child is 3500-4000 cells/mm$^2$ (*e.g.,* there are 390,000-520,000 cells per cornea). Typically, this value decreases as the juvenile gets older and the corneal surface area increases. Graphic plots of this relationship are available in the literature. *See, e.g.,* McCarey et al. 1979 Ophthalmology 86:1848-1860; Hoffer 1979 Am J Ophthalmol 87:252-253; Yee et al. 1985 Curr Eye Res 4:671-678.

According to McCarey et al. 2008, published studies show that 3 year-old children can have 4000 cells/mm$^2$ (Laing et al. 1975 Arch Ophthalmol 93:143-145; McCarey et al. 1979 Ophthalmology 86:1848-1860), middle-aged adults (30 years) can have a range between 2700 and 2900 cells/mm$^2$ (Laing et al. 1975 Arch Ophthalmol 93:143-145; McCarey et al. 1979 Ophthalmology 86:1848-1860; Hoffer 1979 Am J Ophthalmol 87:252-253; Yee et al. 1985 Curr Eye Res 4:671-678), and adults older than 75 years can have a range of endothelial cell densities between 2400 and 2600 cells/mm$^2$ (Laing et al. 1975 Arch Ophthalmol 93:143-145; McCarey et al. 1979 Ophthalmology 86:1848-1860; Hoffer 1979 Am J Ophthalmol 87:252-253; Yee et al. 1985 Curr Eye Res 4:671-678). McCarey et al. 2008 indicates that these values represent the Caucasian race. According to McCarey et al. 2008, the Asian race has greater cell densities per given age group (Matsuda 1985 Arch Ophthalmol 103:68-70). In various embodiments, the range for these mean values can be significant.

In some embodiments, less than about 60%, 55%, 50%, 45%, 40%, 35%, 30%, or 25% of the CECs in a subject's cornea are in the shape of a hexagon (*i.e.,* are 6-sided cells when viewed perpendicular to the cornea, *e.g.,* from outside/above the curve of the comea). A CEC is in the shape of a hexagon if its outline when viewed from an angle that is perpendicular to the cornea has 6 sides. However, the sides need not be straight or equal in length, and the angle where each pair of two sides meets need not be the same.

In certain embodiments, the subject is administered a composition or treatment provided herein if the subject has a CEC density of less than about 3500, 3400, 3300, 3200, 3100, 3000, 2900, 2800, 2700, 2600, 2400, 2300, 2200, 2100, or 2000 cells/mm$^2$.

In various implementations, a subject is Caucasian or white (*i.e.,* the subject self-identifies as Caucasian or white). In some embodiments, the subject is administered a composition or treatment provided herein if the subject (a) is 3, 4, 5, or 6 years old and has a CEC density of less than about 3500, 3400, 3300, 3200, 3100, 3000, 2900, 2800, 2700, 2600, or 2500 cells/mm$^2$; (b) is about 30, 31, 32, 33, 34, 35, 36, 37, 38, 39, 40, 45, 50, 55, 60, 65, or 70 years old and has a CEC density of less than about 2700, 2600, 2500, 2400, 2300, 2200, 2100, or 2000 cells/mm$^2$; or (c) is at least about 75, 76, 77, 78, 79, 80, 81, 82, 83, 84, 85 years old has a CEC density of less than about 2400, 2300, 2200, 2100, 2000, 1900, 1800, 1700, 1600, or 1500 cells/mm$^2$.

In certain implementations, a subject is Asian (*i.e.,* the subject self-identifies as Asian). In various embodiments, the subject is administered a composition or treatment provided herein if the subject (a) is 3, 4, 5, or 6 years old and has a CEC density of less than about 4500, 4400, 4300, 4200, 4100, 4000, 3900, 3800, 3700, 3600, or 3500 cells/mm$^2$; (b) is about 30, 31, 32, 33, 34, 35, 36, 37, 38, 39, 40, 45, 50, 55, 60, 65, or 70 years old and has a CEC density of less than about 3700, 3600, 3500, 3400, 3300, 3200, 3100, or 3000 cells/mm$^2$; or (c) is at least about 75, 76, 77, 78, 79, 80, 81, 82, 83, 84, 85 years old has a CEC density of less than about 3400, 3300, 3200, 3100, 3000, 2900, 2800, 2700, 2600, or 2500 cells/mm$^2$.

In some embodiments, the subject is administered a composition or treatment provided herein if the subject is losing or has lost CECs at a rate of at least about 0.2%, 0.21%, 0.22%, 0.23%, 0.24%, 0.25%, 0.26%, 0.27%, 0.28%, 0.29%, 0.3%, 0.31%, 0.32%, 0.33%, 0.34%, 0.35% cell loss per year over a period of at least about 0.5, 1, 2, 3, 4, or 5 years. In certain embodiments, the subject is between 17 and 85 years old, *e.g.*, about 20, 22.5, 25, 27.5, 30, 35, 40, 45, 50, 55, 60, 65, 70, 75, 80, or 85 years old.

In certain embodiments, the subject is administered a composition or treatment provided herein if the subject has a polymegethism (CV) of at least about 0.3, 0.31, 0.32, 0.33, 0.34, 0.35, 0.36, 0.37, 0.38, 0.39, 0.40, 0.45, or 0.5.

In various embodiments, the subject is administered a composition or treatment provided herein if the cornea of the subject comprises less than about 400 thousand (k), 375k, 350k, 325k, 300k, 275k, or 250k CECs.

In some embodiments, treating a subject comprises preventing the CEC density of the subject from decreasing by more than about 50, 100, 150, 200, 250, 300, 350, 400, 450, or 500 cells/mm$^2$ within the first 1, 2, 3, 4, 5, 6, 12, 24, 26, 48, or 60 months after ocular surgery (*e.g.*, intraocular surgery, cataract surgery, cornea transplantation, injection of CECs into the eye (*e.g.*, cornea), glaucoma surgery, intraocular lens implantation, Descemet stripping, stripping automated endothelial keratoplasty, anterior keratoplasty, anterior lamellar keratoplasty, endothelial keratoplasty, Descemet membrane endothelial keratoplasty, Descemet stripping endothelial keratoplasty, Descemet membrane endothelial transfer, intraocular lens implantation, phototherapeutic keratectomy, penetrating keratoplasty, or laser eye surgery).

<u>General Definitions</u>

Unless specifically defined otherwise, all technical and scientific terms used herein shall be taken to have the same meaning as commonly understood by one of ordinary skill in the art (e.g., in cell culture, molecular genetics, and biochemistry).

As used herein, the term "about" in the context of a numerical value or range means ±10% of the numerical value or range recited or claimed, unless the context requires a more limited range.

In the descriptions above and in the claims, phrases such as "at least one of" or "one or more of" may occur followed by a conjunctive list of elements or features. The term "and/or" may also occur in a list of two or more elements or features. Unless otherwise implicitly or explicitly contradicted by the context in which it is used, such a phrase is intended to mean any of the listed elements or features individually or any of the recited elements or features in combination with any of the other recited elements or features. For example, the phrases "at least one of A and B;" "one or more of A and B;" and "A and/or B" are each intended to mean "A alone, B alone, or A and B together." A similar interpretation is also intended for lists including three or more items. For example, the phrases "at least one of A, B, and C;" "one or more of A, B, and C;" and "A, B, and/or C" are each intended to mean "A alone, B alone, C alone, A and B together, A and C together, B and C together, or A and B and C together." In addition, use of the term "based on," above and in the claims is intended to mean, "based at least in part on," such that an unrecited feature or element is also permissible.

It is understood that where a parameter range is provided, all integers within that range, and tenths thereof, are also provided by the invention. For example, "0.2-5 mg" is a disclosure of 0.2 mg, 0.3 mg, 0.4 mg, 0.5 mg, 0.6 mg etc. up to and including 5.0 mg.

As used herein, an "isolated" or "purified" nucleic acid molecule, polynucleotide, polypeptide, or protein, is substantially free of other cellular material, or culture medium when produced by recombinant techniques, or chemical precursors or other chemicals when chemically synthesized. Purified compounds are at least 60% by weight (dry weight) the compound of interest. Preferably, the preparation is at least 75%, more preferably at least 90%, and most preferably at least 99%, by weight the compound of interest. For example, a purified compound is one that is at least 90%, 91%, 92%, 93%, 94%, 95%, 98%, 99%, or 100% (w/w) of the desired compound by weight. Purity is measured by any appropriate standard method, for example, by column chromatography, thin layer chromatography, or high-performance liquid chromatography (HPLC) analysis. A purified or isolated polynucleotide (ribonucleic acid (RNA) or deoxyribonucleic acid (DNA)) is free of the genes or sequences that flank it in its naturally-occurring state. Similarly, a purified or isolated protein, protein fragment, or polypeptide is free of residues or amino acid sequences that flank the identified protein, fragment, or polypeptide in its naturally-occurring state. Purified also defines a degree of sterility that is safe for administration to a human subject, *e.g.*, lacking infectious or toxic agents.

Similarly, by "substantially pure" is meant a nucleotide or polypeptide that has been separated from the components that naturally accompany it. Typically, the nucleotides and polypeptides are substantially pure when they are at least 60%, 70%, 80%, 90%, 95%, or even 99%, by weight, free from the proteins and naturally-occurring organic molecules with they are naturally associated.

The transitional term "comprising," which is synonymous with "including," "containing," or "characterized by," is inclusive or open-ended and does not exclude additional, unrecited elements or method steps. By contrast, the transitional phrase "consisting of" excludes any element, step, or ingredient not specified in the claim. The transitional phrase "consisting essentially of" limits the scope of a claim to the specified materials or steps "and those that do not materially affect the basic and novel characteristic(s)" of the claimed invention.

As used herein, the singular forms "a," "an," and "the" include the plural reference unless the context clearly dictates otherwise. Thus, for example, a reference to "a disease," "a disease state", or "a nucleic acid" is a reference to one or more such embodiments, and includes equivalents thereof known to those skilled in the art and so forth.

As used herein, "monotherapy" means a therapy that is administered to treat a condition comprising CEC loss without any other therapy that is used to treat the condition. In certain embodiments, the monotherapy is a therapy that is administered to increase CEC survival, proliferation and/or migration without any other therapy that is used to increase CEC survival, proliferation, and/or migration. A monotherapy may optionally be combined with another treatment that is used to ameliorate a symptom of a condition while not being directed against the condition, but may not be combined with any other therapy directed against the condition (*e.g.*, directed against an underlying mechanism of cause of the condition). In some embodiments, agents that are not directed against the disorder, for example pain killers, may be administered concurrently or simultaneously with the monotherapy. The invention also encompasses combination therapy to treat a condition characterized by CEC loss or dysfunction.

A small molecule is a compound that is less than 2000 daltons in mass. The molecular mass of the small molecule is preferably less than 1000 daltons, more preferably less than 600 daltons, e.g., the compound is less than 500 daltons, 400 daltons, 300 daltons, 200 daltons, or 100 daltons.

## EXAMPLES

Examples are provided below to facilitate a more complete understanding of the invention. The following examples illustrate the exemplary modes of making and practicing the invention. However, the scope of the invention is not limited to specific embodiments disclosed in these Examples, which are for purposes of illustration only, since alternative methods can be utilized to obtain similar results.

Example 1: Alpha-Melanocyte Stimulating Hormone for Reduction of Corneal Endothelial Cell Loss

Nerve-derived molecules such as alpha-melanocyte stimulating hormone ($\alpha$-MSH) play a critical role in maintenance of CEC and can be used to prevent or reduce CEC in a variety of conditions.

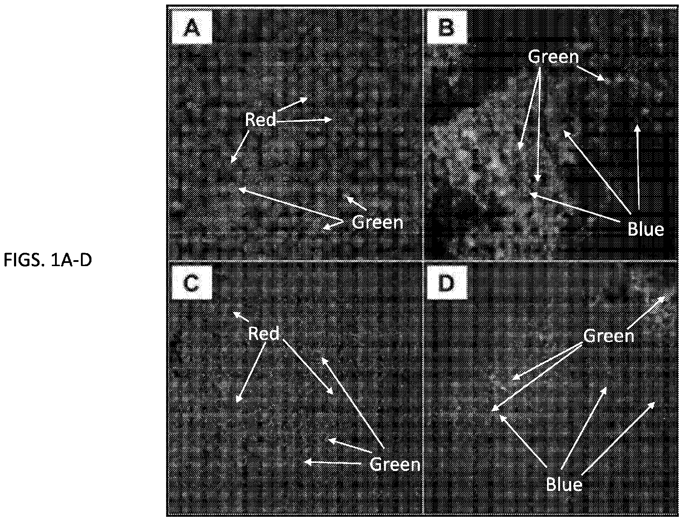

*In vitro* and *in vivo* studies have been performed. Immunohistochemical (IHC) staining of CEC in mice and humans showed that these cells express receptor for $\alpha$-MSH (FIG. 1).

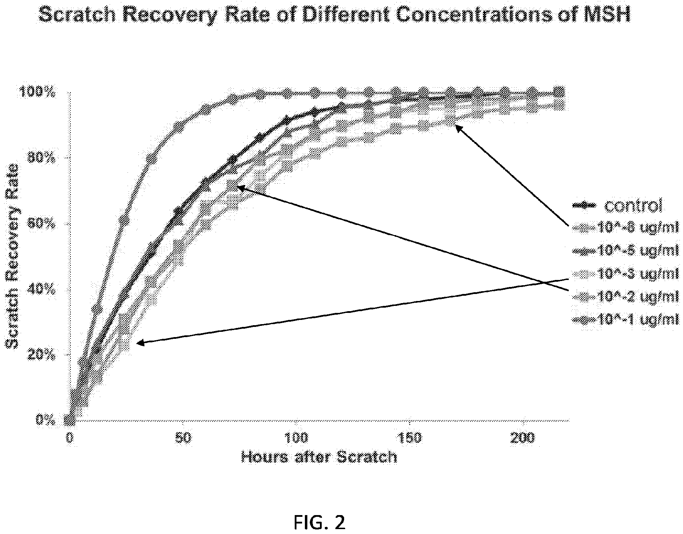

To evaluate the effect of $\alpha$-MSH on migration/proliferation of CEC, a scratch test was performed. In this test, a human CEC line was cultured on culture plates. After reaching confluence, a scratch was created on the cell sheet using a pipette tip. The culture medium contained different concentrations of $\alpha$-MSH. In the control group, no $\alpha$-MSH was used in the culture medium. Then, the culture plates were images at various time points. The percentage of reduction in the size of initial scratch was measured for each concentration of $\alpha$-MSH. As it is seen in FIG. 2, although low concentrations of $\alpha$-MSH had reduced the recovery rate, the high concentration resulted in a significant increase in the recovery rate showing the beneficial effects of this concentration on migration/proliferation of CEC.

To evaluate the effects of $\alpha$-MSH on reducing the induced apoptosis of CEC, the following *in vitro* test was performed. Corneas were harvested from mice. To induce apoptosis, these corneal were exposed to either interferon-$\gamma$ (IFN-y) 60 ng/ml or tumor necrosis factor-$\alpha$ (TNF-$\alpha$) 50 ng/ml for 18 hours. During this time, various concentrations of $\alpha$-MSH were used in the storage media. In the control group, no $\alpha$-MSH was used.

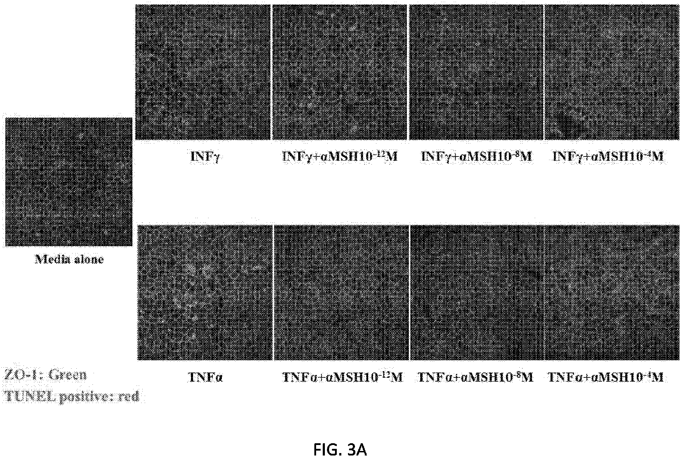

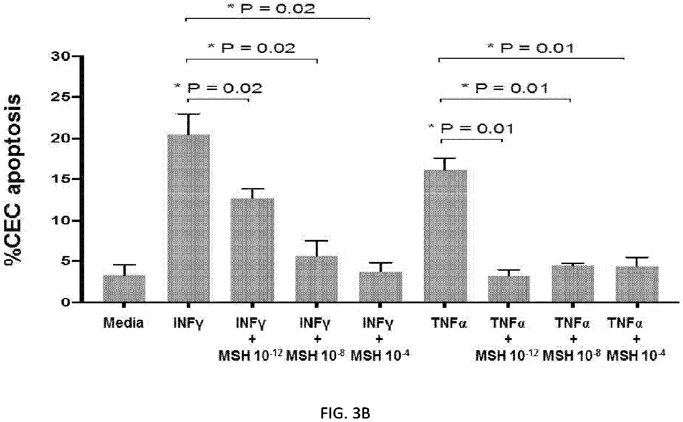

Percentage of apoptotic cells was then evaluated in each group using TUNEL assay. As FIGS. 3A and B reveal, there was a significant reduction of CEC apoptosis with using $\alpha$-MSH.

Example 2: The use of neuropeptides to prevent comeal endothelial cell loss in storage and transplantation.

Experiments are performed using neuropeptides to maintain CEC structure and function, in both preserved human donor corneas and in allogeneic transplantation. The following three effects are evaluated:

1) The effect of neuropeptides on CEC preservation prior to transplantation is determined.
2) The effect of neuropeptides on graft survival in low-risk allogeneic corneal transplantation is evaluated.
3) The effect of neuropeptides on graft survival in high-risk allogeneic corneal transplantation is evaluated.

Intact innervation of the cornea is required for maintenance of corneal structure and function (Müller et al. 2003 Exp Eye Res 76(5):521-42). Comeal nerves release neuropeptides, which are small protein molecules that have a multitude of effects. Neuropeptides have been shown to promote comeal epithelial migration and proliferation (Sabatino et al. 2017 Ocul Surf 15(1):2-14).

The monolayer of CECs is vital for the maintenance of corneal transparency.

Experiments are completed examining the role of neuropeptides using a murine model of syngeneic corneal transplantation. The results demonstrate that some neuropeptides both (i) improve the survival of CECs and (ii) reduce the cytotoxic effects of inflammatory cytokines on CECs. Since allogeneic corneal transplantation is the standard practice in humans, these analyses are extended to an allogeneic model of corneal transplantation. Furthermore, *ex vivo* experiments are performed to examine the capacity of neuropeptides to promote CEC survival in human donor comeas during storage preoperatively.

Determining the effect of neuropeptides on corneal preservation prior to transplantation:

Human corneas are divided into multiple experimental groups. The control group includes corneas stored in Optisol at 4°C. In each of the remaining experimental groups, different concentrations of a particular neuropeptide are added to the storage medium. Corneas are evaluated using standard eye bank methodologies, *e.g.*, specular microscopy image quality assessment according to the SMAS study (*see, e.g.,* Benetz BA, Gal RL, Ruedy KJ, Rice C, Beck RW, Kalajian AD, Lass JH; Cornea Donor Study Group. Specular microscopy ancillary study methods for donor endothelial cell density determination of Cornea Donor Study images. Curr Eye Res. 2006 Apr;31(4):319-27). The CEC density is determined using immunohistochemical analysis at days 7 and 14 to determine the percentage of CEC loss in each group.

The neuropeptide $\alpha$-MSH decreases CEC loss during storage.

Evaluating the effect of neuropeptides on graft survival in low-risk allogeneic corneal transplantation:

Allogeneic corneal transplantation is performed in BALB/c mice in accordance with a standard protocol for murine orthotopic corneal transplantation described previously (Chauhan et al. 2009 J Immunol 182(1):148-53). Following surgery, mice are divided into multiple treatment groups. Intracameral injection of different neuropeptides is performed in the experimental groups, whilst the control group receives intracameral injection of saline solution only. Mice are monitored for 8 weeks to determine graft survival. Optical Coherence Tomography (OCT) and ZO-1 staining is performed every 7 days to assess corneal thickness and CEC density, respectively.

The neuropeptide $\alpha$-MSH increases graft survival in low-risk allogeneic corneal transplantation.

Evaluating the effect of neuropeptides on graft survival in high-risk allogeneic corneal transplantation:

A well-established model of high-risk corneal transplantation described previously is used (Jin et al. 2010 Investig Opthalmology Vis Sci 51(2):816). Briefly, three interrupted 8-shaped sutures are placed in the corneas of BALB/c mice 14 days prior to orthotopic corneal transplantation to induce neovascularization and inflammation in the host beds. Then, allogeneic corneal transplantation is performed in these BALB/c mice and intracameral injection of different neuropeptides or saline is given to mice after the transplantation. Mice are followed for 8 weeks and corneal thickness and CEC density are evaluated.

The neuropeptide $\alpha$-MSH increases graft survival in high-risk allogeneic corneal transplantation.

Example 3: Neuropeptides for Improving Graft Survival in Corneal Transplantation

The effects of nerve-derived molecules on the survival of CECs are studied. The following experiments are performed:

1. Determining the presence of receptors for neuropeptides on CECs:
Without wishing to be bound by any scientific theory, for neuropeptides to have any effect on CECs, a receptor needs to be on the cells. In this experiment, presence of receptors for various nerve-derived molecules (such as $\alpha$-MSH) on CECs is determined using different techniques.
2. Identifying the neuropeptides that enhance CEC proliferation/migration:
The ability of certain neuropeptides such as $\alpha$-MSH to enhance proliferation/migration of CECs is confirmed. In these experiments, both human and mouse cultured CECs undergo injury, and then the proliferation/migration of CECs is compared with and without addition of neuropeptides (such as α-MSH).

3. Determining the effect of addition of neuropeptides on CECs after corneal transplantation in mouse:

Corneal transplantation is a procedure in which the central part of the cornea is replaced by the cornea from another person. Significant reduction of CECs is a major reason for failure of this procedure. Therefore, strategies to enhance CEC survival, such as those disclosed herein, improve the outcome of corneal transplantation. In this experiment, before transplanting the cornea from one mouse to another, CECs of the donor cornea are destroyed mechanically. Then, the survival of the remaining CECs after the transplantation is compared with and without addition of neuropeptides such as α-MSH. Furthermore, to simulate the condition commonly seen in human corneal transplantation, corneal vessels are induced in one subset of mice before transplantation. Then, the survival of corneal transplants is compared with and without addition of neuropeptides such as α-MSH.

Neuropeptides that improve CEC survival can be used in a variety applications and conditions to reduce CEC loss. Non-limiting examples include the treatment of patients with different eye and systemic conditions, as well as following many forms of eye surgeries such as corneal transplantation. The compositions provided herein are useful in methods for treating people suffering from complications of CEC loss, and can reduce or prevent corneal blindness in many patients.

The best example of nerve injury in the cornea includes full-thickness corneal transplantation (penetrating keratoplasty) in which all corneal nerves are severed in the mid-peripheral cornea. It is well established that there is a continuous CEC loss after penetrating keratoplasty even years after the surgery. Such CEC loss is very important as it is the major cause of corneal graft failure.

Without wishing to be bound by any scientific theory, one mechanism involved in prolonged CEC loss after corneal transplantation is corneal nerve injury which takes years, if ever, to regenerate completely. In various embodiments, exogenous replacement of nerve-derived molecules prevents CEC loss and thus improve the survival of corneal graft. These experiments investigate the role of nerve-derived molecules on the survival of corneal grafts. α-MSH is determined to be a key neuropeptide involved in the maintenance of CECs provides important therapeutic strategies to prevent the CEC loss in those with corneal transplantation and thus improving the graft survival.

*In vitro* and *in vivo* models are used in the following experiments.

1. Determining the expression of receptors for neuropeptides on CECs:

To be modulated by neuropeptides, CECs need to express the receptor for these molecules. The expression of receptors for several neuropeptides on CECs is evaluated using immunohistochemical staining and quantitative Real-Time Polymerase Chain Reaction (PCR) techniques. For immunohistochemical study, double staining is performed for the CEC and neuropeptide markers in freshly prepared sections from normal human donor cornea as well as naive mouse cornea. For CEC immunostaining, an antibody against Zonula Occludens-1 (ZO-1) is used. For neuropeptides, the expression of receptors for nerve growth factor (NGF), substance P, calcitonin gene-related peptide (CGRP), vasoactive intestinal polypeptide (VIP), neurotrophin 3, 4/5, and 6, brain-derived neurotrophic factor (BDNF), and α-MSH is evaluated.

In addition, the expression of these neuropeptides is also investigated in cultured human and mouse CECs. Primary cells or established CEC lines are used. After reaching 70% of confluence, the quantity of the above-mentioned neuropeptides is determined using immunohistochemical staining as well as quantitative Real-Time PCR.

CECs express receptors for some neuropeptides (FIG. 1).

2. Identifying the neuropeptides that enhance CEC proliferation/migration in culture:

To identify the neuropeptides which have a supportive role for CECs, human and mouse CEC cultures are used. After reaching confluence in culture plates, the effects of adding the above-mentioned neuropeptides at different concentrations on CEC proliferation and migration is investigated using scratch test. Concentrations of neuropeptides increase the migration of CECs and thus the speed of recovery from the scratch test.

In addition, the effects of addition of the neuropeptides on an *in vitro* model of CEC apoptosis is studied in human and mouse CEC cultures. For this, after the cells reach confluence in culture plates, CEC apoptosis is induced by a validated model using hydrogen peroxide (Hull 1981 Acta Ophthalmol (Copenh) 59:409-21). The effects of adding the above-mentioned neuropeptides on the CEC proliferation and migration are investigated using BrdU labeling and the scratch test.

3. Determining the effect of *in vivo* addition of neuropeptides after syngeneic murine corneal transplantation:

An art-recognized murine model of corneal transplantation is used. To avoid any immunologic reaction after transplantation, syngeneic corneal graft is employed in BALBc mice. After removing the cornea from the donor mice, its endothelium is removed mechanically. Then, the cornea is transplanted to the recipient mouse using the standard technique of transplantation. After surgery, the mice are divided into two groups. To show the effects of neuropeptides on the CEC survival, in one group neuropeptides is injected intracamerally and the other group serves as the control group. The following is evaluated during 8 weeks of follow-up: corneal opacity score (by slit lamp examination), density of CEC (by immunohistochemical staining for ZO-1), and corneal thickness (by Optical Coherence Tomography [OCT]). Treatment with some neuropeptides results in reduced corneal thickness and opacity after syngeneic corneal transplantation compared with the control group.

4. To determine the effect of *in vivo* addition of neuropeptides after high-risk allogeneic murine corneal transplantation:

An art-recognized murine model of high-risk allogeneic corneal transplantation is used, as described before (Qian et al. 2002 Cornea 21:592-7; Qian et al. 2002 J Interferon Cytokine Res 22:1217-25). Here, the donor is a C57BL/6 mouse and the recipient is a BALBc mouse. To produce a high-risk environment, corneal neovascularization is induced in the recipient eye by using corneal suturing prior to transplantation. Corneal transplantation is then performed as described above. After surgery, the mice are divided into two groups, one group receives intracameral neuropeptide and the other group serves as the control group. The following is evaluated during 8 weeks of follow-up: corneal opacity score (by slit lamp examination), density of CEC (by immunohistochemical staining for ZO-1), and corneal thickness (by Optical Coherence Tomography [OCT]). Treatment with some neuropeptides results in increased graft survival and decreased corneal opacity after this high-risk allogeneic corneal transplantation compared with the control group.

## Claims

1. A melanocortin receptor agonist comprising an α-melanocyte stimulating hormone (α-MSH) or a melanocortin receptor binding derivative of α-MSH, for use in a method for treating or preventing corneal endothelial cell (CEC) loss in a subject, comprising locally administering to an eye of the subject a composition comprising an effective amount of the α-MSH or the melanocortin receptor binding derivative of α-MSH.

2. The melanocortin receptor agonist for use of claim 1, wherein:

   (i) the subject comprises a corneal injury, a corneal dystrophy, an anterior corneal dystrophy, a stromal corneal dystrophy, a posterior corneal dystrophy, corneal endothelial dystrophy, Fuchs endothelial dystrophy, congenital hereditary endothelial dystrophy, posterior polymorphous corneal dystrophy, Schnyder crystalline corneal dystrophy, bullous keratopathy, an iridocorneal endothelial syndrome, keratitis, photokeratitis, neurotrophic keratophy, pseudoexfoliation syndrome, ocular hypertension, glaucoma, an ocular infection, a cataract, corneal endothelial cell loss due to contact lens wear, corneal endothelial cell loss due to aging, uveitis, intraocular inflammation, inflammatory disciform keratitis, diabetes, or dry eye disease,
   or
   (ii) the subject comprises a non-inflammatory ocular disorder, optionally wherein the non-inflammatory ocular disorder is a non-autoimmune ocular disorder, optionally wherein the non-autoimmune ocular disorder comprises a corneal injury, a corneal dystrophy, an anterior corneal dystrophy, a stromal corneal dystrophy, a posterior corneal dystrophy, corneal endothelial dystrophy, Fuchs endothelial dystrophy, congenital hereditary endothelial dystrophy, posterior polymorphous corneal dystrophy, Schnyder crystalline corneal dystrophy, bullous keratopathy, an iridocorneal endothelial syndrome, keratitis, neurotrophic keratopathy, ocular hypertension, glaucoma, diabetes, a cataract, an ocular infection, corneal endothelial cell loss due to contact lens wear, or corneal endothelial cell loss due to aging.

3. The melanocortin receptor agonist for use of claim 1, wherein the subject has been scheduled to receive ocular surgery, the subject has been diagnosed as in need of ocular surgery, the subject is receiving ocular surgery, or the subject has received ocular surgery, optionally:

   (i) wherein the surgery comprises intraocular surgery, cataract surgery, glaucoma surgery, cornea transplantation, intraocular lens implantation, injection of CECs into the eye, Descemet stripping, Descemet stripping automated endothelial keratoplasty, anterior keratoplasty, anterior lamellar keratoplasty, endothelial keratoplasty, Descemet membrane endothelial keratoplasty, Descemet stripping endothelial keratoplasty, Descemet membrane endothelial transfer, phototherapeutic keratectomy, penetrating keratoplasty, or laser eye surgery,
   or
   (ii) wherein the surgery comprises vision corrective surgery, optionally wherein the surgery comprises laser vision corrective surgery.

4. The melanocortin receptor agonist for use of claim 1,

wherein donor CECs have been administered to the subject, optionally wherein the CECs have been injected into an eye of the subject.

5. The melanocortin receptor agonist for use of claim 1, for use in a method for treating or preventing CEC loss associated with aging, optionally wherein the subject is at least about 0.5, 1, 5, 10, 15, 20, 25, 30, 35, 40, 45, 50, 55, 60, 65, 70, 75, 80, 85, or 90 years old.

6. The melanocortin receptor agonist for use of claim 1, wherein the subject comprises an ocular infection,

optionally wherein the ocular infection comprises an infection by a virus, bacterium, fungus, or protozoan, optionally wherein the ocular infection comprises a protozoan infection by an acanthamoeba or the ocular infection comprises a keratitis infection by a herpes simplex virus.

7. The melanocortin receptor agonist for use of claim 1, wherein the subject comprises conjunctivitis, optionally wherein the conjunctivitis comprises viral, allergic, bacterial, or chemical conjunctivitis.

8. The melanocortin receptor agonist for use of claim 1, wherein the subject comprises corneal endothelial cell loss due to contact lens wear, and the subject has worn contact lenses at least once per month for at least about 5 years.

9. The melanocortin receptor agonist for use of claim 1, wherein:

(i) the effective amount is effective to increase the number of CECs in the subject,
or
(ii) the effective amount is effective to slow a decrease in the number of CECs in the subject, optionally wherein the effective amount is effective to prevent the density of CECs in the cornea of the subject from decreasing by more than about 50, 100, 150, 200, 250, 300, 350, 400, 450, or 500 cells/mm$^2$ within the first 6 months after ocular surgery,
or
(iii) the effective amount is effective to reduce apoptosis of CECs in the subject,
or
(iv) the effective amount is effective to increase proliferation of CECs in the subject,
or
(v) the effective amount is effective to increase migration of CECs in the subject.

10. The melanocortin receptor agonist for use of claim 1,

(i) wherein the composition is in the form of an aqueous solution, a solid, an ointment, a gel, a liquid, a hydrogel, an aerosol, a mist, a polymer, a contact lens, a film, an emulsion, or a suspension,
or
(ii) wherein said composition is administered topically, optionally wherein the composition is topically administered to the eye of the subject,
or
(iii) wherein said method does not comprise systemic administration or substantial dissemination to non-ocular tissue of the subject,
or
(iv) wherein said method further comprises administering nerve growth factor (NGF) or vasoactive intestinal polypeptide (VIP).

11. The melanocortin receptor agonist for use of claim 1, wherein:

(i) the composition is administered to the eye of the subject

(a) less than 1, 2, 3, 4, 5, or 6 times per day;
(b) about 1, 2, 3, 4, 5, 6, or 7 times per week; or
(c) once daily,

optionally, wherein the composition is administered by the subject.

12. The melanocortin receptor agonist for use of claim 1, further comprising detecting CECs of the subject before or after administration of the melanocortin receptor agonist,
optionally wherein detecting CEC function comprises measuring corneal thickness with optical coherence tomography (OCT), or wherein detecting CECs of the subject comprises detecting the morphology, density, or number of CECs in the cornea of the subject.

13. The melanocortin receptor agonist for use of claim 12, wherein less than about 60%, 55%, 50%, 45%, 40%, 35%, 30%, 25%, 20%, 15%, 10%, 5%, 4%, 3%, 2%, 1%, 0.5%, 0.1%, 0.01%, 0.001%, or 0.0001% of the CECs in the subject's cornea are in the shape of a hexagon, or wherein none of the CECs in the subject's cornea are in the shape of a hexagon.

**Patentansprüche**

1. Melanocortin-Rezeptor-Agonist, umfassend ein $\alpha$-Melanozyten-stimulierendes Hormon ($\alpha$-MSH) oder ein Melanocortin-Rezeptor-bindendes Derivat von $\alpha$-MSH, zur Verwendung in einem Verfahren zur Behandlung oder Verhinderung des Verlusts von Hornhautendothel-zellen (CEC) bei einem Subjekt, umfassend die lokale Verabreichung einer Zusammen-setzung, umfassend eine wirksame Menge des $\alpha$-MSH oder des Melanocortin-Rezeptor-bindenden Derivats von $\alpha$-MSH, an ein Auge des Subjekts.

2. Melanocortin-Rezeptor-Agonist zur Verwendung nach Anspruch 1, wobei:

(i) das Subjekt eine Hornhautverletzung, eine Hornhautdystrophie, eine anteriore Hornhautdystrophie, eine stromale Hornhautdystrophie, eine posteriore Hornhautdystrophie, ei-ne Hornhautendotheldystrophie, eine Fuchs'sche Endotheldystrophie, eine kongenitale hereditäre Endotheldystrophie, eine posteriore polymorphe Hornhautdystrophie, eine kristalline Hornhautdystrophie nach Schnyder, eine bullöse Keratopathie, ein iridokorneales Endothelsyndrom, eine Keratitis, eine Photokeratitis, eine neurotrophe Keratophyse, ein Pseudoexfoliationssyndrom, eine okuläre Hypertension, ein Glaukom, eine Augeninfekti-on, ein Katarakt, einen Verlust von Hornhautendothelzellen aufgrund des Tragens von Kontaktlinsen, einen Verlust von Hornhautendothelzellen aufgrund des Alterns, eine Uveitis, eine intraokuläre Entzündung, eine entzündliche scheibenförmige Keratitis, Diabetes oder eine Erkrankung in der Form von trockenen Augen aufweist, oder
(ii) das Subjekt eine nicht-entzündliche Augenerkrankung aufweist, wobei die nicht-entzündliche Augenerkrankung eine nicht-autoimmune Augenerkrankung ist, wobei die nicht-autoimmune Augenerkrankung eine Hornhautverletzung, eine Hornhautdystrophie, eine anteriore Hornhautdystrophie, eine stromale Hornhautdystrophie, eine posteriore Hornhautdystrophie, eine Hornhautendotheldystrophie, eine Fuchs'sche Endotheldystro-phie, eine kongenitale hereditäre endotheliale Dystrophie, eine posteriore polymorphe Hornhautdystrophie, eine kristalline Hornhautdystrophie nach Schnyder, eine bullöse Keratopathie, ein iridokorneales Endothel-Syndrom, eine Keratitis, eine neurotrophe Keratopathie, eine okuläre Hypertension, ein Glaukom, Diabetes, ein Katarakt, eine Augeninfektion, einen Verlust von Hornhautendothelzellen durch das Tragen von Kontaktlinsen oder einen Verlust von Hornhautendothelzellen aufgrund des Alterns umfasst.

3. Melanocortin-Rezeptor-Agonist zur Verwendung nach Anspruch 1, wobei bei dem Subjekt eine Augenoperation geplant ist, bei dem Subjekt die Notwendigkeit einer Augenoperation diagnostiziert wurde, bei dem Subjekt eine Augenoperation durchgeführt wird oder bei dem Subjekt eine Augenoperation durchgeführt wurde, optional:

(i) wobei die Operation eine intraokuläre Operation, eine Kataraktoperation, eine Glaukom-operation, eine Hornhauttransplantation, eine intraokuläre Linsenimplantation, eine Injektion von CECs in das Auge, ein Descemet-Stripping, eine Descemet-Strippingautomatisierte endotheliale Keratoplastik, eine anteriore Keratoplastik, eine anteriore lamelläre Keratoplastik, eine endotheliale Keratoplastik, eine Descemet-Membran-Endothel-Keratoplastik, eine Descemet-Stripping-Endothel-Keratoplastik, einen Descemet-Membran-Endothel-Transfer, eine phototherapeutische Keratektomie, eine perforierende Keratoplastik oder ei-ne Laser-Augenoperation umfasst, oder
(ii) wobei die Operation eine Sehkorrekturoperation umfasst, wobei die Operation optional eine Laser-Sehkorrekturoperation umfasst.

4. Melanocortin-Rezeptor-Agonist zur Verwendung nach Anspruch 1,

wobei dem Subjekt Donator-CECs verabreicht wurden, wobei optional die CECs in ein Auge des Subjekts injiziert wurden.

5. Melanocortin-Rezeptor-Agonist zur Verwendung nach Anspruch 1, zur Verwendung in einem Verfahren zur Behandlung oder Verhinderung von CEC-Verlust, der mit dem Altern einhergeht, wobei das Subjekt optinional wenigstens etwa 0,5, 1, 5, 10, 15, 20, 25, 30, 35, 40, 45, 50, 55, 60, 65, 70, 75, 80, 85 oder 90 Jahre alt ist.

6. Melanocortin-Rezeptor-Agonist zur Verwendung nach Anspruch 1, wobei das Subjekt eine Augeninfektion aufweist,

   wobei optional die Augeninfektion eine Infektion durch ein Virus, ein Bakterium, einen Pilz oder ein Protozoon umfasst,
   wobei optional die Augeninfektion eine Protozoeninfektion durch eine Acanthamöbe oder die Augeninfektion eine Keratitisinfektion durch ein Herpes-simplex-Virus umfasst.

7. Melanocortin-Rezeptor-Agonist zur Verwendung nach Anspruch 1, wobei das Subjekt eine Bindehautentzündung aufweist,
   wobei optional die Bindehautentzündung eine virale, allergische, bakterielle oder chemische Bindehautentzündung umfasst.

8. Melanocortin-Rezeptor-Agonist zur Verwendung nach Anspruch 1, wobei das Subjekt einen Verlust von Hornhautendothelzellen durch das Tragen von Kontaktlinsen aufweist und das Subjekt wenigstens einmal pro Monat für wenigstens etwa 5 Jahre Kontaktlinsen getragen hat.

9. Melanocortin-Rezeptor-Agonist zur Verwendung nach Anspruch 1, wobei:

   (i) die wirksame Menge wirksam ist, um die Anzahl der CECs im Subjekt zu erhöhen,
   oder
   (ii) die wirksame Menge wirksam ist, um eine Abnahme der Anzahl von CECs im Subjekt zu verlangsamen, wobei optional die wirksame Menge wirksam ist, um zu verhindern, dass die Dichte von CECs in der Hornhaut des Subjekts um mehr als etwa 50, 100, 150, 200, 250, 300, 350, 400, 450 oder 500 Zellen/mm2 innerhalb der ersten 6 Monate nach der Augenoperation abnimmt,
   oder
   (iii) die wirksame Menge wirksam ist, um die Apoptose von CECs im Subjekt zu reduzieren,
   oder
   (iv) die wirksame Menge wirksam ist, um die Vermehrung von CEC im Subjekt zu erhöhen,
   oder
   (v) die wirksame Menge wirksam ist, um die Migration von CECs im Subjekt zu erhöhen.

10. Melanocortin-Rezeptor-Agonist zur Verwendung nach Anspruch 1,

    (i) wobei die Zusammensetzung in Form einer wässrigen Lösung, eines Feststoffs, einer Salbe, eines Gels, einer Flüssigkeit, eines Hydrogels, eines Aerosols, eines Nebels, eines Polymers, einer Kontaktlinse, eines Films, einer Emulsion oder einer Suspension vorliegt,
    oder
    (ii) wobei die Zusammensetzung topisch verabreicht wird, wobei optional die Zusammen-setzung topisch auf das Auge des Subjekts verabreicht wird,
    oder
    (iii) wobei das Verfahren keine systemische Verabreichung oder wesentliche Verbreitung in nicht-okulärem Gewebe des Subjekts umfasst,
    oder
    (iv) wobei das Verfahren ferner die Verabreichung von Nervenwachstumsfaktor (NGF) oder vasoaktivem intestinalem Polypeptid (VIP) umfasst.

11. Melanocortin-Rezeptor-Agonist zur Verwendung nach Anspruch 1, wobei:

    (i) die Zusammensetzung auf das Auge des Subjekts verabreicht wird

       (a) weniger als 1, 2, 3, 4, 5 oder 6 Mal pro Tag;

(b) etwa 1, 2, 3, 4, 5, 6 oder 7 Mal pro Woche; oder
(c) einmal täglich,

wobei optional die Zusammensetzung durch das Subjekt verabreicht wird.

12. Melanocortin-Rezeptor-Agonist zur Verwendung nach Anspruch 1, ferner umfassend das Erfassen von CECs des Subjekts vor oder nach Verabreichung des Melanocortin-Rezeptor-Agonisten,
wobei optional das Erfassen der CEC-Funktion das Messen der Hornhautdicke mit optischer Kohärenztomographie (OCT) umfasst oder wobei das Erfassen von CECs des Subjekts das Erfassen der Morphologie, Dichte oder Anzahl der CECs in der Hornhaut des Subjekts umfasst.

13. Melanocortin-Rezeptor-Agonist zur Verwendung nach Anspruch 12, wobei weniger als etwa 60 %, 55 %, 50 %, 45 %, 40 %, 35 %, 30 %, 25 %, 20 %, 15 %, 10 %, 5 %, 4 %, 3 %, 2 %, 1 %, 0,5 %, 0,1 %, 0,01 %, 0,001 % oder 0,0001 % der CECs in der Hornhaut des Subjekts die Form eines Sechsecks aufweisen oder wobei keines der CECs in der Hornhaut des Subjekts die Form eines Sechsecks aufweist.

**Revendications**

1. Agoniste du récepteur de la mélanocortine comprenant une hormone $\alpha$ de stimulation des mélanocytes ($\alpha$-MSH) ou un dérivé de l'$\alpha$-MSH se liant au récepteur de la mélanocortine, pour utilisation dans un procédé de traitement ou de prévention de la perte de cellules endothéliales cornéennes (CEC) chez un sujet, comprenant l'administration locale à un œil du sujet d'une composition comprenant une quantité efficace de l'$\alpha$-MSH ou du dérivé de l'$\alpha$-MSH se liant au récepteur de la mélanocortine.

2. Agoniste du récepteur de la mélanocortine pour utilisation selon la revendication 1, dans lequel :

(i) le sujet comprend une lésion cornéenne, une dystrophie cornéenne, une dystrophie cornéenne antérieure, une dystrophie cornéenne stromale, une dystrophie cornéenne postérieure, une dystrophie endothéliale cornéenne, une dystrophie endothéliale de Fuchs, une dystrophie endothéliale héréditaire congénitale, une dystrophie cornéenne polymorphe postérieure, une dystrophie cornéenne cristalline de Schnyder, une kératopathie bulleuse, un syndrome irido-cornéen endothélial, une kératite, une photokératite, une kératophytie neurotrophique, un syndrome de pseudo-exfoliation, une hypertension oculaire, un glaucome, une infection oculaire, une cataracte, une perte de cellules endothéliales cornéennes due au port de lentilles de contact, une perte de cellules endothéliales cornéennes due au vieillissement, une uvéite, une inflammation intraoculaire, une kératite disciforme inflammatoire, un diabète ou une sécheresse oculaire,
ou
(ii) le sujet comprend un trouble oculaire non inflammatoire, éventuellement dans lequel le trouble oculaire non inflammatoire est un trouble oculaire non auto-immunitaire, éventuellement dans lequel le trouble oculaire non auto-immunitaire comprend une lésion cornéenne, une dystrophie cornéenne, une dystrophie cornéenne antérieure, une dystrophie cornéenne stromale, une dystrophie cornéenne postérieure, une dystrophie endothéliale cornéenne, une dystrophie endothéliale de Fuchs, une dystrophie endothéliale héréditaire congénitale, une dystrophie cornéenne polymorphe postérieure, une dystrophie cornéenne cristalline de Schnyder, une kératopathie bulleuse, un syndrome irido-cornéen endothélial, une kératite, une kératopathie neurotrophique, une hypertension oculaire, un glaucome, un diabète, une cataracte, une infection oculaire, une perte de cellules endothéliales cornéennes due au port de lentilles de contact, ou une perte de cellules endothéliales cornéennes due au vieillissement.

3. Agoniste du récepteur de la mélanocortine pour utilisation selon la revendication 1, dans lequel le sujet est programmé pour recevoir une chirurgie oculaire, le sujet a été diagnostiqué comme nécessitant une chirurgie oculaire, le sujet reçoit une chirurgie oculaire ou le sujet a reçu une chirurgie oculaire, éventuellement :

(i) dans lequel la chirurgie comprend une chirurgie intraoculaire, une chirurgie de la cataracte, une chirurgie du glaucome, une greffe de cornée, une implantation d'une lentille intraoculaire, une injection de CEC dans l'œil, un stripping de Descemet, une kératoplastie endothéliale automatisée par stripping de Descemet, une kératoplastie antérieure, une kératoplastie lamellaire antérieure, une kératoplastie endothéliale, une kératoplastie endothéliale de la membrane de Descemet, une kératoplastie endothéliale par stripping de Descemet, un transfert endothélial de la membrane de Descemet, une kératectomie photothérapeutique, une kératoplastie pénétrante ou une chirurgie oculaire au laser,

ou

(ii) dans lequel la chirurgie comprend une chirurgie corrective de la vision, éventuellement dans lequel la chirurgie comprend une chirurgie corrective de la vision au laser.

**4.** Agoniste du récepteur de la mélanocortine pour utilisation selon la revendication 1, dans lequel des CEC de donneur ont été administrées au sujet, éventuellement dans lequel les CEC ont été injectées dans un œil du sujet.

**5.** Agoniste du récepteur de la mélanocortine pour utilisation selon la revendication 1, pour utilisation dans un procédé de traitement ou de prévention de la perte de CEC associée au vieillissement, éventuellement dans lequel le sujet est âgé d'au moins environ 0,5, 1, 5, 10, 15, 20, 25, 30, 35, 40, 45, 50, 55, 60, 65, 70, 75, 80, 85 ou 90 ans.

**6.** Agoniste du récepteur de la mélanocortine pour utilisation selon la revendication 1, dans lequel le sujet comprend une infection oculaire,

éventuellement dans lequel l'infection oculaire comprend une infection par un virus, une bactérie, un champignon ou un protozoaire,
éventuellement dans lequel l'infection oculaire comprend une infection protozoaire par un acanthamoeba ou l'infection oculaire comprend une infection kératite par un virus herpès simplex.

**7.** Agoniste du récepteur de la mélanocortine pour utilisation selon la revendication 1, dans lequel le sujet comprend une conjonctivite,
éventuellement dans lequel la conjonctivite comprend une conjonctivite virale, allergique, bactérienne ou chimique.

**8.** Agoniste du récepteur de la mélanocortine pour utilisation selon la revendication 1, dans lequel le sujet comprend une perte de cellules endothéliales cornéennes due au port de lentilles de contact, et le sujet a porté des lentilles de contact au moins une fois par mois pendant au moins environ 5 ans.

**9.** Agoniste du récepteur de la mélanocortine pour utilisation selon la revendication 1, dans lequel :

(i) la quantité efficace est efficace pour augmenter le nombre de CEC chez le sujet,
ou
(ii) la quantité efficace est efficace pour ralentir une diminution du nombre de CEC chez le sujet, éventuellement dans lequel la quantité efficace est efficace pour empêcher la densité des CEC dans la cornée du sujet de diminuer de plus d'environ 50, 100, 150, 200, 250, 300, 350, 400, 450 ou 500 cellules/mm2 au cours des 6 premiers mois suivant une chirurgie oculaire,
ou
(iii) la quantité efficace est efficace pour réduire l'apoptose des CEC chez le sujet,
ou
(iv) la quantité efficace est efficace pour augmenter la prolifération des CEC chez le sujet,
ou
(v) la quantité efficace est efficace pour augmenter la migration des CEC chez le sujet.

**10.** Agoniste du récepteur de la mélanocortine pour utilisation selon la revendication 1,

(i) dans lequel la composition est sous la forme d'une solution aqueuse, d'un solide, d'une pommade, d'un gel, d'un liquide, d'un hydrogel, d'un aérosol, d'une brumisation, d'un polymère, d'une lentille de contact, d'un film, d'une émulsion ou d'une suspension,
ou
(ii) dans lequel ladite composition est administrée topiquement, éventuellement dans lequel la composition est administrée topiquement à l'œil du sujet,
ou
(iii) dans lequel ledit procédé ne comprend pas d'administration systémique ou de dissémination substantielle dans les tissus non oculaires du sujet,
ou
(iv) dans lequel ledit procédé comprend en outre l'administration d'un facteur de croissance nerveuse (NGF) ou d'un polypeptide intestinal vasoactif (VIP).

**11.** Agoniste du récepteur de la mélanocortine pour utilisation selon la revendication 1, dans lequel :

(i) la composition est administrée à l'œil du sujet

(a) moins de 1, 2, 3, 4, 5 ou 6 fois par jour ;
(b) environ 1, 2, 3, 4, 5, 6 ou 7 fois par semaine ; ou
(c) une fois par jour,

éventuellement, dans lequel la composition est administrée par le sujet.

**12.** Agoniste du récepteur de la mélanocortine pour utilisation selon la revendication 1, comprenant en outre la détection des CEC du sujet avant ou après l'administration de l'agoniste du récepteur de la mélanocortine, dans lequel, éventuellement, la détection de la fonction des CEC comprend la mesure de l'épaisseur cornéenne à l'aide d'une tomographie par cohérence optique (OCT), ou dans lequel la détection des CEC du sujet comprend la détection de la morphologie, de la densité ou du nombre de CEC dans la cornée du sujet.

**13.** Agoniste du récepteur de la mélanocortine pour utilisation selon la revendication 12, dans lequel moins d'environ 60 %, 55 %, 50 %, 45 %, 40 %, 35 %, 30 %, 25 %, 20 %, 15 %, 10 %, 5 %, 4 %, 3 %, 2 %, 1 %, 0,5 %, 0,1 %, 0,01 %, 0,001 % ou 0,0001 % des CEC dans la cornée du sujet sont sous la forme d'un hexagone, ou dans lequel aucune des CEC dans la cornée du sujet ne sont sous la forme d'un hexagone.

FIGS. 1A-D

Scratch Recovery Rate of Different Concentrations of MSH

FIG. 2

INFγ  INFγ+αMSH10⁻¹²M  INFγ+αMSH10⁻⁸M  INFγ+αMSH10⁻⁴M

Media alone

ZO-1: Green
TUNEL positive: red

TNFα  TNFα+αMSH10⁻¹²M  TNFα+αMSH10⁻⁸M  TNFα+αMSH10⁻⁴M

FIG. 3A

FIG. 3B

FIG. 4

FIG. 5

FIG. 6

FIG. 7

FIG. 8A

FIG. 8B

FIG. 9

FIG. 10A

FIG. 10B

FIG. 11A

FIG. 11B

FIG. 11C

FIG. 11D

FIG. 12A

FIG. 12B

FIG. 12C

FIG. 12D

FIG. 13

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2015077416 A
- US 8703702 B
- US 7169603 B


**Non-patent literature cited in the description**

- **RU et al.** *Scientific Reports*, 21 December 2015, vol. 5 (1), 1-14
- **HASSELL et al.** *Exp Eye Res*, 2010, vol. 91, 326-35
- **BOURNE**. *Eye (Lond)*, 2003, vol. 17, 912-8
- **ARMITAGE et al.** *2003 Investig Opthalmology Vis Sci.*, 01 August 2003, vol. 44 (8), 3326
- **YEE et al.** *Curr Eye Res.*, 1985, vol. 4 (6), 671-8
- **BOURNE**. *Cornea*, 2001, vol. 20 (6), 560-9
- **PEH et al.** *Transplantation*, 2011, vol. 91 (8), 811-9
- **GUILBERT et al.** *Am J Ophthalmol*, 2013, vol. 155 (3), 560-569.e2
- **MEANS et al.** *Arch Ophthalmol*, 01 June 1995, vol. 113 (6), 805
- **NISHIMURA et al.** *Ophthalmology*, 1999, vol. 106 (10), 1962-5
- **PRICE et al.** *Ophthalmology*, 2008, vol. 115 (5), 857-65
- **DANA et al.** *Cornea*, 2000, vol. 19 (5), 625-43
- **TRIEF et al.** *Review of Ophthalmology*, 2016
- IC3D classification of corneal dystrophies--edition 2. **WEISS et al.** Cornea. 2015, vol. 34, 117-59
- **GRAHAM**. *Neurotrophic Keratopathy, Medscape*, 2016
- **LEMP MA**. Report of the National Eye Institute/Industry Workshop on clinical trials in dry eyes. *CLAO J*, 1995, vol. 21, 221-2
- Definition and Classification Subcommittee of the International Dry Eye WorkShop. *Ocular Surface*, April 2007, vol. 5 (2), 75-92
- **BOYD**. *Cataract Surgery American Academy of Ophthalmology*, 2016, www.aao.org/eye-health/diseases/what-is-cataract-surgery
- **EIPPER** ; **MAINS**. *Endocr Rev*, 1980, vol. 1 (1), 1-27
- **LUGER** ; **BRZOSKA**. *Ann Rheum Dis*, 2007, vol. 66 (3), iii52-5
- **WONG** ; **JENG**. *Journal of Neuroscience Research*, 1994, vol. 37 (1), 97-102
- **LUDWIG**. *Adv. Drug Deliv. Rev*, 2005, vol. 3 (57), 1595-639
- **GREENBAUM**. Optisol vs Dexsol as storage media for preservation of human corneal epithelium. *Eye*, 2004, vol. 18, 519-524
- **LINDSTROM et al.** Optisol corneal storage medium. *Am J Ophthalmol*, 1992, vol. 114 (3), 345-56
- **ARMITAGE**. Preservation of Human Cornea. *Transfus Med Hemother*, 2011, vol. 38 (2), 143-147
- **MCCAREY et al.** Review of Corneal Endothelial Specular Microscopy for FDA Clinical Trials of Refractive Procedures, Surgical Devices, and New Intraocular Drugs and Solutions. *Cornea*, 2008, vol. 27 (1), 1-16
- **PATEL et al.** Quantitative analysis of in vivo confocal microscopy images: A review. *Survey of Opthamology*, 2013, vol. 58, 466-475
- Cornea: Fundamentals, Diagnosis and Management. **SAYEGH et al.** Specular Microscopy. Elsevier, 2017, 160-179
- Cornea: Fundamentals, Diagnosis and Management. **PETROLL et al.** Confocal Microscopy. Elsevier, 2017, 180-191
- **MCCAREY et al.** *Ophthalmology*, 1979, vol. 86, 1848-1860
- **HOFFER**. *Am J Ophthalmol*, 1979, vol. 87, 252-253
- **YEE et al.** *Curr Eye Res*, 1985, vol. 4, 671-678
- **LAING et al.** *Arch Ophthalmol*, 1975, vol. 93, 143-145
- **MATSUDA**. *Arch Ophthalmol*, 1985, vol. 103, 68-70
- **MÜLLER et al.** *Exp Eye Res*, 2003, vol. 76 (5), 521-42
- **SABATINO et al.** *Ocul Surf*, 2017, vol. 15 (1), 2-14
- **BENETZ BA** ; **GAL RL** ; **RUEDY KJ** ; **RICE C** ; **BECK RW** ; **KALAJIAN AD** ; **LASS JH**. Cornea Donor Study Group. Specular microscopy ancillary study methods for donor endothelial cell density determination of Cornea Donor Study images. *Curr Eye Res*, April 2006, vol. 31 (4), 319-27

- **CHAUHAN et al.** *J Immunol*, 2009, vol. 182 (1), 148-53
- **JIN et al.** *Investig Opthalmology Vis Sci*, 2010, vol. 51 (2), 816
- **HULL**. *Acta Ophthalmol (Copenh)*, 1981, vol. 59, 409-21
- **QIAN et al.** *Cornea*, 2002, vol. 21, 592-7
- **QIAN et al.** *J Interferon Cytokine Res*, 2002, vol. 22, 1217-25